(19) Europäisches Patentamt
European Patent Office
Office européen des brevets

(11) EP 4 674 984 A1

(12) EUROPEAN PATENT APPLICATION

(43) Date of publication:
**07.01.2026 Bulletin 2026/02**

(21) Application number: **24186463.6**

(22) Date of filing: **04.07.2024**

(51) International Patent Classification (IPC):
***C12Q 1/70*** *(2006.01)* ***B82Y 5/00*** *(2011.01)*

(52) Cooperative Patent Classification (CPC):
**C12Q 1/70;** B82Y 5/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **CPTx GmbH**
**82152 Planegg (DE)**

(72) Inventors:
- **BERGER,, Nadja**
  **82152 Planegg (DE)**
- **MONFERRER,, Alba**
  **82152 Planegg (DE)**
- **KOHLER,, Fabian**
  **82152 Planegg (DE)**
- **SIGL,, Christian**
  **82152 Planegg (DE)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) COMPOSITIONS

(57) Provided herein is a DNA-based nanostructure at least a first plurality of virus-binding moieties, wherein each virus-binding moiety of the at least first plurality of virus-binding moiety is neuraminic acid or a derivative thereof. Also provided herein is a self-assembling DNA-based building block formed by a single-stranded DNA template strand and a set of oligonucleotides complementary to said single-stranded DNA template, wherein: each of the oligonucleotides is either complementary to one contiguous DNA sequence stretch or to at least two non-contiguous DNA sequence stretches on said single-stranded DNA template; and the self-assembling DNA-based building block comprises at least one first virus-binding moiety, wherein the first virus-binding moiety is neuraminic acid or a derivative thereof. Also provided herein is a DNA-based nanostructure wherein the DNA-based nanostructure comprises at least five self-assembling DNA-based building blocks; each of the self-assembling DNA-based building blocks is formed by a single-stranded DNA template strand and a set of oligonucleotides that are at least partially complementary to said single-stranded DNA template; and wherein each of the oligonucleotides is either complementary to one contiguous DNA sequence stretch or to at least two non-contiguous DNA sequence stretches on said single-stranded DNA template. Also provided herein are associated methods, uses, compositions, and kits.

Figure 1

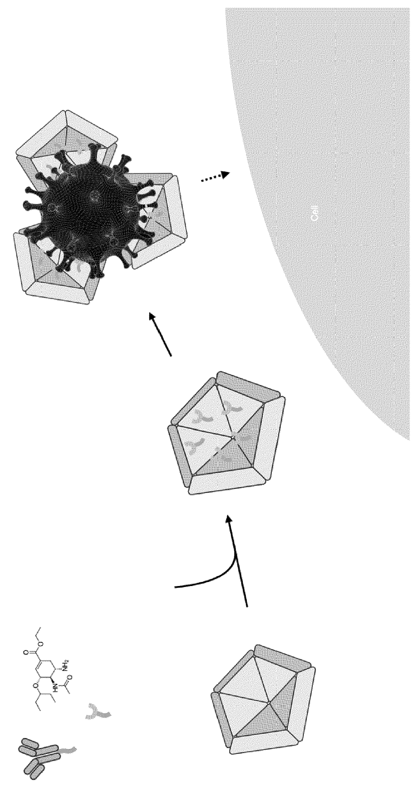

EP 4 674 984 A1

## Description

### Field of the invention

The present invention is in the field of therapeutic nanostructures, more particularly in the field of antiviral DNA-based nanostructures.

### Background

Influenza is a pervasive global health threat, causing annual epidemics and sporadic pandemics that pose serious public health and economic challenges. Influenza affects millions of people worldwide each year, leading to between 290,000 and 650,000 respiratory deaths annually according to the World Health Organization. The economic impact is also significant, with billions spent on healthcare costs and lost productivity.

Influenza viruses enter the body primarily through the respiratory tract, where they bind to sialic acid containing receptors on epithelial cells using their hemagglutinin (HA) proteins. Once bound, the virus is internalized and replicates. The neuraminidase (NA) protein facilitates the release of new virus particles by cleaving sialic acid residues, enabling the spread to new cells.

Sialyllactose, a derivative of sialic acid (N-acetylneuraminic acid), plays a crucial role in the influenza virus lifecycle by acting as a receptor for the hemagglutinin (HA) protein. The interaction between the influenza virus HA and sialic acid receptors has been a significant factor in interspecies transmission and the emergence of pandemics for nearly a century. Notably, the 1918 influenza pandemic, one of the most devastating outbreaks in human history, highlighted the importance of receptor binding in the virus's ability to cross species barriers. Key mutations near the receptor-binding site (RBS) of HA are associated with shifts in host specificity and pathogenicity, affecting the virus's capacity to infect and replicate in different hosts, including humans.

The specificity of influenza virus HA for sialic acid (SA)-linked glycan receptors determines the host range. Avian influenza viruses typically bind to SA receptors connected to galactose by an 2,3 linkage, a preference crucial for their replication in avian hosts. In contrast, human influenza strains commonly bind to SA receptors featuring an 2,6 linkage, predominant in the human upper respiratory tract. This difference in receptor preference is a critical barrier to cross-species transmission and is a focal point of surveillance and research, especially regarding the potential for avian viruses to adapt to human hosts.

Of the four currently recommended FDA-approved antiviral drugs, three - peramivir, zanamivir, and oseltamivir - are neuraminidase inhibitors, which function by preventing cleavage of sialic acid residues on the host cell surface by the influenza neuraminidase, inhibiting viral entry into host cells. The rapid evolution of influenza viruses has led to resistance to these and other therapeutic agents in some influenza serotypes. Furthermore, neuraminidase inhibitors are associated with unpleasant side effects including nausea, vomiting, and adverse respiratory symptoms; and although effective in treating mild flu infections by reducing time to first alleviation of symptoms, it has been reported that oseltamivir (for example) has limited effect on hospital admission, pneumonia, or bronchitis in more serious cases.

There is therefore a need for novel and improved therapeutic agents for use in treating influenza infection, and other viruses with similar cellular infection strategies.

### Brief description of the invention

The present invention provides DNA-based nanostructures and DNA-based building blocks thereof, for example a DNA-based nanostructure comprising virus binding moieties, and DNA-based building blocks thereof, that are suitable for a wide range of *in vivo* and *in vitro* purposes. Also provided herein are methods and uses for the DNA-based nanostructure and DNA-based building blocks thereof. For example, the DNA-based nanostructure, DNA-based building blocks thereof, and methods and uses therefor are suitable for use in treating infection with a virus such as an influenza virus. The DNA-based nanostructure, DNA-based building blocks thereof, and methods and uses therefor are also suitable for use in encapsulating or neutralising a virus such as an influenza virus, *in vivo* or *in vitro.*

As will be understood, the utility of the DNA-based nanostructure provided, DNA-based building blocks thereof, and methods and uses therefor provided herein is not limited to treatment of infection with a virus such as influenza virus, or encapsulation or neutralisation of a virus such as an influenza virus. Infection with any virus that is capable of being bound, encapsulated, or neutralised by the DNA-based nanostructure or DNA-based building blocks provided herein may suitably be treated using the method and uses provided herein. Furthermore, the DNA-based nanostructure provided herein is also capable of binding biological entities that are larger than viruses such as influenza virus, for example capable of binding cells. The DNA-based nanostructure provided, DNA-based building blocks thereof, and methods and uses therefor provided herein may therefore suitably be used to treat, for example, bacterial infection, fungal infection, and to bind, encapsulate, or neutralise host cells such as human cells.

Also provided herein are methods of making the DNA-based nanostructure and DNA-based building blocks thereof.

**Detailed description of the invention**

The invention provides a range of DNA-based nanostructures, DNA-based building blocks, and methods, compositions, and uses as set out below.

A virus-binding DNA-based nanostructure

In one aspect, provided herein is a DNA-based nanostructure, wherein the DNA-based nanostructure comprises at least a first plurality of virus-binding moieties, wherein each virus-binding moiety of the at least first plurality of virus-binding moiety is neuraminic acid or a derivative thereof.

It will be appreciated that a DNA-based nanostructure may be formed in any way that is suitable for producing 3-dimensional DNA structures. For example, the DNA-based nanostructure may comprise, for example, an extended lattice or DNA origami, which are known in the art. Preferably, the DNA-based nanostructure is formed from DNA origami. The DNA-based nanostructure may be formed from one individual extended lattice or DNA origami block. Alternatively, the DNA-based nanostructure may be formed by multiple subunits, wherein each subunit is formed from one individual extended lattice or DNA origami block.

Accordingly, in some embodiments the multiple subunits are self-assembling subunits such that the DNA-based nanostructure is formed by self-assembling DNA-based building blocks (or "subunits"). Each of the self-assembling DNA-based building blocks may be formed from a single-stranded DNA template strand and a set of oligonucleotides that are at least partially complementary to said single-stranded DNA template; and each of the oligonucleotides is either complementary to one contiguous DNA sequence stretch or to at least two non-contiguous DNA sequence stretches on said single-stranded DNA template.

Any suitable single-stranded DNA template strand and/or oligonucleotides that are at least partially complementary to said single-stranded DNA template may be used. For example, the single-stranded DNA template strand and/or oligonucleotides that are at least partially complementary to said single-stranded DNA template may be chemically synthesised, or may derived, for example, from an organism such as a bacterium, a fungus, an archaeon, or a bacteriophage. In some embodiments, the single-stranded DNA template strand and/or oligonucleotides that are at least partially complementary to said single-stranded DNA template is single-stranded DNA from a filamentous bacteriophage or is derived from single-stranded DNA of a filamentous bacteriophage. In some embodiments, the single-stranded DNA template strand and/or oligonucleotides that are at least partially complementary to said single-stranded DNA template is single-stranded DNA are obtained from different sources. For example, in some embodiments, the single-stranded DNA template strand is single-stranded DNA from a filamentous bacteriophage or is derived from single-stranded DNA of a filamentous bacteriophage, and the oligonucleotides that are at least partially complementary to said single-stranded DNA template is single-stranded DNA is chemically synthesised. Methods of chemically synthesising single-stranded DNA is known to the skilled person. In some embodiments, the single-stranded DNA template strand and/or oligonucleotides that are at least partially complementary to said single-stranded DNA template is single-stranded DNA are obtained from the same source, optionally from the same filamentous bacteriophage or is derived from single-stranded DNA of the same filamentous bacteriophage, for example using the methods disclosed in Praetorius et al., 2017, "Biotechnological mass production of DNA origami" Nature, 552:84-87, and International Patent Application No. PCT/EP2017/068051, published as WO 2018/054571 (each of which is herein incorporated by reference in its entirety).

As used herein, the term "filamentous bacteriophage" refers to a bacteriophage that is characterised by its filament-like shape that usually contains a genome of circular single-stranded DNA and infects Gram-negative bacteria. Exemplary filamentous bacteriophages include but are not limited to Ff phage such as M13, f1 and fd1 phage, and PF1 phage.

A single-stranded DNA template that is "derived from single-stranded DNA of filamentous bacteriophage" refers to a DNA construct that is derived from a naturally occurring of published DNA sequence of a filamentous bacteriophage by one or more of: (i) opening of the circular structure to a linear sequence; (ii) deletion of one or more nucleotides; (iii) insertion of one or more nucleotides; (iii) substitution of one or more nucleotides; (iv) addition of one or more nucleotides; and (v) modification of one or more nucleotides.

In some embodiments, the single-stranded DNA template has a sequence of SEQ ID NO: 1 of International Patent Application No. PCT/EP2023/080373, published as WO 2024/094708 A1; SEQ ID NO: 1 of International Patent Application No. PCT/EP2023/061264, published as WO 2023/209161 A1; SEQ ID NOs: 1 or 2 of International Patent Application No. PCT/EP2021/054307, published as WO 2021/165528 A1; or a sequence with:

a) at least 80%, at least 90%, at least 95%, at least 96%, at least 97%, at least 98%, at least 99% or more sequence

identity thereto;
b) about 80%, about 90%, about 95%, about 96%, about 97%, about 98%, about 99% sequence identity thereto; and/or
c) 80%, 90%, 95%, 96%, 97%, 98%, 99% or more sequence identity thereto.

In some embodiments, the single-stranded DNA-template has a sequence of SEQ ID NO: 1 or SEQ ID NO: 2 set out in herein. In some embodiments, the single-stranded DNA-template has a sequence with:

a) at least 80%, at least 90%, at least 95%, at least 96%, at least 97%, at least 98%, at least 99% or more sequence identity to SEQ ID NO: 1 or SEQ ID NO: 2 set out in herein;
b) about 80%, about 90%, about 95%, about 96%, about 97%, about 98%, about 99% sequence identity to SEQ ID NO: 1 or SEQ ID NO: 2 set out in herein; and/or
c) 80%, 90%, 95%, 96%, 97%, 98%, 99% or more sequence identity to SEQ ID NO: 1 or SEQ ID NO: 2 set out in herein.

In some embodiments, the oligonucleotides that are at least partially complementary to said single-stranded DNA template each have a sequence selected from SEQ ID NOs: 2-1407 of International Patent Application No. PCT/EP2023/080373, published as WO 2024/094708 A1; SEQ ID NOs: 2-1820 of International Patent Application No. PCT/EP2023/061264, published as WO 2023/209161 A1; SEQ ID NOs: 3-4089 of International Patent Application No. PCT/EP2021/054307, published as WO 2021/165528 A1; or a sequence with:

a) at least 80%, at least 90%, at least 95%, at least 96%, at least 97%, at least 98%, at least 99% or more sequence identity thereto;
b) about 80%, about 90%, about 95%, about 96%, about 97%, about 98%, about 99% sequence identity thereto; and/or
c) 80%, 90%, 95%, 96%, 97%, 98%, 99% or more sequence identity thereto.

In some embodiments, the oligonucleotides that are at least partially complementary to said single-stranded DNA template each have a sequence selected from SEQ ID NOs: 3-323 set out in **Table 1** herein. In some embodiments, the oligonucleotides that are at least partially complementary to said single-stranded DNA template each have a sequence selected from a sequence with:

a) at least 80%, at least 90%, at least 95%, at least 96%, at least 97%, at least 98%, at least 99% or more sequence identity to SEQ ID NO: 3-323 set out in **Table 1** herein;
b) about 80%, about 90%, about 95%, about 96%, about 97%, about 98%, about 99% sequence identity to SEQ ID NO: 3-323 set out in **Table 1** herein; and/or
c) 80%, 90%, 95%, 96%, 97%, 98%, 99% or more sequence identity to SEQ ID NO: 3-323 set out in **Table 1** herein.

In some embodiments:

a) the single-stranded DNA template has:

i) a sequence of SEQ ID NO: 1 set out herein;
ii) a sequence with at least 80%, at least 90%, at least 95%, at least 96%, at least 97%, at least 98%, at least 99% or more sequence identity to SEQ ID NO: 1 set out in herein;
iii) a sequence with about 80%, about 90%, about 95%, about 96%, about 97%, about 98%, about 99% sequence identity to SEQ ID NO: 1 set out in herein; and/or
iv) a sequence with 80%, 90%, 95%, 96%, 97%, 98%, 99% or more sequence identity to SEQ ID NO: 1 set out in herein;
and/or

b) the oligonucleotides that are at least partially complementary to said single-stranded DNA template each have:

i) a sequence set out in SEQ ID NOs: 3-131 set out in **Table 1;**
ii) a sequence with at least 80%, at least 90%, at least 95%, at least 96%, at least 97%, at least 98%, at least 99% or more sequence identity to SEQ ID NO: 3-131 set out in **Table 1** herein;

b) a sequence with about 80%, about 90%, about 95%, about 96%, about 97%, about 98%, about 99% sequence identity to SEQ ID NO: 3-131 set out in **Table 1** herein; and/or

c) a sequence with 80%, 90%, 95%, 96%, 97%, 98%, 99% or more sequence identity to SEQ ID NO: 3-131 set out in **Table 1** herein.

In some embodiments:

a) the single-stranded DNA template has:

i) a sequence of SEQ ID NO: 2 set out herein;
ii) a sequence with at least 80%, at least 90%, at least 95%, at least 96%, at least 97%, at least 98%, at least 99% or more sequence identity to SEQ ID NO: 2 set out in herein;
iii) a sequence with about 80%, about 90%, about 95%, about 96%, about 97%, about 98%, about 99% sequence identity to SEQ ID NO: 2 set out in herein; and/or
iv) a sequence with 80%, 90%, 95%, 96%, 97%, 98%, 99% or more sequence identity to SEQ ID NO: 2 set out in herein;
and/or

b) the oligonucleotides that are at least partially complementary to said single-stranded DNA template each have:

i) a sequence set out in SEQ ID NOs: 132-323 set out in **Table 1;**
ii) a sequence with at least 80%, at least 90%, at least 95%, at least 96%, at least 97%, at least 98%, at least 99% or more sequence identity to SEQ ID NO: 132-323 set out in **Table 1** herein;

b) a sequence with about 80%, about 90%, about 95%, about 96%, about 97%, about 98%, about 99% sequence identity to SEQ ID NO: 132-323 set out in **Table 1** herein; and/or
c) a sequence with 80%, 90%, 95%, 96%, 97%, 98%, 99% or more sequence identity to SEQ ID NO: 132-323 set out in **Table 1** herein.

As used herein, the term "neuraminic acid or a derivative thereof" includes any neuraminic acid or any derivative thereof. Preferably, the neuraminic acid or derivative thereof is capable of binding to the influenza virus haemagglutinin (HA) protein. In this way, binding of the neuraminic acid or derivative thereof to the influenza virus haemagglutinin protein recruits the DNA-based nanostructure to the surface of the influenza virus, thereby encapsulating or neutralising the virus. In some embodiments, the neuraminic acid or derivative thereof is selected from the group comprising or consisting of: neuraminic acid; sialic acid; sialyllactose including 3'-sialyllactose, 6'-sialyllactose, 2,3'-sialyllactose, and 2,6'-sialyllactose; N-acetylneruaminic acid; 2-keto-deoxynonic acid; 6'-sialyl-D-lactose; 3'- β -sialyl-β-lactose; N-glycolylneuraminic acid; and the following neuraminic acid derivatives disclosed in Song et al., 2011, "A Sialylated Glycan Microarray Reveals Novel Interactions of Modified Sialic Acids with Proteins and Viruses", J Biol. Chem, 286(36):31610-31622 (herein incorporated by reference in its entirety): Neu5Ac; Nau5Ac8Me; Neu5Ac9Me; Neu5,9$Ac_2$; Neu5Ac9Lt; Neu5Gc; Neu5GcMe; Neu5Gc9Ac; Neu5GcAc; Kdn; Kdn9Ac; Kdn5Ac; Kdn,9$Ac_2$; Kdn9Me; Kdn5Me; and Kdn7Me.

As will be appreciated, the DNA-based nanostructure may advantageously comprise more than one type of virus-binding agent. Accordingly, in some embodiment, the DNA-based nanostructure comprises a second plurality of virus-binding moieties. In some embodiments, the DNA-based nanostructure comprises a third plurality of virus-binding moieties, a fourth plurality of virus binding moieties, a fifth plurality of virus binding moieties, and/or a sixth plurality of virus binding moieties. DNA-based nanostructures comprising a second plurality of virus-binding moieties and/or optional third plurality of virus-binding moieties, fourth plurality of virus binding moieties, fifth plurality of virus binding moieties, and/or sixth plurality of virus binding moieties may have increased affinity, binding, and/or neutralisation of influenza virus compared to DNA-based nanostructures comprising only a first plurality of virus-binding moieties.

Suitable virus binding moieties of the second, and when present, third, fourth, fifth, and/or sixth pluralities of virus binding moieties include any virus binding moiety capable of binding to a ligand on the surface of the influenza virus. For example, the ligand may suitably be selected from the group comprising or consisting of: haemagglutinin (HA), neuraminidase (NA), M2 ion channel, and viral envelope. In some embodiments, the virus binding moieties of the second, and when present, third, fourth, fifth, and/or sixth pluralities of virus binding moieties are selected from the group comprising or consisting of: neuraminic acid or a derivative thereof; an antibody or a virus-binding fragment thereof, a nanobody or a virus-binding fragment thereof, an affimer, heparin, heparan sulfate, hybrid heparan sulfates, a host receptor protein or virus-binding fragment thereof, a *de novo* virus-binding peptide, an aptamer, and a neuraminidase inhibitor; optionally wherein the neuraminidase inhibitor is selected from the group comprising or consisting of: oseltamivir, zanamivir, ianamivir, and peramivir; optionally wherein the neuraminidase inhibitor is oseltamivir. As will be understood, the inclusion of a neuraminidase inhibitor on the nanostructure provides an additional virus-binding moiety, strengthening binding of the nanostructure to the target virus; and/or may inhibit neuraminidase activity of the virus, thereby preventing

cleavage of neuraminic acid residues from the DNA-based nanostructure and/or further inhibiting viral entry into a cell. Suitably, the antibody or virus-binding fragment thereof, nanobody or virus-binding fragment thereof, and/or affimer are capable of binding to a ligand selected from the group comprising or consisting of: haemagglutinin (HA), neuraminidase (NA), M2 ion channel, and viral envelope.

In some embodiments, the virus-binding moieties of each plurality of virus binding moieties is different to the virus-binding moieties of every other plurality of virus-binding moieties. In some embodiments, each plurality of virus-binding moieties may comprise the same virus binding moiety. In some embodiments, each plurality virus-binding moieties may comprise neuraminic acid or a derivative thereof, where each plurality comprises a different neuraminic acid or derivative thereof; optionally wherein the virus-binding moieties of each plurality are selected from the group comprising or consisting of: neuraminic acid; sialic acid; sialyllactose including 3'-sialyllactose, 6'-sialyllactose, 2,3'-sialyllactose, and 2,6'-sialyllactose; N-acetylneruaminic acid; 2-keto-deoxynonic acid; 6'-sialyl-D-lactose; 3'- β -sialyl-β-lactose; N-glycolylneuraminic acid; and the following neuraminic acid derivatives disclosed in Song et al., 2011, "A Sialylated Glycan Microarray Reveals Novel Interactions of Modified Sialic Acids with Proteins and Viruses", J Biol. Chem, 286(36):31610-31622 (herein incorporated by reference in its entirety): Neu5Ac; Nau5Ac8Me; Neu5Ac9Me; Neu5,9$Ac_2$; Neu5Ac9Lt; Neu5Gc; Neu5GcMe; Neu5Gc9Ac; Neu5GcAc; Kdn; Kdn9Ac; Kdn5Ac; Kdn,9$Ac_2$; Kdn9Me; Kdn5Me; and Kdn7Me. In some embodiments, at least two of the pluralities of the virus-binding moiety are different neuraminic acids or are derivatives thereof; optionally wherein the virus-binding moieties of each plurality are selected from the group comprising or consisting of: neuraminic acid; sialic acid; sialyllactose including 3'-sialyllactose, 6'-sialyllactose, 2,3'-sialyllactose, and 2,6'-sialyllactose; N-acetylneruaminic acid; 2-keto-deoxynonic acid; 6'-sialyl-D-lactose; 3'- β -sialyl-β-lactose; N-glycolylneuraminic acid; and the following neuraminic acid derivatives disclosed in Song et al., 2011, "A Sialylated Glycan Microarray Reveals Novel Interactions of Modified Sialic Acids with Proteins and Viruses", J Biol. Chem, 286(36):31610-31622 (herein incorporated by reference in its entirety): Neu5Ac; Nau5Ac8Me; Neu5Ac9Me; Neu5,9$Ac_2$; Neu5Ac9Lt; Neu5Gc; Neu5GcMe; Neu5Gc9Ac; Neu5GcAc; Kdn; Kdn9Ac; Kdn5Ac; Kdn,9$Ac_2$; Kdn9Me; Kdn5Me; and Kdn7Me..

The virus-binding moieties of the plurality or pluralities of virus-binding moieties may be bound to the DNA-based nanostructure in any suitable way. For example, in some embodiments, the virus-binding moieties are covalently bound to the nanostructure. In some embodiments, the virus-binding moieties are covalently bound to the single-stranded DNA template strand. In some embodiments, the virus-binding moieties are covalently bound to the oligonucleotides of the set of oligonucleotides complementary to said single-stranded DNA template.

In some embodiments, the virus-binding moieties are covalently bound to nanostructure by a linker. The suitability of a linker depends on the chemistry of the virus-binding moiety that is to be covalently bound to the nanostructure. The person skilled in the art will be aware of which linkers are suitable for use with specific virus-binding moieties. In some embodiments, the linker is selected from the group comprising or consisting of:

a) an oligo-PEG-linker, optionally wherein the oligo-PEG linker has the structure:

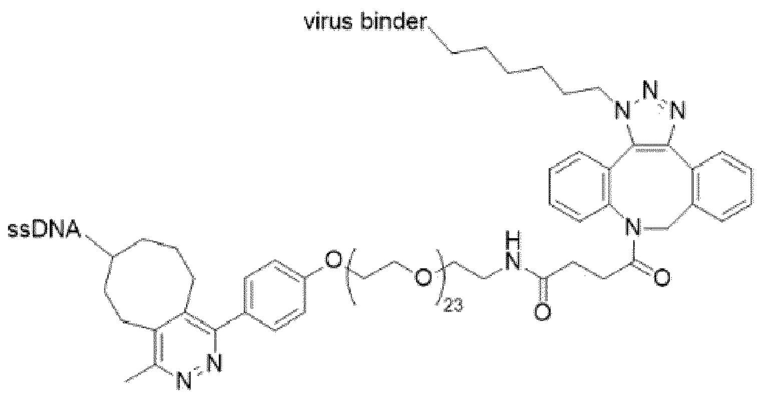

;

b) a PEG linker, optionally wherein the PEG linker has the structure:

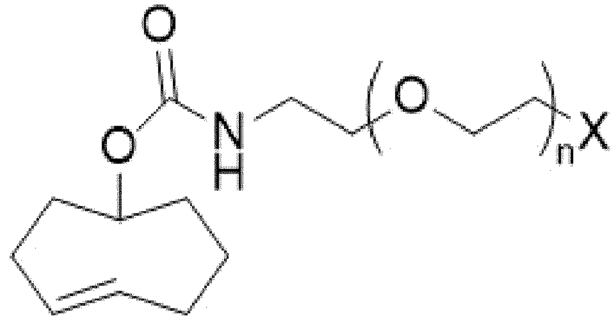

Optionally wherein n is an integer between 4 and 227 and X is a linker covalently bound to the virus-binding moiety, optionally wherein the linker is DBCO.

In some embodiments, the virus-binding moiety is non-covalently bound to the DNA-based nanostructure, for example is bound to an entity that forms part of the DNA-based nanostructure directly or indirectly *via* an electrostatic or ionic interaction, $\pi$-effect, van der Waal forces, hydrophobic effect, or in any other suitable way.

In some embodiments, the DNA-based nanostructure comprises one or more handle sites; optionally wherein the one or more handle sites comprise single-stranded DNA overhangs. Any suitable number of handle sites may be used. In some embodiments, where the DNA-based nanostructure is formed from self-assembling DNA building blocks, the handle sites are comprised by the self-assembling DNA building blocks, optionally wherein each self-assembling DNA building block comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 or more handle sites. In some embodiments, where the DNA-based nanostructure is formed using a single-stranded DNA template strand (e.g. when the structure is formed using DNA origami) the one or more handle sites are comprised by the single-stranded DNA template strand (also known as the "scaffold"). In some embodiments, the one or more handle sites are comprised by the set of oligonucleotides that are at least partially complementary to the single-stranded DNA template (also known as the "staples" or "staple strands"). In some embodiments, the handle sites are single-stranded oligonucleotides having a length of between 20 and 200 nucleotides, 50 and 150 nucleotides, 75 and 125 nucleotides, 20 and 60 nucleotides, 40 and 55 nucleotides; 20 and 30 nucleotides, 22 and 28 nucleotides, 25 and 27 nucleotides, 45 and 55 nucleotides, or 47 and 53 nucleotides.

Suitable handle sites (also known as an "anchor sites") and their placement in the DNA-based nanostructure are disclosed for example: on pages 16, 77, and 86-87, in SEQ ID NOs: 2437-2454, 2692-2702, 2915-2929, 3191-3199, and 3422-2429, and in Example 4, Example 5, and Example 6 of International Patent Application No. PCT/EP2021/054307, published as WO 2021/165528 A1; pages 2-7, 9-10, 13, 16-17, and 20, in SEQ ID NOs: 177-203, 398-415, 607-633, 821-829, 1018-1026, 1216-1224, 1416-1424, 1613-1621, and 1812-1820, and Examples 1, 2, and 4 of International Patent Application No. PCT/2023/061264, published as WO 2023/209161 A1; and on pages 8 and 11, in SEQ ID NOs: 146-163, 437-468, 698-729, 915-932, 1136-1167, 1376-1407, and in the Examples of International Patent Application No. PCT 2023/080373, published as WO 2024/094708 A1 (each of which is herein incorporated by reference in its entirety); or a sequence with:

a) at least 80%, at least 90%, at least 95%, at least 96%, at least 97%, at least 98%, at least 99% or more sequence identity to the said sequences;
b) about 80%, about 90%, about 95%, about 96%, about 97%, about 98%, about 99% sequence identity to the said sequences; and/or
c) 80%, 90%, 95%, 96%, 97%, 98%, 99% or more sequence identity to the said sequences.

In some embodiments, the handle site has a sequence of SEQ ID NO: 324-330 set out in **Table 1** herein. In some embodiments, the handle site has a sequence with:

a) at least 80%, at least 90%, at least 95%, at least 96%, at least 97%, at least 98%, at least 99% or more sequence identity to SEQ ID NO: 324-330 set out in **Table 1** herein;
b) about 80%, about 90%, about 95%, about 96%, about 97%, about 98%, about 99% sequence identity to SEQ ID NO: 324-330 set out in **Table 1** herein; and/or
c) 80%, 90%, 95%, 96%, 97%, 98%, 99% or more sequence identity to SEQ ID NO: 324-330 set out in **Table 1** herein.

In some embodiments, the virus-binding moieties are conjugated to a handle-binding oligonucleotide that is able to hybridise to the handle sites; optionally wherein the handle-binding oligonucleotide is complementary to the handle site. Any suitable handle-binding oligonucleotide may be used, provided it is complementary to and anneals to the handle site comprised by the DNA-based nanostructure. Exemplary suitable handle-binding oligonucleotides are disclosed: on pages

6, 13, 14, 17-19, 29, 34-35, and Examples 4, 6, and 7 of International Patent Application No. PCT/EP2021/054307, published as WO 2021/165528 A1; on pages 4, 6, 16, in SEQ ID NO: 1821, and Examples 1, 2, and 4 of International Patent Application No. PCT/2023/061264, published as WO 2023/209161 A1; and on pages 8 and 11, in SEQ ID NO: 1408, and in the Examples of International Patent Application No. PCT 2023/080373, published as WO 2024/094708 A1 (each of which is herein incorporated by reference in its entirety); or a sequence with:

a) at least 80%, at least 90%, at least 95%, at least 96%, at least 97%, at least 98%, at least 99% or more sequence identity to the said sequences;
b) about 80%, about 90%, about 95%, about 96%, about 97%, about 98%, about 99% sequence identity to the said sequences; and/or
c) 80%, 90%, 95%, 96%, 97%, 98%, 99% or more sequence identity to the said sequences.

In some embodiments, the handle-binding oligonucleotide has a sequence of SEQ ID NO: 331 set out in **Table 1** herein. In some embodiments, the handle-binding oligonucleotide has a sequence with:

a) at least 80%, at least 90%, at least 95%, at least 96%, at least 97%, at least 98%, at least 99% or more sequence identity to SEQ ID NO: 331 set out in **Table 1** herein;
b) about 80%, about 90%, about 95%, about 96%, about 97%, about 98%, about 99% sequence identity to SEQ ID NO: 331 set out in **Table 1** herein; and/or
c) 80%, 90%, 95%, 96%, 97%, 98%, 99% or more sequence identity to SEQ ID NO: 331 set out in **Table 1** herein.

In some embodiments, the virus-binding moieties are conjugated to the handle-binding oligonucleotide by a linker. In some embodiments, the linker is selected from the group comprising or consisting of:

a) by an ssDNA-$PEG_4$-azide linker; optionally wherein the ssDNA-$PEG_4$-azide linker has the structure:

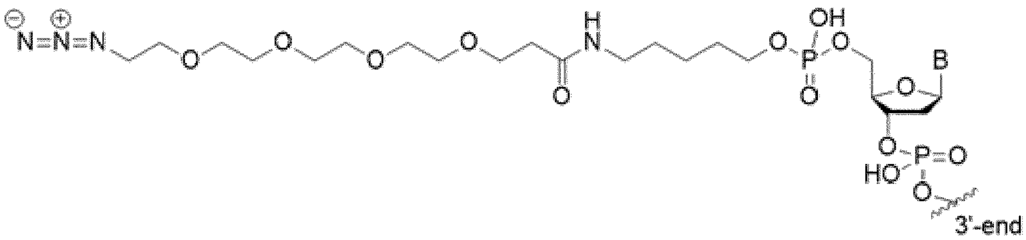

;

b) an ssDNA-DBCO linker; optionally wherein the ssDNA-DBCO linker has the structure:

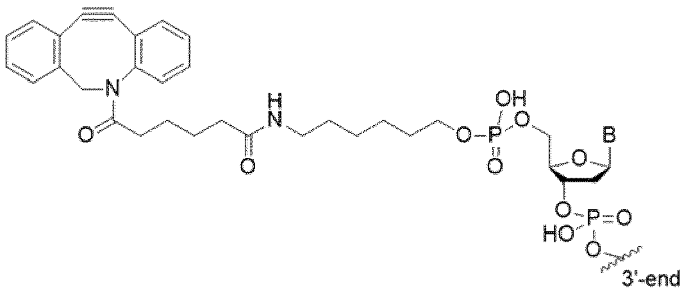
;

c) an ssDNA-SH linker; optionally wherein the ssDNA-SH linker has the structure:

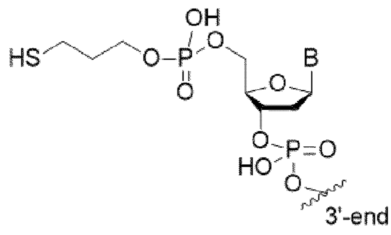

;

d) an ssDNA-PEGs-methyltetrazine linker; optionally wherein the ssDNA-PEGs-methyltetrazine linker has the structure:

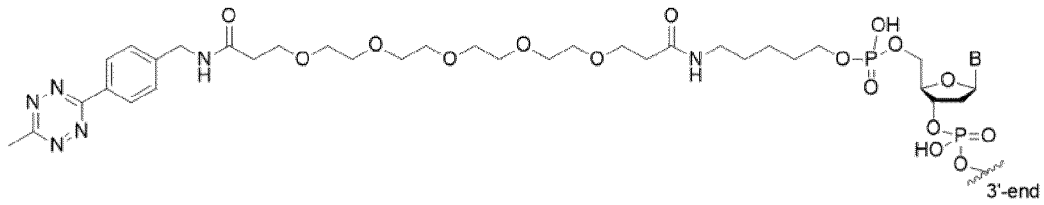

; and/or

e) an oligo-PEG-linker, optionally wherein the oligo-PEG-linker has the structure:

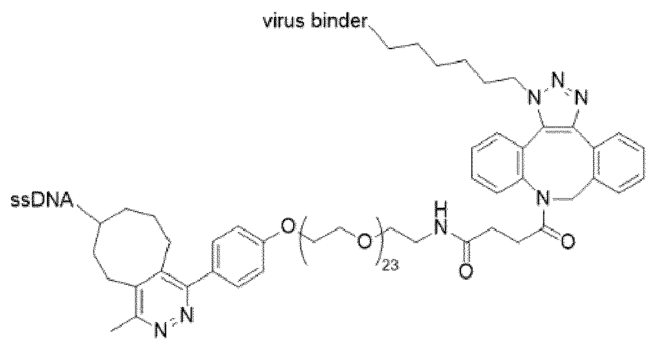

.

In some instances, it is advantageous to covalently bind the handle-binding oligonucleotide to the handle oligonucleotide, for example to improve the stability of the DNA-based nanostructure. In some embodiments, the handle-binding oligonucleotide is covalently bound to the handle site. In some embodiments, the handle-binding oligonucleotide is covalently bound to the handle site by a DNA crosslinking agent or an alkylating agent. In some embodiments, the handle-binding oligonucleotide is covalently bound to the handle site by UV-welding. As is known in the art, excitation of two neighbouring thymine residues using UV light leads to the formation of a covalently bonded thymine dimer. The thymine residues may be present on the same DNA strand or may be present on complementary strands. The person skilled in the art is capable of designing complementary handle-binding oligonucleotides and handle oligonucleotides containing thymine residues at appropriate positions to permit UV welding.

Several neuraminic acid and derivatives thereof may be hydrolysed by the influenza virus neuraminidase (NA). In some instances, it may be advantageous to inhibit degradation of the neuraminic acid and derivative thereof by influenza NA. Accordingly, in some embodiments, the neuraminic acid or derivative thereof is resistant to cleavage or degradation by a neuraminidase; or is a non-cleavable neuraminic acid or non-cleavable neuraminic acid derivative. As used herein, the terms "non-cleavable neuraminic acid" and "non-cleavable neuraminic acid derivative" include neuraminic acids and neuraminic acid derivatives that are resistant to cleavage by influenza neuraminidase or are not cleaved by influenza neuraminidase. Methods of determining the resistance to cleavage by influenza neuraminidase are known in the art, and include, for example, subjecting a test neuraminic acid or test neuraminic acid derivative and a known reference molecule to neuraminidase treatment, and comparing the surviving quantity of said test neuraminic acid or test neuraminic acid derivative to the surviving quantity of the reference molecule that is present after neuraminidase treatment. If the surviving quantity of the test neuraminic acid or test neuraminic acid derivative is higher than the surviving quantity of the reference molecule after neuraminidase treatment, then the test neuraminic acid or test neuraminic acid derivative is identified as a non-cleavable neuraminic acid or non-cleavable neuraminic acid derivative, respectively. Suitable reference molecules include, for example, 2,3'-sialyllactose and 2,6'-sialyllactose.

Any non-cleavable neuraminic acid or neuraminic acid derivative may suitably be used as a virus-binding moiety.

The skilled person will appreciate that the non-cleavable neuraminic acid or neuraminic acid derivative must retain the ability to bind to viral hemagglutinin (HA) proteins. In some embodiments, the neuraminic acid or derivative thereof has a structure selected from the group comprising or consisting of:

a)

Compound I:

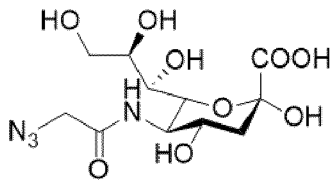

;

b)

Compound II:

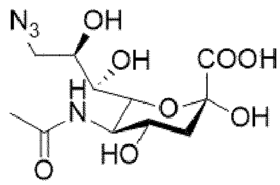

;

c)

Compound III:

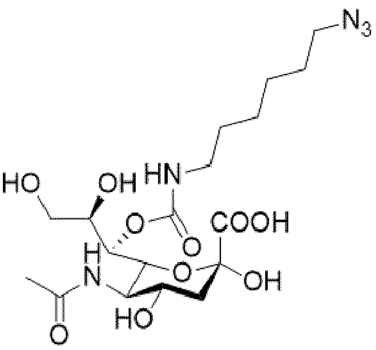

;

d)

Compound IV:

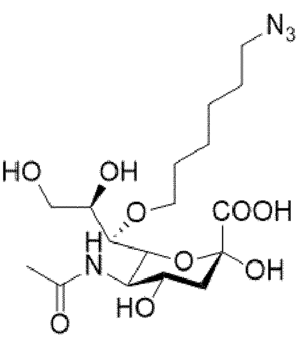

;

e)

Compound Va:

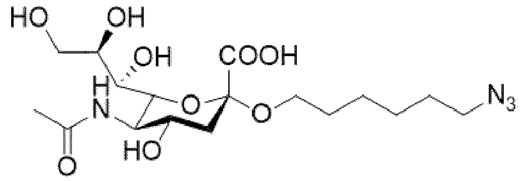

;

f)

Compound Vb:

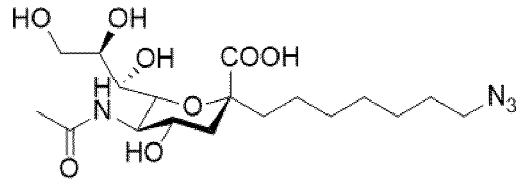

;

And

g)

Compound VI:

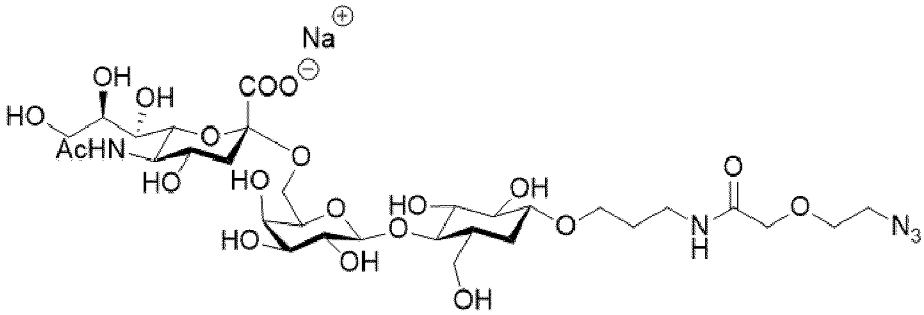

.

Where the DNA-based nanostructure is formed from self-assembling DNA-based building blocks (or "subunits"), the virus-binding moieties may be arranged on the self-assembling DNA-based building blocks in any permutation. For example, a given self-assembling DNA-based building block may comprise virus binding moieties from only one plurality of virus-binding moieties. Alternatively, a given self-assembling DNA-based building block may comprise virus-binding moieties from two, three, four, five, or six different pluralities of virus-binding moieties. Furthermore, each self-assembling DNA-based building block comprised by the DNA nanostructure may comprise a different complement of virus-binding moieties selected from the first, second, third, fourth, fifth, and sixth pluralities of virus-binding moieties; or may comprise an identical complement of virus-binding moieties selected from the first, second, third, fourth, fifth, and sixth pluralities of virus-binding moieties.

Accordingly, in some embodiments, each self-assembling DNA-based building block of the nanostructure comprises at least one virus-binding moiety of the first plurality of virus-binding moieties. In some embodiments, each self-assembling DNA-based building block of the nanostructure comprises:

a) at least one virus-binding moiety of the first plurality of virus-binding moieties, at least one virus-binding moiety of the second plurality of virus-binding moieties, at least one virus-binding moiety of the third plurality of virus-binding moieties, at least one virus-binding moiety of the fourth plurality of virus binding moieties, at least one virus-binding moiety of the fifth plurality of virus binding moieties, and/or at least one virus-binding moiety of the sixth plurality of virus binding moieties;

b) at least one virus-binding moiety of the first plurality of virus-binding moieties, at least one virus-binding moiety of the second plurality of virus-binding moieties, at least one virus-binding moiety of the third plurality of virus-binding moieties, at least one virus-binding moiety of the fourth plurality of virus binding moieties, at least one virus-binding moiety of the fifth plurality of virus binding moieties, and at least one virus-binding moiety of the sixth plurality of virus

binding moieties;

c) at least one virus-binding moiety of the first plurality of virus-binding moieties, at least one virus-binding moiety of the second plurality of virus-binding moieties, at least one virus-binding moiety of the third plurality of virus-binding moieties, at least one virus-binding moiety of the fourth plurality of virus binding moieties, at least one virus-binding moiety of the fifth plurality of virus binding moieties, or at least one virus-binding moiety of the sixth plurality of virus binding moieties.

The DNA-based nanostructure may comprise any suitable number of virus-binding moieties. For example, in some embodiments, the DNA-based nanostructure comprises:

a) between 1-30 virus-binding moieties, such as 2-28, 2-26, 4-24, 6-22, 8-20, 10-18, or 12-16 virus binding moieties;
b) at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 27, at least 28, at least 29, or more virus-binding moieties;
c) about 1, about 2, about 3, about 4, about 5, about 6, about 7, about 8, about 9, about 10, about 11, about 12, about 13, about 14, about 15, about 16, about 17, about 18, about 19, about 20, about 22, about 23, about 24, about 25, about 26, about 27, about 28, about 29, or about 30 virus-binding moieties; and/or
d) 1, 2, 3, 4, 5, 6, 7, 8, 9, 10, 11, 12, 13, 14, 15, 16, 17, 18, 19, 20, 21, 22, 23, 24 25, 26, 27, 28, 29, or 30 virus-binding moieties.

In some embodiments, the DNA-based nanostructure comprises:

a) at least 5, at least 10, at least 20, at least 25, at least 30, at least 35, at least 40, at least 45, at least 50, at least 55, at least 60, at least 65, at least 70, at least 75, at least 80, at least 85, at least 90, at least 95, at least 100, at least 105, at least 110, at least 115, at least 120, at least 125, at least 130, at least 135, at least 140, at least 145, at least 150, at least 155, at least 160, at least 165, at least 170, at least 175, at least 180, at least 185, at least 190, at least 195, at least 200, at least 205, or more virus-binding moieties;
b) about 5, about 10, about 20, about 25, about 30, about 35, about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, about 205, or about 201 virus-binding moieties;
c) 5, 10, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, or 201 virus-binding moieties.

In some embodiments, each self-assembling DNA-based building block of the nanostructure comprises:

a) between 1-30 virus-binding moieties, such as 2-28, 2-26, 4-24, 6-22, 8-20, 10-18, or 12-16 virus binding moieties;
b) at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 27, at least 28, at least 29, or more virus-binding moieties;
c) about 1, about 2, about 3, about 4, about 5, about 6, about 7, about 8, about 9, about 10, about 11, about 12, about 13, about 14, about 15, about 16, about 17, about 18, about 19, about 20, about 22, about 23, about 24, about 25, about 26, about 27, about 28, about 29, or about 30 virus-binding moieties; and/or
d) 1, 2, 3, 4, 5, 6, 7, 8, 9, 10, 11, 12, 13, 14, 15, 16, 17, 18, 19, 20, 21, 22, 23, 24 25, 26, 27, 28, 29, or 30 virus-binding moieties.

As described herein, the DNA-based nanostructure is suitable for use in treating infection with a virus such as an influenza virus, and in encapsulating or neutralising a virus such as an influenza virus, *in vivo* or *in vitro.*

Accordingly, in one embodiment, the nanostructure is configured to bind to the surface of a target virus or to at least partially encapsulate a target virus, optionally wherein the target virus is an influenza virus. Without wishing to be bound by theory, the inventors hypothesise that binding of the nanostructure to or at least partial encapsulation of a target virus effectively prevents the binding of a virus to and/or entry of the virus into a host cell, thereby limiting viral propagation. As will be understood, where a virus cannot propagate in a host during infection, said infection is treated.

As will be understood, the utility of the DNA-based nanostructure provided herein is not limited to influenza. For example, the DNA-based nanostructure may be configured to bind to the surface or at least partially encapsulate any target virus that binds to neuraminic acid or a derivative thereof. Furthermore, the DNA-based nanostructure may be configured to bind to the surface of or at least partially encapsulate a target virus that is not an influenza virus by including as virus-binding moieties in the second, third, fourth, fifth, or sixth pluralities of virus-binding moieties compounds or

molecules that bind to a virus that is not an influenza virus. By way of non-limiting example, in embodiments where the DNA-based nanostructure comprises a first plurality of virus-binding moieties that are neuraminic acid or a derivative thereof, and a second plurality of virus-binding moieties that are, for example, anti-SARS-CoV-2 spike protein antibodies, the DNA-based nanostructure would be able to bind to the surface of or at least partially encapsulate both influenza virus and SARS-CoV-2. The same approach may be taken *mutatis mutandis* with the third, fourth, fifth, and sixth pluralities of virus-binding moieties, and other target viruses that are not influenza virus. Accordingly, in some embodiments, the DNA-based nanostructure is configured to bind to the surface of or at least partially encapsulate influenza virus and at least one virus selected from the group comprising or consisting of: AAV2, hepatitis B (HBV), SARS-CoV-2, chikungunya virus, and Zika virus. Suitable virus-binding moieties for targeting a DNA-based nanostructure to AAV2, hepatitis B (HBV), SARS-CoV-2, chikungunya virus, and Zika virus are disclosed in: International Patent Application No. PCT/EP2021/054307, published as WO 2021/165528 A1; International Patent Application No. PCT/2023/061264, published as WO 2023/209161 A1; and International Patent Application No. PCT 2023/080373, published as WO 2024/094708 A1 (each of which is herein incorporated by reference in its entirety).

The DNA-based nanostructure provided herein may be configured to bind to any proportion of the surface of a target virus. For example, in some embodiments the nanostructure is configured to bind to:

a) at least 5% of the surface of the target virus, optionally at least 10%, at least 20%, at least 30%, at least 40%, at least 50%, at least 60%, at least 70%, at least 75%, at least 80%, at least 85%, at least 90%, at least 95%, at least 96%, at least 97%, at least 98%, at least 99%, or more of the surface of the target virus;
b) about 5% of the surface of the target virus, optionally about 10%, about 20%, about 30%, about 40%, about 50%, about 60%, about 70%, about 75%, about 80%, about 85%, about 90%, about 95%, about 96%, about 97%, about 98%, about 99%, or 100% of the surface of the target virus; and/or
c) 5% of the surface of the target virus, optionally 10%, 20%, 30%, 40%, 50%, 60%, 70%, 75%, 80%, 85%, 90%, 95%, 96%, 97%, 98%, 99%, 100% of the target virus.

In some embodiments, the nanostructure is configured to fully encapsulate the target virus.

The DNA-based nanostructure may bind to the surface of or at least partially encapsulate any influenza type. Accordingly, in some embodiments, the DNA-based nanostructure is configured to bind to the surface of or at least partially encapsulate an influenza virus selected from the group comprising or consisting of: Influenza A, Influenza B, Influenza C, and Influenza D; optionally wherein the influenza virus is Influenza A or Influenza B.

In some embodiments, the DNA-based nanostructure is configured to bind to the surface of or at least partially encapsulate an influenza virus wherein:

a) the influenza virus has a haemagglutinin subtype selected from the group comprising or consisting of: H1, H2, H3, H4, H5, H6, H7, H8, H9, H10, H11, H12, H13, H14, H15, H16, H17, and H18; and/or
b) the influenza virus has a neuraminidase subtype selected from: N1, N2, N3, N4, N5, N6, N7, N8, N9, N10, and N11.

The DNA-based nanostructure provided herein may be configured to bind to the surface of or at least partially encapsulate any influenza virus. However, it may be advantageous in some instances to configure the NDA-based nanostructure to bind to the surface of or at least partially encapsulate an influenza virus subtype that is known to infect and/or be pathogenic to humans. Accordingly, in some embodiments, the DNA-based nanostructure is configured to bind to the surface of or at least partially encapsulate an influenza virus, wherein the influenza virus has a subtype selected from the group comprising or consisting of: H1N1, H2N2, H3N2, H5N1, H5N5, H5N6, H5N8, H6N1, H7N2, H7N7, H7N9, and H10N7; optionally wherein the influenza has a subtype that is H3N2, H5N1, H7N9, or H1N1.

One therapeutic approach to treating infection with a virus is to prevent entry of the virus into a host cell. Accordingly, in some embodiments, binding of the nanostructure to the target virus inhibits the virus from binding to and/or entering a host cell; optionally wherein the host cell is an animal cell, optionally wherein the host cell is a human cell.

In some embodiments, binding of the nanostructure to the target virus neutralises the target virus, optionally wherein binding of the nanostructure to the target virus neutralises the target virus:

a) to at least 50%, at least 60%, at least 70%, at least 80%, at least 85%, at least 90%, at least 95%, at least 96%, at least 97%, at least 98%, at least 99%, or more;
b) to about 50%, about 60%, about 70%, about 80%, about 85%, about 90%, about 95%, about 96%, about 97%, about 98%, about 99%, or about 100%; and/or
c) to 50%, 60%, 70%, 80%, 85%, 90%, 95%, 96%, 97%, 98%, 99%, or 100%.

The person skilled in the art will be well aware of suitable techniques to monitor the viral neutralising ability of a DNA-based nanostructure provided herein. One example of such a method involves allowing one or more target viruses to

infect a population of potential host cells, wherein the DNA-based nanostructure under assay is mixed with the virus, and then the mixture is incubated with the potential host cells. The number of cells infected or the total amount of influenza viruses gives a measure of the neutralising ability of the DNA-based nanostructure, *i.e.,* the ability of the DNA-based nanostructure to prevent infection. In one particular example, the neutralising potential of the DNA-based nanostructure can be determined using the plaque reduction neutralisation testing (PRNT), where the reduction in the number of viral plaques formed on a cell monolayer is compared to a control (no DNA-based nanostructure). In another example, the amount of generated viruses is measured with quantitative polymerase chain reaction (qPCR). Other such examples will be known to those skilled in the art, for example hemagglutination inhibition assays, pseudotyped virus neutralization assays.

In addition to preventing entry of a virus into a host cell, it may in some instances be advantageous to recruit immune cells including phagocytic cells such as macrophages and dendritic cells to an infection site, and/or label a target virus for phagocytosis. Accordingly, in some embodiments, the DNA-based nanostructure further comprises at least a first plurality of recruitment moieties, which are capable of recruiting immune cells and/or mark the target virus for phagocytosis. Recruitment moieties that are capable of recruiting immune cells and/or marking a target virus are known to the person skilled in the art. In some embodiments, each recruitment moiety of the first plurality of recruitment moieties is selected from the group comprising or consisting of: an antibody Fc fragment, a TLR ligand, and an opsonin.

The DNA-based nanostructure may have any shape that is suitable for binding to at the surface of or at least partially encapsulating a target virus. As will be appreciated, the shape of the DNA-based nanostructure may be determined by the shape of the self-assembling DNA-based building blocks used to form the DNA-based nanostructure. The self-assembling DNA-based building blocks may have any suitable shape. In some embodiments each of the self-assembling DNA-based building blocks has an identical shape. In some embodiments, the self-assembling DNA-based building blocks have a plurality of different shapes.

In some embodiments, the DNA-based nanostructure has a concave surface and/or a convex surface. The concave surface may suitably be configured to bind to the surface of the target virus. Accordingly, in some embodiments the at least a first plurality of virus-binding moieties is bound to the concave surface. In some embodiments, when present the second plurality of virus-binding moieties, third plurality of virus-binding moieties, fourth plurality of virus binding moieties, fifth plurality of virus binding moieties, and/or sixth plurality of virus binding moieties are bound to the concave surface.

In some embodiments, when present, the at least a first plurality of recruitment moieties is bound to the convex surface. In this way, when the DNA-based nanostructure is bound to a target *via* virus-binding moieties bound to the concave surface, the recruitment moieties are available to recruit immune cells to the target virus.

In some embodiments, each of the DNA-based building blocks is a triangular prismoid. Suitable DNA-based building blocks in the shape of triangular prismoids and methods for producing the same are disclosed, for example, in International Patent Application No. PCT/EP2021/054307, published as WO 2021/165528 A1; International Patent Application No. PCT/2023/061264, published as WO 2023/209161 A1; International Patent Application No. PCT 2023/080373, published as WO 2024/094708 A1; and Sigl, C., 2021, Programmable DNA Origami Shell Platform for Virus Neutralization [Doctoral thesis, Technische Universität München], available at: https://mediatum.ub.tum.de/1625147 (each of which is herein incorporated by reference in its entirety).

In some embodiments, each triangular prismoid is formed by m triangular planes, wherein m is an integer independently selected from 4, 5, 6, 7 and 8, in particular independently selected from 5, 6 and 7, more particularly wherein said integer is 6;

> the three edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4;
> wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes; and
> wherein at least two of the three side trapezoids comprise a specific pattern of recesses and/or extrusions formed by missing or additional DNA double helical stretches for specific interaction with a complementary pattern on the side trapezoid of another one of said self-assembling DNA-based building blocks.

In some embodiments, for at least part of the self-assembling DNA-based building blocks the length of at least one edge of each of said m planes is decreasing from the first to the $m^{th}$ plane, so that bevel angles results between planes perpendicular to said first plane and each of the trapezoid planes formed by said m edges.

In some embodiments the bevel angle is:

a) between 0° and 30°, for example between 2° and 28°, 4° and 26°, 6° and 24°, 8° and 22°, or 10° and 20°;
b) between 8° and 15°, for example between 10° and 13°, or 11° and 12°;
c) about 0°, about 1°, about 2°, about 3°, about 4°, about 5°, about 6°, about 7°, about 8°, about 9°, about 10°, about 11°, about 12°, about 13°, about 14°, about 15°, about 16°, about 17°, about 18°, about 19°, about 20°, about 21°, about 22°, about 23°, about 24°, about 25°, about 26°, about 27°, about 28°, about 29°, or about 30°; and/or
c) 0°, 1°, 2°, 3°, 4°, 5°, 6°, 7°, 8°, 9°, 10°, 11°, 12°, 13°, 14°, 15°, 16°, 17°, 18°, 19°, 20°, 21°, 22°, 23°, 24°, 25°, 26°, 27°, 28°, 29°, or 30°.

In some embodiments the bevel angle is:

a) between 11° and 12°, 11.1° and 11.9°, 11.2° and 11.8°, 11.3° and 11.7°;
b) between 11.5° and 11.8°, 11.55° and 11.75°, or between 11.6° and 11.7°;
c) about 11°, about 11.2°, about 11.4°, about 11.6°, about 11.8°, or about 12°;
d) 11°, 11.2°, 11.4°, 11.6°, 11.8°, or 12°; and/or
e) 11.64°.

In some embodiments, the bevel angle is between 16° and 26°, between 18° and 24°, more particularly between 20° and 22°. In some embodiments, the bevel angle is 20.9°.

In some embodiments, the DNA-based nanostructure comprises at least one set of self-assembling DNA based-building blocks, wherein one, two, or all three side trapezoids comprises a specific pattern of recesses and/or extrusions formed by missing or additional DNA double helical stretches for specific interaction with a complementary pattern on the side trapezoid of another one of said self-assembling DNA-based building blocks. In some embodiments, the DNA-based nanostructure comprises at least one set of self-assembling DNA based-building blocks, wherein two side trapezoids comprises a specific pattern of recesses and/or extrusions formed by missing or additional DNA double helical stretches for specific interaction with a complementary pattern on the side trapezoid of another one of said self-assembling DNA-based building blocks, and one side trapezoid does not comprise said specific pattern of recesses and/or extrusions formed by missing or additional DNA double helical stretches.

The presence of the specific pattern of recesses and/or extrusions formed by missing or additional DNA double helical stretches on a side trapezoid of a first self-assembling DNA-based building block allows base stacking interactions between two side trapezoids. The base stacking interactions render said side trapezoid capable of specifically interacting with the side trapezoid of a second self-assembling DNA-based building block, permitting dimerization of the said first and second self-assembling DNA-based building blocks. In this way, a first self-assembling DNA-based building block comprising two side trapezoids comprises a specific pattern of recesses and/or extrusions formed by missing or additional DNA double helical stretches for specific interaction with a complementary pattern on the side trapezoid of another one of said self-assembling DNA-based building blocks, and one side trapezoid does not comprise said specific pattern of recesses and/or extrusions formed by missing or additional DNA double helical stretches, is capable of dimerising with a second self-assembling DNA-based building block along one side trapezoid, and a third DNA-based building block along a second side trapezoid. Because the third side-trapezoid does not comprise a specific pattern of recesses and/or extrusions formed by missing or additional DNA double helical stretches, the first self-assembling DNA-based building block cannot dimerise with a fourth self-assembling DNA-based building block along the third side-trapezoid.

The strength of base stacking interactions between side trapezoids may be tuned, as disclosed for example in Sigl, C., 2021, Programmable DNA Origami Shell Platform for Virus Neutralization [Doctoral thesis, Technische Universität München], available at: https://mediatum.ub.tum.de/1625147 (herein incorporated by reference in its entirety). For example, the base stacking between a first and second side trapezoid may:

a) be strengthened by introducing at least one single-stranded overhang in a double helix of a first protrusion or recess of a first side trapezoid, that is complementary to the double helix of a first recess or protrusion (respectively) of a second side trapezoid;
b) be weakened by creating single-stranded overhangs in the double helix of a protrusion or recess of a first side trapezoid and/or in the double helix of a recess or protrusion (respectively) of a second side trapezoid, wherein the single-stranded in the double helix of a protrusion or recess of a first side trapezoid are not complementary and/or cannot anneal to the single-stranded overhangs in the double helix of a recess or protrusion (respectively) of a second side trapezoid; and/or
c) be deactivated by adding single-stranded nucleic acids consisting thymine to each strand in the double helix of a protrusion or recess of a first side trapezoid and/or in the double helix of a recess or protrusion (respectively) of a second side trapezoid, thereby preventing base stacking between the first and second side trapezoids.

In some embodiments, the nanostructure comprises or consists of self-assembling DNA-based building blocks

wherein each self-assembling DNA-based building blocks each has a triangular prismoid shape, and wherein each self-assembling DNA-based building block comprises two side trapezoids comprising a specific pattern of recesses and/or extrusions formed by missing or additional DNA double helical stretches for specific interaction with a complementary pattern on the side trapezoid of another one of said self-assembling DNA-based building blocks, and one side trapezoid that does not comprise said specific pattern of recesses and/or extrusions formed by missing or additional DNA double helical stretches.

In some embodiments, the DNA-based nanostructure is formed from:

a) a homopentamer of self-assembling DNA-based building blocks with a triangular prismoid shape, wherein the DNA-based nanostructure has a substantially pentagonal outline in one plane;
b) a homohexamer of self-assembling DNA-based building blocks with a triangular prismoid shape, wherein the DNA-based nanostructure has a substantially hexagonal outline in one plane; or
c) a homoheptamer of self-assembling DNA-based building blocks with a triangular prismoid shape, wherein the DNA-based nanostructure has a substantially heptagonal outline in one plane.

Different viruses have a range of sizes. Accordingly, for a defined arc length along the virus surface, a first larger virus will have a smaller degree of curvature or curvature than a second smaller virus for the same defined arc length. Accordingly, the degree of curvature or curvature of the concave surface of the DNA-based nanostructure may be tuned to the size of the target virus. For example, where the target virus is a relatively large virus, the concave surface of the DNA-based nanostructure may suitably have relatively small degree of curvature or curvature. Similarly, where the target virus is a relatively small virus, the concave surface of the DNA-based nanostructure may suitably have a relatively large degree of curvature or curvature. The person skilled in the art is aware of the sized of different viruses, and is therefore able to understand the terms "large virus", "relatively large virus", "small virus", and "relatively small virus".

A large virus may have an exemplary diameter of at least 200 nm. For example, a large virus may have a diameter of 200 nm, about 300 nm, about 400 nm, about 500 nm, about 600 nm, about 700 nm, about 800 nm, about 900 nm, about 1000 nm, or more. Exemplary large viruses include mimivirus and parovirus, which have a diameter of about 500 nm to about 1000 nm.

A relatively large virus may have a diameter that is larger than an average virus, but which is not the same as a large virus. For example, a relatively large virus may have an exemplary diameter of between 100 nm and 200 nm. Exemplary relatively large viruses include influenza virus, which may have a diameter of about 100 nm to about 150 nm, and herpesviruses, which may have a diameter of about 150 nm to about 200 nm.

A relatively small virus may have a diameter that is smaller than average, but which is not the same as a small virus. For example, a relatively small virus may have an exemplary diameter of between 50 nm and 100 nm. Exemplary relatively small viruses include flaviviruses, which may have a diameter of about 40 nm to about 60 nm, and adenoviruses, which may have a diameter of about 70 nm to about 90 nm.

A small virus may have a diameter of less than 50 nm or up to 50 nm. Exemplary small viruses include parovirus, which may have a diameter of about 18 nm to about 26 nm, and picornavirus, which may have a diameter of about 22 nm to about 30 nm.

In some embodiments, the concave surface of the DNA-based nanostructure has a curvature (k) of:

a) at least 10 $\mu m^{-1}$, at least 20 $\mu m^{-1}$, at least 40 $\mu m^{-1}$, at least 60 $\mu m^{-1}$, at least 80 $\mu m^{-1}$, 100 $\mu m^{-1}$;
b) about 10 $\mu m^{-1}$, about 20 $\mu m^{-1}$, about 40 $\mu m^{-1}$, about 60 $\mu m^{-1}$, about 80 $\mu m^{-1}$, 100 $\mu m^{-1}$; and/or
c) 10 $\mu m^{-1}$, 20 $\mu m^{-1}$, 40 $\mu m^{-1}$, 60 $\mu m^{-1}$, 80 $\mu m^{-1}$, 100 $\mu m^{-1}$.

In some embodiments, the concave surface of the DNA-based nanostructure has a curvature (k) of 60 $\mu m^{-1}$.

The skilled person is aware of methods for calculating curvature (k). In some embodiments:

$$k = \frac{1}{R}$$

Where R is the radius of a given circle, sphere, half-sphere, or hemisphere, for example the radius of a circle that has the same radius as the DNA-based nanostructure provided herein.

In some embodiments, the DNA-based nanostructure has a diameter of:

a) at least 60 nm, at least 75 nm, at least 80 nm, or at least 100 nm;
b) about 60 nm, about 75 nm, about 80 nm, or about 100 nm; and/or
c) 60nm, 75 nm, 80 nm or 100 nm.

In some embodiments, the DNA-based nanostructure has a molecular mass of:

a) at least 18 MDa, at least 19 MDa, at least 20 MDa, at least 23 MDa, at least 25 MDa, at least 27 MDa, at least 30 MDa, at least 35 MDa, or more;
b) about 18 MDa, about 19 MDa, about 20 MDa, about 23 MDa, about 25 MDa, about 27 MDa, about 30 MDa, or about 35 MDa; and/or
c) 18 MDa, 19 MDa, 20 MDa, 23 MDa, 25 MDa, 27 MDa, 30 MDa, or 35 MDa.

In preferred embodiments the DNA-nanoparticle is not planar or is not substantially planar.

In some embodiments, the DNA-based nanostructure comprises a single-stranded DNA template strand; a set of oligonucleotides that are at least partially complementary to said single-stranded DNA template; handle sites; and/or handle-binding oligonucleotides with sequences of SEQ ID NO: 1-330 as set out herein and in **Table 1.** In some embodiments, the DNA-based nanostructure comprises a single-stranded DNA template strand; a set of oligonucleotides that are at least partially complementary to said single-stranded DNA template; handle sites; and/or handle-binding oligonucleotides with sequences with:

a) at least 80%, at least 90%, at least 95%, at least 96%, at least 97%, at least 98%, at least 99% or more sequence identity to SEQ ID NO: 1-330 as set out herein and in **Table 1;**
b) about 80%, about 90%, about 95%, about 96%, about 97%, about 98%, about 99% sequence identity to SEQ ID NO: 1-330 set out herein and in **Table 1**; and/or
c) 80%, 90%, 95%, 96%, 97%, 98%, 99% or more sequence identity to SEQ ID NO: 1-330 set out herein and in **Table 1.**

In some embodiments, the self-assembling DNA-based building blocks each comprise a single-stranded DNA template strand; a set of oligonucleotides that are at least partially complementary to said single-stranded DNA template; handle sites; and/or handle-binding oligonucleotides with sequences of SEQ ID NO: 1-330 set out herein and in **Table 1.** In some embodiments, the self-assembling DNA-based building blocks comprise a single-stranded DNA template strand; a set of oligonucleotides that are at least partially complementary to said single-stranded DNA template; handle sites; and/or handle-binding oligonucleotides with sequences with:

a) at least 80%, at least 90%, at least 95%, at least 96%, at least 97%, at least 98%, at least 99% or more sequence identity to SEQ ID NO: 1-330 set out herein and in **Table 1;**
b) about 80%, about 90%, about 95%, about 96%, about 97%, about 98%, about 99% sequence identity to SEQ ID NO: 1-330 set out herein and in **Table 1**; and/or
c) 80%, 90%, 95%, 96%, 97%, 98%, 99% or more sequence identity to SEQ ID NO: 1-330 set out herein and in **Table 1.**

In some embodiments, the DNA-based nanostructure is a shell that defines a cavity at least partially enclosed by the DNA-based nanostructure.

In some embodiments, the cavity is fully enclosed, for example in some embodiments the DNA-based nanostructure is a closed sphere. In some embodiments, the cavity is an open cavity, for example in some embodiments the DNA-based nanostructure is an open sphere such as a half-sphere.

The enclosed cavity may be any size suitable for trapping a target virus and may be tailored to the size of a target virus particle. In some embodiments, the cavity has a diameter of:

a) at least 20 nm; at least 30 nm; at least 40 nm; at least 50 nm; at least 60 nm; at least 70 nm; at least 80 nm; at least 90 nm; at least 100 nm; at least 110 nm; at least 120 nm; at least 130 nm; at least 140 nm; at least 150 nm; at least 160 nm; at least 170 nm; at least 180 nm; at least 190 nm; at least 200 nm; at least 250 nm; at least 300 nm; at least 350 nm; at least 400 nm at least 450 nm; at least 500 nm; at least 550 nm; at least 600 nm; at least 700 nm; at least 800 nm; at least 900 nm; or more;
b) about 20 nm; about 30 nm; about 40 nm; about 50 nm; about 60 nm; about 70 nm; about 80 nm; about 90 nm; about 100 nm; about 110 nm; about 120 nm; about 130 nm; about 140 nm; about 150 nm; about 160 nm; about 170 nm; about 180 nm; about 190 nm; about 200 nm; about 250 nm; about 300 nm; about 350 nm; about 400 nm about 450 nm; about 500 nm; about 550 nm; about 600 nm; about 700 nm; about 800 nm; about 900 nm; or about 1000 nm; and/or
c) 20 nm; 30 nm; 40 nm; 50 nm; 60 nm; 70 nm; 80 nm; 90 nm; 100 nm; 110 nm; 120 nm; 130 nm; 140 nm; 150 nm; 160 nm; 170 nm; 180 nm; 190 nm; 200 nm; 250 nm; 300 nm; 350 nm; 400 nm 450 nm; 500 nm; 550 nm; 600 nm; 700 nm; 800 nm; 900 nm; or 1000 nm.

In some embodiments, the cavity has a diameter of at most 1000 nm.

In some embodiments, the DNA-based nanostructure has a molecular mass of:

a) at least 1 MDa, at least 10 MDa, at least 20 MDa, at least 30 MDa, at least 40 MDa, at least 50 MDa, at least 60 MDa, at least 70 MDa, at least 80 MDa, at least 90 MDa, at least 100 MDa, at least 150 MDa, at least 200 MDa, at least 250 MDa, at least 300 MDa, at least 350 MDa, at least 400 MDa, at least 450 MDa, at least 500 MDa, at least 600 MDa, at least 700 MDa, at least 800 MDa, at least 900 MDa, at least 1000 MDa, at least 1100 MDa, at least 1200 MDa, at least 1300 MDa, at least 1400 MDa, or more;
b) about 1 MDa, about 10 MDa, about 20 MDa, about 30 MDa, about 40 MDa, about 50 MDa, about 60 MDa, about 70 MDa, about 80 MDa, about 90 MDa, about 100 MDa, about 150 MDa, about 200 MDa, about 250 MDa, about 300 MDa, about 350 MDa, about 400 MDa, about 450 MDa, about 500 MDa, about 600 MDa, about 700 MDa, about 800 MDa, about 900 MDa, about 1000 MDa, about 1100 MDa, about 1200 MDa, about 1300 MDa, about 1400 MDa, or about 1500 MDa; and/or
c) 1 MDa, 10 MDa, 20 MDa, 30 MDa, 40 MDa, 50 MDa, 60 MDa, 70 MDa, 80 MDa, 90 MDa, 100 MDa, 150 MDa, 200 MDa, 250 MDa, 300 MDa, 350 MDa, 400 MDa, 450 MDa, 500 MDa, 600 MDa, 700 MDa, 800 MDa, 900 MDa, 1000 MDa, 1100 MDa, 1200 MDa, 1300 MDa, 1400 MDa, or 1500 MDa.

In some embodiments, the DNA-based nanostructure has a molecular mass of at most 1,500 MDa.

In some embodiments, the ratio between the numerical value of the molecular mass of the DNA-based nanostructure (in MDa) and the numerical value of the volume of the cavity encased by said DNA-based nanostructure (in $nm^3$) is less than 10,000, particularly less than 9,000. In some embodiments said ratio has a value of between 1,000 and 10,000, particularly between 2,000 and 9,000. For example, in the case of certain nanostructures, where the molecular mass is about 40 MDa, and where the encased volume is about 113,000 $nm^3$, said ratio is about 2,800.

In some embodiments, the ratio between the outer surface area of the DNA-based nanostructure covered by the macromolecules forming said DNA-based nanostructure and the outer surface area not covered by said macromolecules (excluding the area of the opening of a DNA-based nanostructure in the form of a shell) is:

a) at least 1, at least 2, at least 4, at least 6, at least 8. at least 10, at least 12, at least 14, at least 16, or higher;
b) about 1, about 2, about 4, about 6, about 8. about 10, about 12, about 14, about 16, or about 18; and/or
c) 1, 2, 4, 6, 8. 10, 12, 14, 16, or 18.

In some embodiments, the ratio is between 1 and 20, in particular between 2 and 18, between 4 and 16, between 6 and 14, and more particularly between 8 and 12.

For example, in a case where the DNA-based nanostructure is a shell in the form of a half sphere, only the area of the curved surface, but not that of the opening, *i.e.,* the area of the flat face of the half sphere, is used for calculating said ratio.

In some embodiments:

a) the at least first plurality of virus-binding moieties is bound to the cavity facing surface of the shell; optionally wherein when present the second plurality of virus-binding moieties, third plurality of virus-binding moieties, fourth plurality of virus binding moieties, fifth plurality of virus binding moieties, and/or sixth plurality of virus binding moieties are bound the cavity facing surface of the shell; and/or
b) when present, the at least a first plurality of recruitment moieties is bound to the exterior surface.

In some embodiments, the nanostructure has a closed three-dimensional geometric shape, in particular a closed three-dimensional geometric shape selected from a sphere, a spherocylinder, and a polyhedron, in particular a tetrahedron, an octahedron or an icosahedron.

In some embodiments, the nanostructure is a shell with an opening for accessing the cavity.

In some embodiments, the nanostructure is a combination of a first and a second subshell, each with an opening to access a first and a second inner cavity, respectively, wherein said first and said second inner cavity together form said cavity, in particular wherein said first and said second subshell are connected by at least one linker.

In some embodiments, the nanostructure is based on an icosahedral structure.

In some embodiments, each of said self-assembling DNA-based building blocks is a triangular and/or a rectangular prismoid, particularly a triangular prismoid.

In some embodiments, each said triangular and/or a rectangular prismoid is formed by m triangular, or rectangular, respectively, planes, wherein m is an integer independently selected from 4, 5, 6, 7 and 8, in particular independently selected from 5, 6 and 7, more particularly wherein said integer is 6;

the three, or four, respectively, edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4;

wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes; and

wherein at least two of the three, or four, respectively, side trapezoids comprise a specific pattern of recesses and/or extrusions formed by missing or additional DNA double helical stretches for specific interaction with a complementary pattern on the side trapezoid of another one of said self-assembling DNA-based building blocks.

In some embodiments, for at least part of the self-assembling DNA-based building blocks the length of at least one edge of each of said m planes is decreasing from the first to the $m^{th}$ plane, so that bevel angles results between planes perpendicular to said first plane and each of the trapezoid planes formed by said m edges.

In some embodiments, the DNA-based nanostructure comprises at least one set of self-assembling DNA based-building blocks, wherein all three or four, respectively, side trapezoids comprises a specific pattern of recesses and/or extrusions formed by missing or additional DNA double helical stretches for specific interaction with a complementary pattern on the side trapezoid of another one of said self-assembling DNA-based building blocks.

In some embodiments, the DNA-based nanostructure is a half shell selected from:

(a) a half octahedron DNA-based nanostructure based on T_octa self-assembling DNA-based building blocks, which consists of a set of four copies of a triangular frustum, wherein the base-pair stacking contacts on one of the triangular edges of the triangular frustum are inactivated by either strand shortening or by adding unpaired thymidines;

(b) a T=1 half shell based on T1_pentamer triangle self-assembling DNA-based building blocks, which consists of two sets of in each case five copies of two different triangular frusta, wherein the five copies of the first set form a closed pentamer, and the five copies of the second set dock onto the edges of said pentamer; and

(c) a "trap" T=1 half shell with a missing pentagon vertex based on a combination of T1_pentamer triangle and T1_ring triangle self-assembling DNA-based building blocks, which consists of three sets of in each case five copies of three different triangular frusta, wherein the five copies of the first set form a closed pentamer, the five copies of the second set dock onto the edges of said pentamer the five copies of the second set dock onto the edges of said pentamer, and the five copies of the third set dock into the gaps between the five copies of said second set;

(d) a T=3 icosahedral half shell based on T3_6_triangle based self-assembling DNA-based building blocks, which consists of a total of 30 triangular subunits partitioned as five copies of six different full-size DNA triangle designs with specific edge docking rules.

In some embodiments, the DNA-based nanostructure further comprises:

(a) one or more types of DNA brick constructs, each type of such DNA brick constructs being characterized by one or more interaction sites for specific interaction by edge-to-edge stacking contacts with one or more complementary interaction sites present on the plane of a triangular or rectangular frustum on the outer surface of said DNA-based nanostructure, wherein said DNA brick constructs cover the free space between the three, or four, respectively, edges of said plane;

(b) one or more cross-linkages within one of said triangular or rectangular prismoids, and/or between two of said triangular and/or rectangular prismoids; and/or

(c) at least one moiety specifically interacting with said viruses or viral particles.

In some embodiments, the nanostructure is formed *in situ* from the self-assembling DNA-based building blocks in the presence of the target virus.

In some embodiments, the DNA-based nanostructure is a nanostructure described in International Patent Application No. PCT/EP2021/054307, published as WO 2021/165528 A1 (herein incorporated by reference in its entirety). In some embodiments, the DNA-based nanostructure is a nanostructure described in International Patent Application No. PCT/EP2023/061264, published as WO 2023/209161 A1 (herein incorporated by reference in its entirety). In some embodiments, the DNA-based nanostructure comprises and/or each self-assembling DNA-based building block comprises a single-stranded DNA template strand; a set of oligonucleotides that are at least partially complementary to said single-stranded DNA template; handle sites; and/or handle-binding oligonucleotides with a sequence selected from the single-stranded DNA template strand; oligonucleotides that are at least partially complementary to said single-stranded DNA template ("staple strands"); handle sites; and/or handle-binding oligonucleotides set out in SEQ ID NOs: 1-4095 and Table 1 of International Patent Application No. PCT/EP2021/054307, published as WO 2021/165528 A1 (herein incorporated by reference in its entirety). In some embodiments, the DNA-based nanostructure comprises and/or each self-assembling DNA-based building block comprises a single-stranded DNA template strand; a set of oligonucleotides that are at least partially complementary to said single-stranded DNA template; handle sites; and/or handle-binding

oligonucleotides with a sequence selected from the single-stranded DNA template strand; oligonucleotides that are at least partially complementary to said single-stranded DNA template ("staple strands"); handle sites; and/or handle-binding oligonucleotides set out in SEQ ID NOs: 1-1821 and Tables 1-10 of International Patent Application No. PCT/EP2023/061264, published as WO 2023/209161 A1 (herein incorporated by reference in its entirety). In some embodiments, the DNA-based nanostructure and/or each self-assembling DNA-based building block comprises a single-stranded DNA template strand; a set of oligonucleotides that are at least partially complementary to said single-stranded DNA template; handle sites; and/or handle-binding oligonucleotides with a sequence that has:

a) at least 80%, at least 90%, at least 95%, at least 96%, at least 97%, at least 98%, at least 99% or more sequence identity to to the above sequences;
b) about 80%, about 90%, about 95%, about 96%, about 97%, about 98%, about 99% sequence identity to the above sequences; and/or
c) 80%, 90%, 95%, 96%, 97%, 98%, 99% or more sequence identity to the above sequences.

In some embodiments, the DNA-based nanostructure is a three-dimensional open shell defining a cavity and comprising an opening for accessing said cavity, comprising:
an n-gonal pyramid formed by n identical copies of a first type of an acute isosceles triangular prismoid t1, wherein n is an integer selected from 7, 8, 9, 10, 11, 12, 13, 14 and 15, wherein the base plane of each prismoid points to the outside of said open shell, and the upper plane points to said cavity, wherein the two large side planes of each prismoid contain a first pattern and a second pattern of one or more protrusions and/or one or more receptacles, wherein said first and said second patterns are complementary to each other, and wherein the small side plane comprises a third pattern of one or more protrusions and/or one or more receptacles; wherein said first type of an acute isosceles triangular prismoid is a self-assembling DNA-based building block.

In some embodiments, the DNA-based nanostructure further comprises n copies of a second type of an acute isosceles triangular prismoid t2, wherein a first side of each prismoid points to the outside of said open shell, and the opposite side points to said cavity and/or to said opening for accessing said cavity, wherein one plane of said second type of prismoid structure comprises a fourth pattern of one or more protrusions and/or one or more receptacles which is complementary to said third pattern. In some embodiments, n is an integer selected from 9, 10, 11, 12 and 13.

In some embodiments, the DNA-based nanostructure further comprises chemical crosslinks between different prismoids; optionally wherein said chemical crosslinks are obtained by UV irradiation or UV welding.

In some embodiments, the DNA-based nanostructure is a nanostructure described in International Patent Application No. PCT/EP2023/080373, published as WO 2024/094708 A1 (herein incorporated by reference in its entirety). In some embodiments, the DNA-based nanostructure comprises and/or the each self-assembling DNA-based building block comprises a single-stranded DNA template strand; a set of oligonucleotides that are at least partially complementary to said single-stranded DNA template; handle sites; and/or handle-binding oligonucleotides with a sequence selected from the single-stranded DNA template strand; oligonucleotides that are at least partially complementary to said single-stranded DNA template ("staple strands"); handle sites; and/or handle-binding oligonucleotides set out in SEQ ID NOs: 1-1408 and Tables 1-3 of International Patent Application No. PCT/EP2023/080373, published as WO 2024/094708 A1 (herein incorporated by reference in its entirety). In some embodiments, the DNA-based nanostructure and/or each self-assembling DNA-based building blocks comprises a single-stranded DNA template strand; a set of oligonucleotides that are at least partially complementary to said single-stranded DNA template; handle sites; and/or handle-binding oligonucleotides with a sequence that has:

a) at least 80%, at least 90%, at least 95%, at least 96%, at least 97%, at least 98%, at least 99% or more sequence identity to to the above sequences;
b) about 80%, about 90%, about 95%, about 96%, about 97%, about 98%, about 99% sequence identity to the above sequences; and/or
c) 80%, 90%, 95%, 96%, 97%, 98%, 99% or more sequence identity to the above sequences.

In some embodiments, the DNA-based nanostructure is a nanostructure described in Sigl, C., 2021, Programmable DNA Origami Shell Platform for Virus Neutralization [Doctoral thesis, Technische Universität München], available at: https://mediatum.ub.tum.de/1625147 (herein incorporated by reference in its entirety). In some embodiments, each self-assembling DNA-based building block is a self-assembling DNA based building block described in Sigl, C., 2021, Programmable DNA Origami Shell Platform for Virus Neutralization [Doctoral thesis, Technische Universität München], available at: https://mediatum.ub.tum.de/1625147 (herein incorporated by reference in its entirety).

A DNA-based building block

In one aspect, provided herein is a self-assembling DNA-based building block formed by a single-stranded DNA template strand and a set of oligonucleotides complementary to said single-stranded DNA template, wherein:

each of the oligonucleotides is either complementary to one contiguous DNA sequence stretch or to at least two non-contiguous DNA sequence stretches on said single-stranded DNA template; and
the self-assembling DNA-based building block comprises at least one first virus-binding moiety, wherein the first virus-binding moiety is neuraminic acid or a derivative thereof.

It will be appreciated that a self-assembling DNA-based building block may be formed in any way that is suitable for producing 3-dimensional DNA structures. For example, the self-assembling DNA-based building block may comprise, for example, an extended lattice or DNA origami, which are known in the art. Preferably, the self-assembling DNA-based building block is formed from DNA origami.

Any suitable single-stranded DNA template strand and/or oligonucleotides that are at least partially complementary to said single-stranded DNA template may be used. For example, the single-stranded DNA template strand and/or oligonucleotides that are at least partially complementary to said single-stranded DNA template may be chemically synthesised, or may derived, for example, from an organism such as a bacterium, a fungus, an archaeon, or a bacteriophage. In some embodiments, the single-stranded DNA template strand and/or oligonucleotides that are at least partially complementary to said single-stranded DNA template is single-stranded DNA from a filamentous bacteriophage or is derived from single-stranded DNA of a filamentous bacteriophage. In some embodiments, the single-stranded DNA template strand and/or oligonucleotides that are at least partially complementary to said single-stranded DNA template is single-stranded DNA are obtained from different sources. For example, in some embodiments, the single-stranded DNA template strand is single-stranded DNA from a filamentous bacteriophage or is derived from single-stranded DNA of a filamentous bacteriophage, and the oligonucleotides that are at least partially complementary to said single-stranded DNA template is single-stranded DNA is chemically synthesised. Methods of chemically synthesising single-stranded DNA is known to the skilled person. In some embodiments, the single-stranded DNA template strand and/or oligonucleotides that are at least partially complementary to said single-stranded DNA template is single-stranded DNA are obtained from the same source, optionally from the same filamentous bacteriophage or is derived from single-stranded DNA of the same filamentous bacteriophage, for example using the methods disclosed in Praetorius et al., 2017, "Biotechnological mass production of DNA origami" Nature, 552:84-87, and International Patent Application No. PCT/EP2017/068051, published as WO 2018/054571 (each of which is herein incorporated by reference in its entirety).

In some embodiments, the single-stranded DNA template has a sequence of SEQ ID NO: 1 of International Patent Application No. PCT/EP2023/080373, published as WO 2024/094708 A1; SEQ ID NO: 1 of International Patent Application No. PCT/EP2023/061264, published as WO 2023/209161 A1; SEQ ID NOs: 1 or 2 of International Patent Application No. PCT/EP2021/054307, published as WO 2021/165528 A1; or a sequence with:

a) at least 80%, at least 90%, at least 95%, at least 96%, at least 97%, at least 98%, at least 99% or more sequence identity thereto;
b) about 80%, about 90%, about 95%, about 96%, about 97%, about 98%, about 99% sequence identity thereto; and/or
c) 80%, 90%, 95%, 96%, 97%, 98%, 99% or more sequence identity thereto.

In some embodiments, the single-stranded DNA-template has a sequence of SEQ ID NO: 1 or SEQ ID NO: 2 set out herein. In some embodiments, the single-stranded DNA-template has a sequence with:

a) at least 80%, at least 90%, at least 95%, at least 96%, at least 97%, at least 98%, at least 99% or more sequence identity to SEQ ID NO: 1 or SEQ ID NO: 2 set out herein;
b) about 80%, about 90%, about 95%, about 96%, about 97%, about 98%, about 99% sequence identity to SEQ ID NO: 1 or SEQ ID NO: 2 set out herein; and/or
c) 80%, 90%, 95%, 96%, 97%, 98%, 99% or more sequence identity to SEQ ID NO: 1 or SEQ ID NO: 2 set out herein.

In some embodiments, the oligonucleotides that are at least partially complementary to said single-stranded DNA template each have a sequence selected from SEQ ID NOs: 2-1407 of International Patent Application No. PCT/EP2023/080373, published as WO 2024/094708 A1; SEQ ID NOs: 2-1820 of International Patent Application No. PCT/EP2023/061264, published as WO 2023/209161 A1; SEQ ID NOs: 3-4089 of International Patent Application No. PCT/EP2021/054307, published as WO 2021/165528 A1; or a sequence with:

a) at least 80%, at least 90%, at least 95%, at least 96%, at least 97%, at least 98%, at least 99% or more sequence identity thereto;
b) about 80%, about 90%, about 95%, about 96%, about 97%, about 98%, about 99% sequence identity thereto; and/or
c) 80%, 90%, 95%, 96%, 97%, 98%, 99% or more sequence identity thereto.

In some embodiments, the oligonucleotides that are at least partially complementary to said single-stranded DNA template each have a sequence selected from SEQ ID NOs: 3-323 set out in **Table 1** herein. In some embodiments, the oligonucleotides that are at least partially complementary to said single-stranded DNA template each have a sequence selected from a sequence with:

a) at least 80%, at least 90%, at least 95%, at least 96%, at least 97%, at least 98%, at least 99% or more sequence identity to SEQ ID NO: 3-323 set out in **Table 1** herein;
b) about 80%, about 90%, about 95%, about 96%, about 97%, about 98%, about 99% sequence identity to SEQ ID NO: 3-323 set out in **Table 1** herein; and/or
c) 80%, 90%, 95%, 96%, 97%, 98%, 99% or more sequence identity to SEQ ID NO: 3-323 set out in **Table 1** herein.

In some embodiments:

a) the single-stranded DNA template has:

i) a sequence of SEQ ID NO: 1 set out herein;
ii) a sequence with at least 80%, at least 90%, at least 95%, at least 96%, at least 97%, at least 98%, at least 99% or more sequence identity to SEQ ID NO: 1 set out in herein;
iii) a sequence with about 80%, about 90%, about 95%, about 96%, about 97%, about 98%, about 99% sequence identity to SEQ ID NO: 1 set out in herein; and/or
iv) a sequence with 80%, 90%, 95%, 96%, 97%, 98%, 99% or more sequence identity to SEQ ID NO: 1 set out in herein;

and/or
b) the oligonucleotides that are at least partially complementary to said single-stranded DNA template each have:

i) a sequence set out in SEQ ID NOs: 3-131 set out in **Table 1;**
ii) a sequence with at least 80%, at least 90%, at least 95%, at least 96%, at least 97%, at least 98%, at least 99% or more sequence identity to SEQ ID NO: 3-131 set out in **Table 1** herein;

b) a sequence with about 80%, about 90%, about 95%, about 96%, about 97%, about 98%, about 99% sequence identity to SEQ ID NO: 3-131 set out in **Table 1** herein; and/or
c) a sequence with 80%, 90%, 95%, 96%, 97%, 98%, 99% or more sequence identity to SEQ ID NO: 3-131 set out in **Table 1** herein.

In some embodiments:

a) the single-stranded DNA template has:

i) a sequence of SEQ ID NO: 2 set out herein;
ii) a sequence with at least 80%, at least 90%, at least 95%, at least 96%, at least 97%, at least 98%, at least 99% or more sequence identity to SEQ ID NO: 2 set out in herein;
iii) a sequence with about 80%, about 90%, about 95%, about 96%, about 97%, about 98%, about 99% sequence identity to SEQ ID NO: 2 set out in herein; and/or
iv) a sequence with 80%, 90%, 95%, 96%, 97%, 98%, 99% or more sequence identity to SEQ ID NO: 2 set out in herein;

and/or
b) the oligonucleotides that are at least partially complementary to said single-stranded DNA template each have:

i) a sequence set out in SEQ ID NOs: 132-323 set out in **Table 1;**
ii) a sequence with at least 80%, at least 90%, at least 95%, at least 96%, at least 97%, at least 98%, at least 99% or

more sequence identity to SEQ ID NO: 132-323 set out in **Table 1** herein;

b) a sequence with about 80%, about 90%, about 95%, about 96%, about 97%, about 98%, about 99% sequence identity to SEQ ID NO: 132-323 set out in **Table 1** herein; and/or
c) a sequence with 80%, 90%, 95%, 96%, 97%, 98%, 99% or more sequence identity to SEQ ID NO: 132-323 set out in **Table 1** herein.

As will be appreciated, the self-assembling DNA-based building block may advantageously comprise more than one type of virus-binding agent. Accordingly, in one embodiment, the self-assembling DNA-based building block comprises at least one second virus binding moiety; at least one second virus-binding moiety; at least one third virus-binding moiety; at least one fourth virus-binding moiety; at least one fifth virus-binding moiety; and/or at least one sixth virus-binding moiety.

In some embodiments, the self-assembling DNA-based building block comprises at least a plurality of first virus-binding moieties that is a first plurality of virus-binding moieties; optionally wherein the building block further comprises a second plurality of virus-binding moieties, a third plurality of virus-binding moieties, a fourth plurality of virus binding moieties, a fifth plurality of virus binding moieties, and/or a sixth plurality of virus binding moieties.

Suitable second, third, fourth, fifth, and/or sixth virus binding moieties, and/or virus-binding moieties of the second, and when present, third, fourth, fifth, and/or sixth pluralities of virus binding moieties include any virus binding moiety capable of binding to a ligand on the surface of the influenza virus. For example, the ligand may suitably be selected from the group comprising or consisting of: haemagglutinin (HA), neuraminidase (NA), M2 ion channel, and viral envelope. In some embodiments, the second, third, fourth, fifth, and/or sixth virus binding moieties, and/or virus binding moieties of the second, and when present, third, fourth, fifth, and/or sixth pluralities of virus binding moieties are selected from the group comprising or consisting of: neuraminic acid or a derivative thereof; an antibody or a virus-binding fragment thereof, a nanobody or a virus-binding fragment thereof, an affimer, heparin, heparan sulfate, hybrid heparan sulfates, a host receptor protein or virus-binding fragment thereof, a de novo virus-binding peptide, an aptamer, and a neuraminidase inhibitor; optionally wherein the neuraminidase inhibitor is selected from the group comprising or consisting of: oseltamivir, zanamivir, ianamivir, and peramivir; optionally wherein the neuraminidase inhibitor is oseltamivir. Suitably, the antibody or virus-binding fragment thereof, nanobody or virus-binding fragment thereof, and/or affimer are capable of binding to a ligand selected from the group comprising or consisting of: haemagglutinin (HA), neuraminidase (NA), M2 ion channel, and viral envelope.

In some embodiments, each virus-binding moiety or virus-binding moieties of each plurality of virus binding moieties is different to every other virus binding moiety or the virus-binding moieties of every other plurality of virus-binding moieties. In some embodiments, each virus-binding moiety or plurality of virus-binding moieties may comprise the same virus binding moiety. In some embodiments, each virus-binding moiety or plurality virus-binding moieties may comprise neuraminic acid or a derivative thereof, where each virus-binding moiety or plurality comprises a different neuraminic acid or derivative thereof; optionally wherein the virus-binding moieties or virus-binding moieties of each plurality are selected from the group comprising or consisting of: neuraminic acid; sialic acid; sialyllactose including 3'-sialyllactose, 6'-sialyllactose, 2,3'-sialyllactose, and 2,6'-sialyllactose; N-acetylneruaminic acid; 2-keto-deoxynonic acid; 6'-sialyl-D-lactose; 3'- β -sialyl-β-lactose; N-glycolylneuraminic acid; and the following neuraminic acid derivatives disclosed in Song et al., 2011, "A Sialylated Glycan Microarray Reveals Novel Interactions of Modified Sialic Acids with Proteins and Viruses", J Biol. Chem, 286(36):31610-31622 (herein incorporated by reference in its entirety): Neu5Ac; Nau5Ac8Me; Neu5Ac9Me; Neu5,9$Ac_2$; Neu5Ac9Lt; Neu5Gc; Neu5GcMe; Neu5Gc9Ac; Neu5GcAc; Kdn; Kdn9Ac; Kdn5Ac; Kdn,9$Ac_2$; Kdn9Me; Kdn5Me; and Kdn7Me.. In some embodiments, at least two virus-binding moieties or at least two of the pluralities of the virus-binding moiety are different neuraminic acids or are derivatives thereof; optionally wherein the virus-binding moieties of each plurality are selected from the group comprising or consisting of: neuraminic acid; sialic acid; sialyllactose including 3'-sialyllactose, 6'-sialyllactose, 2,3'-sialyllactose, and 2,6'-sialyllactose; N-acetylneruaminic acid; 2-keto-deoxynonic acid; 6'-sialyl-D-lactose; 3'- β -sialyl-β-lactose; N-glycolylneuraminic acid; and the following neuraminic acid derivatives disclosed in Song et al., 2011, "A Sialylated Glycan Microarray Reveals Novel Interactions of Modified Sialic Acids with Proteins and Viruses", J Biol. Chem, 286(36):31610-31622 (herein incorporated by reference in its entirety): Neu5Ac; Nau5Ac8Me; Neu5Ac9Me; Neu5,9$Ac_2$; Neu5Ac9Lt; Neu5Gc; Neu5GcMe; Neu5Gc9Ac; Neu5GcAc; Kdn; Kdn9Ac; Kdn5Ac; Kdn,9$Ac_2$; Kdn9Me; Kdn5Me; and Kdn7Me..

As described herein, a virus-binding moiety or plurality of virus-binding moieties may be bound to the DNA-based nanostructure in any suitable way. Accordingly, in some embodiments:

a) each virus-binding moiety or plurality of virus-binding moieties is covalently bound to the single-stranded DNA template strand; and/or
b) each virus-binding moiety or plurality of virus binding moieties is covalently bound to the oligonucleotides of the set of oligonucleotides complementary to said single-stranded DNA template.

In some embodiments, the at least one virus-binding moiety is covalently bound to the building block by a linker; optionally wherein the linker is selected from the group comprising or consisting of:

a) an oligo-PEG-linker, optionally wherein the oligo-PEG linker has the structure:

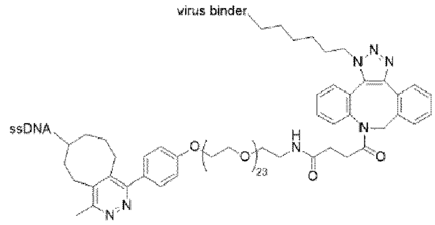

;

b) a PEG linker, optionally wherein the PEG linker has the structure:

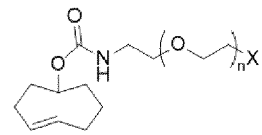

wherein n is an integer between 4 and 227 and X is a linker covalently bound to the virus-binding moiety, optionally wherein the linker is DBCO.

In some embodiments, the virus-binding moiety is non-covalently bound to the self-assembling DNA-based building block, for example is bound to the self-assembling DNA-based building block directly or indirectly *via* an electrostatic or ionic interaction, π-effect, van der Waal forces, hydrophobic effect, or in any other suitable way.

In some embodiments, the self-assembling DNA-based building block comprises one or more handle sites; optionally wherein the one or more handle sites comprise single-stranded DNA overhangs. Any suitable number of handle sites may be used. In some embodiments, the self-assembling DNA building block comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 handle sites.

In some embodiments, the one or more handle sites are comprised by the single-stranded DNA template strand (also known as the "scaffold"). In some embodiments, the one or more handle sites are comprised by the set of oligonucleotides that are at least partially complementary to the single-stranded DNA template (also known as the "staples" or "staple strands"). In some embodiments, the handle sites are single-stranded oligonucleotides having a length of between 20 and 200 nucleotides, 50 and 150 nucleotides, 75 and 125 nucleotides, 20 and 60 nucleotides, 40 and 55 nucleotides; 20 and 30 nucleotides, 22 and 28 nucleotides, 25 and 27 nucleotides, 45 and 55 nucleotides, or 47 and 53 nucleotides.

Suitable handle sites (also known as an "anchor sites") and their placement in the self-assembling DNA-based building block are disclosed for example: on pages 16, 77, and 86-87, in SEQ ID NOs: 2437-2454, 2692-2702, 2915-2929, 3191-3199, and 3422-2429, and in Example 4, Example 5, and Example 6 of International Patent Application No. PCT/EP2021/054307, published as WO 2021/165528 A1; pages 2-7, 9-10, 13, 16-17, and 20, in SEQ ID NOs: 177-203, 398-415, 607-633, 821-829, 1018-1026, 1216-1224, 1416-1424, 1613-1621, and 1812-1820, and Examples 1, 2, and 4 of International Patent Application No. PCT/2023/061264, published as WO 2023/209161 A1; and on pages 8 and 11, in SEQ ID NOs: 146-163, 437-468, 698-729, 915-932, 1136-1167, 1376-1407, and in the Examples of International Patent Application No. PCT 2023/080373, published as WO 2024/094708 A1 (each of which is herein incorporated by reference in its entirety); or a sequence with:

a) at least 80%, at least 90%, at least 95%, at least 96%, at least 97%, at least 98%, at least 99% or more sequence identity to the said sequences;
b) about 80%, about 90%, about 95%, about 96%, about 97%, about 98%, about 99% sequence identity to the said sequences; and/or
c) 80%, 90%, 95%, 96%, 97%, 98%, 99% or more sequence identity to the said sequences.

In some embodiments, the handle site has a sequence of SEQ ID NO: 324-330 set out in **Table 1** herein. In some embodiments, the handle site has a sequence with:

a) at least 80%, at least 90%, at least 95%, at least 96%, at least 97%, at least 98%, at least 99% or more sequence identity to SEQ ID NO: 324-330 set out in **Table 1** herein;

b) about 80%, about 90%, about 95%, about 96%, about 97%, about 98%, about 99% sequence identity to SEQ ID NO: 324-330 set out in **Table 1** herein; and/or

c) 80%, 90%, 95%, 96%, 97%, 98%, 99% or more sequence identity to SEQ ID NO: 324-330 set out in **Table 1** herein.

In some embodiments, the virus-binding moieties are conjugated to a handle-binding oligonucleotide that is able to hybridise to the handle sites; optionally wherein the handle-binding oligonucleotide is complementary to the handle site. Any suitable handle-binding oligonucleotide may be used, provided it is complementary to and anneals to the handle site comprised by the DNA-based nanostructure. Exemplary suitable handle-binding oligonucleotides are disclosed: on pages 6, 13, 14, 17-19, 29, 34-35, and Examples 4, 6, and 7 of International Patent Application No. PCT/EP2021/054307, published as WO 2021/165528 A1; on pages 4, 6, 16, in SEQ ID NO: 1821, and Examples 1, 2, and 4 of International Patent Application No. PCT/2023/061264, published as WO 2023/209161 A1; and on pages 8 and 11, in SEQ ID NO: 1408, and in the Examples of International Patent Application No. PCT 2023/080373, published as WO 2024/094708 A1; or a sequence with:

a) at least 80%, at least 90%, at least 95%, at least 96%, at least 97%, at least 98%, at least 99% or more sequence identity to the said sequences;

b) about 80%, about 90%, about 95%, about 96%, about 97%, about 98%, about 99% sequence identity to the said sequences; and/or

c) 80%, 90%, 95%, 96%, 97%, 98%, 99% or more sequence identity to the said sequences.

In some embodiments, the handle-binding oligonucleotide has a sequence of SEQ ID NO: 331 set out in **Table 1** herein. In some embodiments, the handle-binding oligonucleotide has a sequence with:

a) at least 80%, at least 90%, at least 95%, at least 96%, at least 97%, at least 98%, at least 99% or more sequence identity to SEQ ID NO: 331 set out in **Table 1** herein;

b) about 80%, about 90%, about 95%, about 96%, about 97%, about 98%, about 99% sequence identity to SEQ ID NO: 331 set out in **Table 1** herein; and/or

c) 80%, 90%, 95%, 96%, 97%, 98%, 99% or more sequence identity to SEQ ID NO: 331 set out in **Table 1** herein.

In some embodiments, the virus-binding moieties are conjugated to the handle-binding oligonucleotide by a linker. In some embodiments, the linker is selected from the group comprising or consisting of:

a) an ssDNA-$PEG_4$-azide linker; optionally wherein the ssDNA-$PEG_4$-azide linker has the structure:

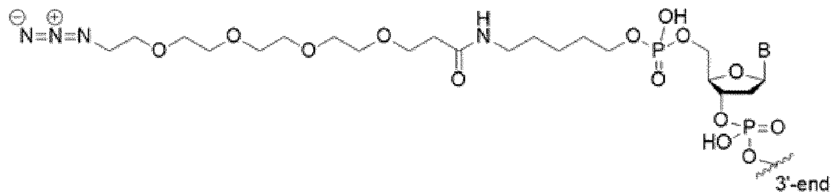

;

b) an ssDNA-DBCO linker; optionally wherein the ssDNA-DBCO linker has the structure:

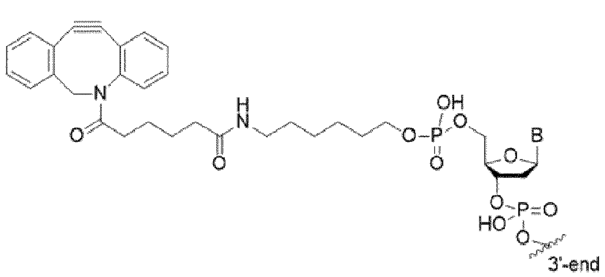

;

c) an ssDNA-SH linker; optionally wherein the ssDNA-SH linker has the structure:

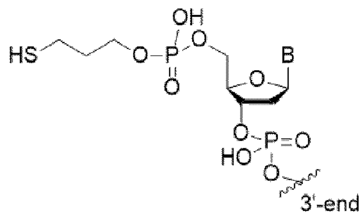

;

d) an ssDNA-PEGs-methyltetrazine linker; optionally wherein the ssDNA-PEGs-methyltetrazine linker has the structure:

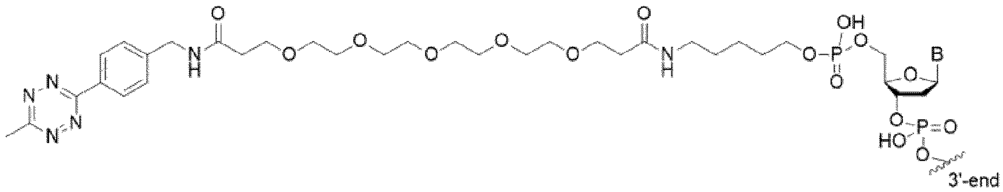

;

and/or

e) an oligo-PEG-linker, optionally wherein the oligo-PEG-linker has the structure:

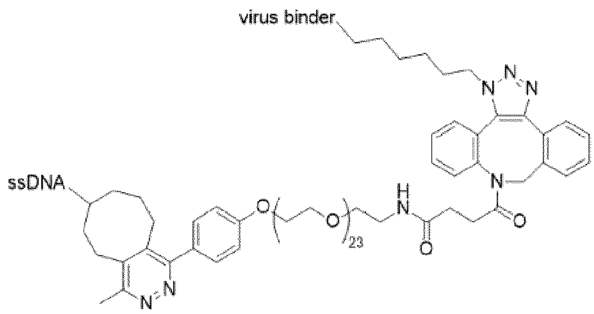

.

In some instances, it is advantageous to covalently bind the handle-binding oligonucleotide to the handle oligonucleotide, for example to improve the stability of the DNA-based nanostructure. In some embodiments, the handle-binding oligonucleotide is covalently bound to the handle site. In some embodiments, the handle-binding oligonucleotide is covalently bound to the handle site by a DNA crosslinking agent or an alkylating agent. In some embodiments, the handle-binding oligonucleotide is covalently bound to the handle site by UV-welding. As is known in the art, excitation of two neighbouring thymine residues using UV light leads to the formation of a covalently bonded thymine dimer. The thymine residues may be present on the same DNA strand or may be present on complementary strands. The person skilled in the art is capable of designing complementary handle-binding oligonucleotides and handle oligonucleotides containing thymine residues at appropriate positions to permit UV welding.

Several neuraminic acid and derivatives thereof may be hydrolysed by the influenza virus neuraminidase (NA). In some instances, it may be advantageous to inhibit neuraminic acid and derivative thereof degradation by influenza NA. Accordingly, in some embodiments, neuraminic acid or derivative thereof is resistant to cleavage or degradation by a neuraminidase; or is a non-cleavable neuraminic acid or non-cleavable neuraminic acid derivative. As used herein, the terms "non-cleavable neuraminic acid" and "non-cleavable neuraminic acid derivative" include neuraminic acids and neuraminic acid derivatives that are resistant to cleavage by influenza neuraminidase or are not cleaved by influenza neuraminidase. Methods of determining the resistance to cleavage by influenza neuraminidase are known in the art, and include, for example, subjecting a test neuraminic acid or test neuraminic acid derivative and a known reference molecule to neuraminidase treatment, and comparing the surviving quantity of said test neuraminic acid or test neuraminic acid derivative to the surviving quantity of the reference molecule that is present after neuraminidase treatment. If the surviving quantity of the test neuraminic acid or test neuraminic acid derivative is higher than the surviving quantity of the reference molecule after neuraminidase treatment, then the test neuraminic acid or test neuraminic acid derivative is identified as a non-cleavable neuraminic acid or non-cleavable neuraminic acid derivative, respectively. Suitable reference molecules include, for example, 2,3'-sialyllactose or 2,6'-sialyllactose.

Any non-cleavable neuraminic acid or neuraminic acid derivative may suitably used as a virus-binding moiety. In some embodiments, the neuraminic acid or derivative thereof has a structure selected from the group comprising or

consisting of:

a)

Compound I:

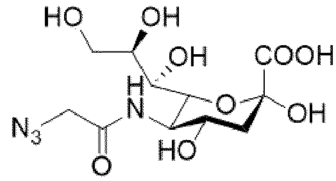

;

b)

Compound II:

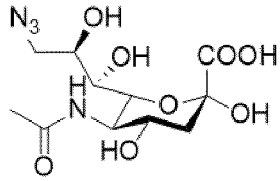

;

c)

Compound III:

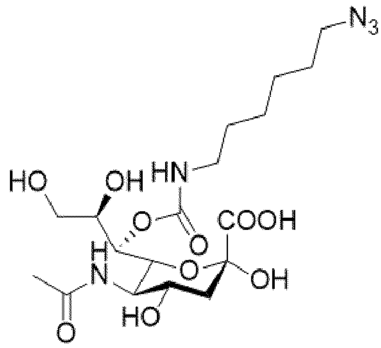

;

d)

Compound IV:

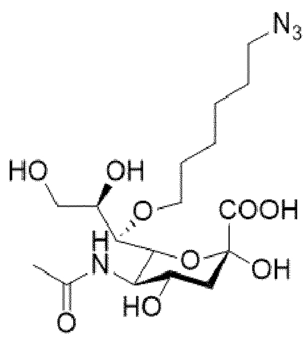

;

e)

Compound Va:

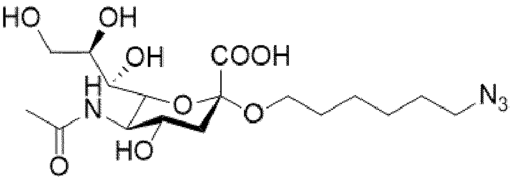

;

f)

Compound Vb:

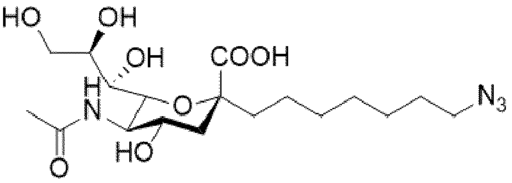

;

And

g)

Compound VI:

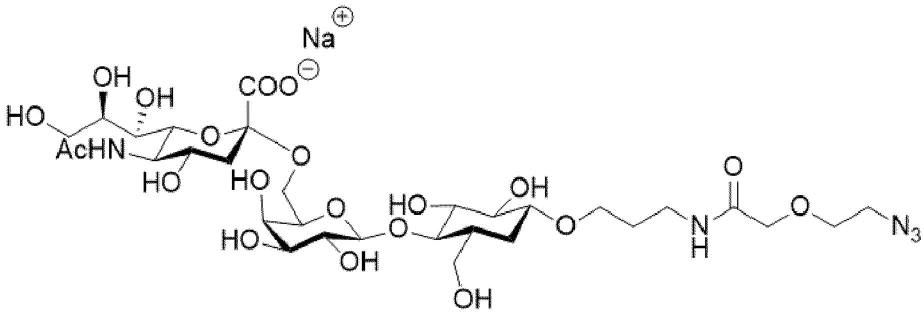

.

The virus-binding moieties may be arranged on the self-assembling DNA-based building blocks in any permutation. For example, a given self-assembling DNA-based building block may comprise virus binding moieties from only one plurality of virus-binding moieties. Alternatively, a given self-assembling DNA-based building block may comprise virus-binding moieties from two, three, four, five, or six different pluralities of virus-binding moieties.

Accordingly, in some embodiments, the self-assembling DNA-based building block comprises a plurality of virus binding moieties; optionally wherein:

a) each virus binding moiety is identical; or
b) each virus binding moiety is selected from a first virus-binding moiety, a second virus-binding moiety, a third virus-binding moiety, a fourth virus-binding moiety, a fifth virus-binding moiety, and/or a sixth virus binding moiety.

In some embodiments, the self-assembling DNA-based building block comprises:

a) at least one first virus-binding moiety, at least one second virus-binding moiety, at least one third virus-binding moiety, at least one fourth virus-binding moiety, at least one fifth virus-binding moiety, and/or at least one sixth virus-binding moiety;
b) at least one first virus-binding moiety, at least one second virus-binding moiety, at least one third virus-binding moiety, at least one fourth virus-binding moiety, at least one fifth virus-binding moiety, and at least one sixth virus-binding moiety;
c) at least one first virus-binding moiety, at least one second virus-binding moiety, at least one third virus-binding moiety, at least one fourth virus-binding moiety, at least one fifth virus-binding moiety, or at least one sixth virus-binding moiety.

The self-assembling DNA-based building block may comprise any suitable number of virus-binding moieties. For example, in some embodiments, the self-assembling DNA-based building block comprises:

a) between 1-30 virus-binding moieties, such as 2-28, 2-26, 4-24, 6-22, 8-20, 10-18, or 12-16 virus binding moieties;
b) at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 27, at least 28, at least 29, or more virus-binding moieties;
c) about 1, about 2, about 3, about 4, about 5, about 6, about 7, about 8, about 9, about 10, about 11, about 12, about 13, about 14, about 15, about 16, about 17, about 18, about 19, about 20, about 22, about 23, about 24, about 25, about 26, about 27, about 28, about 29, or about 30 virus-binding moieties; and/or
d) 1, 2, 3, 4, 5, 6, 7, 8, 9, 10, 11, 12, 13, 14, 15, 16, 17, 18, 19, 20, 21, 22, 23, 24 25, 26, 27, 28, 29, or 30 virus-binding moieties.

The self-assembling DNA-based building block may have any shape suitable for forming a DNA-based nanostructure provided herein. In some embodiments, the self-assembling DNA-based building block is a triangular and/or a rectangular prismoid, particularly a triangular prismoid. Suitable DNA-based building blocks in the shape of triangular prismoids and methods for producing the same are disclosed, for example, in International Patent Application No. PCT/EP2021/054307, published as WO 2021/165528 A1; International Patent Application No. PCT/2023/061264, published as WO 2023/209161 A1; International Patent Application No. PCT 2023/080373, published as WO 2024/094708 A1; and Sigl, C., 2021, Programmable DNA Origami Shell Platform for Virus Neutralization [Doctoral thesis, Technische Universität München], available at: https://mediatum.ub.tum.de/1625147 (each of which is herein incorporated by reference in its entirety).

In some embodiments, the triangular prismoid is formed by m triangular planes, wherein m is an integer independently selected from 4, 5, 6, 7 and 8, in particular independently selected from 5, 6 and 7, more particularly wherein said integer is 6;

the three edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4;
wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes; and
wherein at least two of the three side trapezoids comprise a specific pattern of recesses and/or extrusions formed by missing or additional DNA double helical stretches for specific interaction with a complementary pattern on the side trapezoid of another one of said self-assembling DNA-based building blocks.

In some embodiments, the length of at least one edge of each of said m planes is decreasing from the first to the $m^{th}$ plane, so that bevel angles results between planes perpendicular to said first plane and each of the trapezoid planes formed by said m edges.

In some embodiments the bevel angle is:

a) between 0° and 30°, for example between 2° and 28°, 4° and 26°, 6° and 24°, 8° and 22°, or 10° and 20°;
b) between 8° and 15°, for example between 10° and 13°, or 11° and 12°;
c) about 0°, about 1°, about 2°, about 3°, about 4°, about 5°, about 6°, about 7°, about 8°, about 9°, about 10°, about 11°, about 12°, about 13°, about 14°, about 15°, about 16°, about 17°, about 18°, about 19°, about 20°, about 21°, about 22°, about 23°, about 24°, about 25°, about 26°, about 27°, about 28°, about 29°, or about 30°; and/or
c) 0°, 1°, 2°, 3°, 4°, 5°, 6°, 7°, 8°, 9°, 10°, 11°, 12°, 13°, 14°, 15°, 16°, 17°, 18°, 19°, 20°, 21°, 22°, 23°, 24°, 25°, 26°, 27°, 28°, 29°, or 30°.

In some embodiments the bevel angle is:

a) between 11° and 12°, 11.1° and 11.9°, 11.2° and 11.8°, 11.3° and 11.7°;
b) between 11.5° and 11.8°, 11.55° and 11.75°, or between 11.6° and 11.7°;
c) about 11°, about 11.2°, about 11.4°, about 11.6°, about 11.8°, or about 12°;
d) 11°, 11.2°, 11.4°, 11.6°, 11.8°, or 12°; and/or
e) 11.64°.

In some embodiments, the bevel angle is between 16° and 26°, between 18° and 24°, more particularly between 20° and 22°. In some embodiments, the bevel angle is 20.9°.

In some embodiments, one, two, or all three side trapezoids comprises a specific pattern of recesses and/or extrusions formed by missing or additional DNA double helical stretches for specific interaction with a complementary pattern on the side trapezoid of another one of said self-assembling DNA-based building blocks. In some embodiments, two of the side trapezoids are capable of specifically interacting with a complementary pattern on the side trapezoid of another one of said self-assembling DNA-based building blocks, and one of the side trapezoids is not capable of said specific interaction. As described herein, the positioning of the specific pattern of recesses and/or extrusions formed on the side trapezoids of a self-assembling DNA-based building-block allows interaction with other self-assembling DNA-based building blocks by base stacking, which determines the shape of the DNA-based nanostructure that may be formed from said self-assembling DNA-based building block or plurality of self-assembling DNA-based building blocks. The strength of the base stacking interactions may be tuned as described herein.

In some embodiments, the self-assembling DNA-based building block has a diameter of:

a) at least 40 nm, at least 41 nm, at least 42 nm, at least 43 nm, at least 44 nm, at least 45 nm at least 46 nm, at least 47 nm, at least 48 nm, at least 49 nm, at least 50 nm, at least 51 nm, at least 52 nm, at least 53 nm, at least 54 nm, at least 55 nm, at least 56 nm, at least 57 nm, at least 58 nm, at least 59 nm, or more;
b) about 40 nm, about 41 nm, about 42 nm, about 43 nm, about 44 nm, about 45 nm about 46 nm, about 47 nm, about 48 nm, about 49 nm, about 50 nm, about 51 nm, about 52 nm, about 53 nm, about 54 nm, about 55 nm, about 56 nm, about 57 nm, about 58 nm, about 59 nm, or about 60 nm; and/or
c) 40 nm, 41 nm, 42 nm, 43 nm, 44 nm, 45 nm 46 nm, 47 nm, 48 nm, 49 nm, 50 nm, 51 nm, 52 nm, 53 nm, 54 nm, 55 nm, 56 nm, 57 nm, 58 nm, 59 nm or 60 nm.

In some embodiments, the self-assembling DNA-based building block has a molecular mass of:

a) at least 3.7 MDa, at least 3.8 MDa, at least 3.9 MDa, at least 4 MDa, at least 4.1 MDa, at least 4.2 MDa, at least 4.3 MDa, at least 4.4 MDa, at least 4.5 MDa, at least 4.6 MDa, at least 4.7 MDa, at least 4.8 MDa, at least 4.9 MDa, at least 5 MDa, at least 5.1 MDa, or more;
b) about 3.7 MDa, about 3.8 MDa, about 3.9 MDa, about 4 MDa, about 4.1 MDa, about 4.2 MDa, about 4.3 MDa, about 4.4 MDa, about 4.5 MDa, about 4.6 MDa, about 4.7 MDa, about 4.8 MDa, about 4.9 MDa, about 5 MDa, about 5.1 MDa, or about 5.2 MDa;
c) 3.7 MDa, 3.8 MDa, 3.9 MDa, 4 MDa, 4.1 MDa, 4.2 MDa, 4.3 MDa, 4.4 MDa, 4.5 MDa, 4.6 MDa, 4.7 MDa, 4.8 MDa, 4.9 MDa, 5 MDa, 5.1 MDa, or 5.2 MDa .

In some embodiments, the self-assembling DNA-based building block comprises a In some embodiments, the self-assembling DNA-based building block is configured to bind a target virus, optionally wherein the target virus is an influenza virus. In some embodiments, the influenza virus is an influenza virus as disclosed herein.

As will be understood, the utility of the self-assembling DNA-based building block provided herein is not limited to influenza. For example, the self-assembling DNA-based building block may be configured to bind to the surface or at least partially encapsulate any target virus that binds to neuraminic acid or a derivative thereof. Furthermore, the DNA-based nanostructure may be configured to bind to the surface of or at least partially encapsulate a target virus that is not an influenza virus by including as virus-binding moieties in the second, third, fourth, fifth, or sixth pluralities of virus-binding moieties compounds or molecules that bind to a virus that is not an influenza virus. By way of non-limiting example, in embodiments where the self-assembling DNA-based building block comprises a first plurality of virus-binding moieties that are neuraminic acid or a derivative thereof, and a second plurality of virus-binding moieties that are, for example, anti-SARS-CoV-2 spike protein antibodies, the self-assembling DNA-based building block would be able to bind to the surface of or at least partially encapsulate both influenza virus and SARS-CoV-2. The same approach may be taken *mutatis mutandis* with the third, fourth, fifth, and sixth pluralities of virus-binding moieties, and other target viruses that are not influenza virus. Accordingly, in some embodiments, the self-assembling DNA-based building block is configured to bind to the surface of or at least partially encapsulate influenza virus and at least one virus selected from the group comprising or consisting of: AAV2, hepatitis B (HBV), SARS-CoV-2, chikungunya virus, and Zika virus. Suitable virus-binding moieties for targeting a DNA-based nanostructure to AAV2, hepatitis B (HBV), SARS-CoV-2, chikungunya virus, and Zika virus are disclosed in: International Patent Application No. PCT/EP2021/054307, published as WO 2021/165528 A1; International Patent Application No. PCT/2023/061264, published as WO 2023/209161 A1; and International Patent Application No. PCT 2023/080373, published as WO 2024/094708 A1 (each of which is herein incorporated by reference in its entirety).

In some embodiments, the self-assembling DNA-based building block is configured to form the nanostructure provided herein with a plurality of self-assembling DNA-based building blocks *in situ* in the presence of the target virus. In some embodiments, the self-assembling DNA-based building block is configured to form the nanostructure provided

herein with a plurality of self-assembling DNA-based building blocks as provided herein, in the absence of the target virus.

The plurality of self-assembling DNA-based building blocks may comprise any DNA-based building block that may be configured to form the nanostructure provided herein. Suitable DNA-based building blocks are disclosed, for example, in International Patent Application No. PCT/EP2021/054307, published as WO 2021/165528 A1; International Patent Application No. PCT/2023/061264, published as WO 2023/209161 A1; and International Patent Application No. PCT 2023/080373, published as WO 2024/094708 A1; and Sigl, C., 2021, Programmable DNA Origami Shell Platform for Virus Neutralization [Doctoral thesis, Technische Universität München], available at: https://mediatum.ub.tum.de/1625147 (each of which is herein incorporated by reference in its entirety). In some embodiments, each self-assembling DNA-based building block of the plurality of self-assembling DNA-based building blocks is a self-assembling DNA-based building block provided herein.

In some embodiments, the self-assembling DNA-based building block comprises a single-stranded DNA template strand; a set of oligonucleotides that are at least partially complementary to said single-stranded DNA template; handle sites; and/or handle-binding oligonucleotides with sequences of SEQ ID NO: 1-331 set out herein and in **Table 1.** In some embodiments, the self-assembling DNA-based building blocks comprise a single-stranded DNA template strand; a set of oligonucleotides that are at least partially complementary to said single-stranded DNA template; handle sites; and/or handle-binding oligonucleotides with sequences with:

a) at least 80%, at least 90%, at least 95%, at least 96%, at least 97%, at least 98%, at least 99% or more sequence identity to SEQ ID NO: 1-331 set out herein and in **Table 1**;
b) about 80%, about 90%, about 95%, about 96%, about 97%, about 98%, about 99% sequence identity to SEQ ID NO: 1-331 set out herein and in **Table 1**; and/or
c) 80%, 90%, 95%, 96%, 97%, 98%, 99% or more sequence identity to SEQ ID NO: 1-331 set out herein and in **Table 1.**

<u>A broad-spectrum target-binding DNA-based nanostructure</u>

Also provided herein is a DNA-based nanostructure wherein the DNA-based nanostructure comprises at least five self-assembling DNA-based building blocks;

each of the self-assembling DNA-based building blocks is formed by a single-stranded DNA template strand and a set of oligonucleotides that are at least partially complementary to said single-stranded DNA template; and
wherein each of the oligonucleotides is either complementary to one contiguous DNA sequence stretch or to at least two non-contiguous DNA sequence stretches on said single-stranded DNA template.

The self-assembling DNA-based building blocks may be any self-assembling DNA-based building block provided herein. In some embodiments, the DNA-based nanostructure has a concave surface and/or a convex surface.

In some embodiments, the concave surface of the DNA-based nanostructure has a curvature (k) of:

a) at least 10 $\mu m^{-1}$, at least 20 $\mu m^{-1}$, at least 40 $\mu m^{-1}$, at least 60 $\mu m^{-1}$, at least 80 $\mu m^{-1}$, at least 100 $\mu m^{-1}$;
b) about 10 $\mu m^{-1}$, about 20 $\mu m^{-1}$, about 40 $\mu m^{-1}$, about 60 $\mu m^{-1}$, about 80 $\mu m^{-1}$, 100 $\mu m^{-1}$; and/or
c) 10 $\mu m^{-1}$, 20 $\mu m^{-1}$, 40 $\mu m^{-1}$, 60 $\mu m^{-1}$, 80 $\mu m^{-1}$, 100 $\mu m^{-1}$.

In some embodiments, the concave surface of the DNA-based nanostructure has a curvature (k) of 60 $\mu m^{-1}$.

The skilled person is aware of methods for calculating curvature (k). In some embodiments:

$$k = \frac{1}{R}$$

Where R is the radius of a given circle, sphere, half-sphere, or hemisphere, for example the radius of a circle that has the same radius as the DNA-based nanostructure provided herein.

In some embodiments, the DNA-based nanostructure has a diameter of:

a) at least 60 nm, at least 75 nm, at least 80 nm, or at least 100 nm;
b) about 60 nm, about 75 nm, about 80 nm, or about 100 nm; and/or
c) 60nm, 75 nm, 80 nm or 100 nm.

In some embodiments, the DNA-based nanostructure has a molecular mass of:

a) at least 18 MDa, at least 19 MDa, at least 20 MDa, at least 23 MDa, at least 25 MDa, at least 27 MDa, at least 30 MDa, at least 35 MDa, or more;
b) about 18 MDa, about 19 MDa, about 20 MDa, about 23 MDa, about 25 MDa, about 27 MDa, about 30 MDa, or about 35 MDa; and/or
c) 18 MDa, 19 MDa, 20 MDa, 23 MDa, 25 MDa, 27 MDa, 30 MDa, or 35 MDa.

In some embodiments, each of the DNA-based building blocks is a triangular prismoid.
In some embodiments, the DNA-based building blocks form a symmetrical structure.
In some embodiments, each said triangular prismoid is formed by m triangular planes, wherein m is an integer independently selected from 4, 5, 6, 7 and 8, in particular independently selected from 5, 6 and 7, more particularly wherein said integer is 6;

the three edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4;
wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes; and
wherein at least two of the three side trapezoids comprise a specific pattern of recesses and/or extrusions formed by missing or additional DNA double helical stretches for specific interaction with a complementary pattern on the side trapezoid of another one of said self-assembling DNA-based building blocks.

In some embodiments, for at least part of the self-assembling DNA-based building blocks the length of at least one edge of each of said m planes is decreasing from the first to the $m^{th}$ plane, so that bevel angles results between planes perpendicular to said first plane and each of the trapezoid planes formed by said m edges.
In some embodiments, one, two, or all three side trapezoids comprises a specific pattern of recesses and/or extrusions formed by missing or additional DNA double helical stretches for specific interaction with a complementary pattern on the side trapezoid of another one of said self-assembling DNA-based building blocks. In one embodiment, two of the side trapezoids are capable of specifically interacting with a complementary pattern on the side trapezoid of another one of said self-assembling DNA-based building blocks, and one of the side trapezoids is not capable of said specific interaction. The role of the specific pattern of recesses and/or extrusions formed by missing or additional DNA double helical stretches comprised by the side trapezoids is discussed herein, as is the tuneability of the same.
In some embodiments, the nanostructure comprises at least a first plurality of target-binding moieties. The target-binding moieties may be any moiety suitable for enabling the nanostructure to bind to a target entity.
As disclosed herein, the DNA-based nanostructure provided herein may suitably bind to the surface of or at least partially encapsulate a virus. Accordingly, in some embodiments the at least first plurality of target-binding moieties is a virus-binding moiety that is a neuraminic acid or a derivative thereof. In some embodiments, the at least first plurality of target-binding moieties is a virus-binding moiety selected from the group comprising or consisting of: an antibody or a virus-binding fragment thereof, a nanobody or virus-binding fragment thereof, an affimer, heparin, heparan sulfate, hybrid heparan sulfates, a host receptor protein or virus-binding fragment thereof, a de novo virus-binding peptide, an aptamer, and a neuraminidase inhibitor. In some embodiments, the neuraminidase inhibitor is selected from the group comprising or consisting of: oseltamivir, zanamivir, ianamivir, and peramivir; optionally wherein the neuraminidase inhibitor is oseltamivir.
As discussed herein, the utility of the DNA-based nanostructure provided herein is not limited to treatment of infection with a virus such as influenza virus, or encapsulation or neutralisation of a virus such as an influenza virus. For example, the surface of any target may be bound, and/or any target may be at least partially encapsulated using the DNA-based nanostructure provided herein. Accordingly, in some embodiments the at least first plurality of target-binding moieties is a cell-binding moiety, optionally a cell-binding moiety that is capable of binding to the surface of a cell selected from the group comprising or consisting of: a bacterial cell, an archaeal cell, a fungal cell, an animal cell, a mammalian cell, a plant cell, an amoeba cell, a protozoan cell, a helminth cell, a sporozoan cell, a ciliate cell, a trematode cell, a nematode cell, and a cestode cell. In some embodiments, the cell-binding moiety is selected from the group comprising or consisting of: an antibody or cell-binding fragment thereof, a nanobody or cell-binding fragment thereof, heparin, heparan sulfate, hybrid heparan sulfates, a host receptor protein or virus-binding fragment thereof, a de novo virus-binding peptide, an aptamer, and an affimer.
In some embodiments, the at least a first plurality of target-binding moieties is bound to the concave surface. In some embodiments, when present the second plurality of target-binding moieties, third plurality of target-binding moieties, fourth plurality of target-binding moieties, fifth plurality of target-binding moieties, and/or sixth plurality of target-binding

moieties are bound to the concave surface.

In some embodiments, the nanostructure further comprises at least a first plurality of recruitment moieties, optionally wherein each recruitment moiety of the first plurality of recruitment moieties is selected from the group comprising or consisting of: an antibody Fc fragment, a TLR ligand, and an opsonin.

In some embodiments, the DNA-based nanostructure is a nanostructure is the nanostructure described herein above. In some embodiments, each self-assembling DNA-based building block is a self-assembling DNA-based building block provided herein and/or described above.

A nucleic acid-conjugate

Also provided herein is a nucleic acid conjugated to a neuraminic acid residue or a derivative thereof. The neuraminic acid residue or derivative thereof may be any neuraminic acid or derivative thereof as described herein. In some embodiments, the nucleic acid is a handle-binding oligonucleotide as provided herein. Accordingly, as will be understood, the nucleic acid conjugated to a neuraminic acid residue or derivative thereof may be used to form the DNA-based nanostructure provided herein and/or the self-assembling DNA-based building block provided herein.

Compositions comprising the inventions provided herein, and their uses

Also provided herein is a composition comprising the self-assembling DNA building block provided herein.

Also provided herein is a composition comprising a plurality of self-assembling DNA building blocks provided herein.

Also provided herein is a plurality of self-assembling DNA-based building blocks, wherein each self-assembling DNA-based building block of the plurality of self-assembling DNA-based building blocks is a self-assembling DNA-based building block provided herein.

Also provided herein is a composition comprising a plurality of self-assembling DNA-based building blocks, wherein each self-assembling DNA-based building block of the plurality of self-assembling DNA-based building blocks is a self-assembling DNA-based building block provided herein.

Also provided herein is a composition comprising the DNA-based nanostructure provided herein.

In some embodiments, the composition further comprises a neuraminidase inhibitor. In some embodiments, the neuraminidase inhibitor is selected from the group comprising or consisting of: oseltamivir, zanamivir, ianamivir, and peramivir; optionally wherein the neuraminidase inhibitor is oseltamivir.

Also provided herein is a method of encapsulating a virus, a viral particle, or a subviral particle, comprising contacting the virus, viral particle, or subviral particle with the DNA-based nanostructure provided herein, the self-assembling DNA-based building block provided herein, a composition provided herein, or the plurality of self-assembling DNA based building blocks provided herein.

Also provided herein is a method of neutralising a virus, a viral particle, or a subviral particle, comprising contacting the virus, viral particle, or subviral particle with the DNA-based nanostructure provided herein, the self-assembling DNA-based building block provided herein, the composition provided herein, or the plurality of self-assembling DNA based building blocks provided herein.

In some embodiments, the method of encapsulating a virus, a viral particle, or a subviral particle, or method of neutralising a virus, a viral particle, or a subviral particle, comprises contacting the virus, viral particle, or subviral particle with a neuraminidase inhibitor. In some embodiments, the neuraminidase inhibitor is selected from the group comprising or consisting of: oseltamivir, zanamivir, ianamivir, and peramivir; optionally wherein the neuraminidase inhibitor is oseltamivir.

Suitable methods for encapsulating a virus and/or neutralising a virus are provided herein, and in International Patent Application No. PCT/EP2021/054307, published as WO 2021/165528 A1; International Patent Application No. PCT/2023/061264, published as WO 2023/209161 A1; and International Patent Application No. PCT 2023/080373, published as WO 2024/094708 A1; and Sigl, C., 2021, Programmable DNA Origami Shell Platform for Virus Neutralization [Doctoral thesis, Technische Universität München], available at: https://mediatum.ub.tum.de/1625147 (each of which is herein incorporated by reference in its entirety).

Also provided herein is a composition comprising a virus, a viral particle, or a subviral particle and the DNA-based nanostructure provided herein, the self-assembling DNA-based building block provided herein, the DNA-based nanostructure provided herein, or plurality of self-assembling DNA based building blocks provided herein.

In some embodiments, the virus, viral particle, or subviral particle is bound or at least partially encapsulated by the DNA-based nanostructure, self-assembling DNA-based building block, or plurality of self-assembling DNA-based building blocks.

In some embodiments, the virus, viral particle, or subviral particle is influenza virus as described herein.

In one embodiment, the methods provided herein are *in vitro* methods. In one embodiment, the methods provided

herein are *in vivo* methods.

Medical uses of the inventions provided herein

As discussed herein, the present invention has particular utility in medicine, for example in treating infection with a virus such as influenza virus.

Accordingly, in one aspect, provided herein is the DNA-based nanostructure provided herein or self-assembling DNA-based building block provide herein for use in medicine.

In one aspect, provided herein is the DNA-based nanostructure provided herein or self-assembling DNA-based building block provided herein for use in treating Influenza infection.

In one aspect, provided herein is the DNA-based nanostructure provided herein or self-assembling DNA-based building block provided herein for use in prophylaxis against influenza infection.

In some embodiments, the DNA-based nanostructure is administered to a subject in need thereof. In some embodiments, the DNA-based nanostructure is administered to a subject in a pharmaceutically acceptable carrier, excipient, or diluent.

In one aspect, provided herein is a method of treating a disease in a subject comprising administering the DNA-based nanostructure provided herein or self-assembling DNA-based building block provided herein to the subject.

In one aspect, provided herein is a method of treating influenza infection in a subject comprising administering the DNA-based nanostructure provided herein or self-assembling DNA-based building block provided herein to the subject.

In one aspect, provided herein is a method of preventing Influenza infection comprising administering the DNA-based nanostructure provided herein or self-assembling DNA-based building block provided herein to a subject.

Also provided herein is the DNA-based nanostructure provided herein or self-assembling DNA-based building block provided herein for use in the manufacture of a medicament.

Also provided herein is the DNA-based nanostructure provided herein or the self-assembling DNA-based building block provided herein for use in the manufacture of a medicament for treating influenza infection.

Also provided herein is a composition comprising the DNA-based nanostructure provided herein or the self-assembling DNA-based building block provided herein.

In one embodiment, the composition provided herein further comprises a pharmaceutically acceptable excipient, buffer, or carrier. Suitable pharmaceutically acceptable excipients, buffers, and carriers are known to the person skilled in the art.

The inventors consider that it may be advantageous to administer the DNA-based nanostructure, self-assembling DNA-based building block, and/or composition comprising the same provided herein with a neuraminidase inhibitor. Without wishing to be bound by theory, the inventors hypothesise that a neuraminidase inhibitor may prevent viral neuraminidase cleavage of neuraminic acid residues and/or derivatives thereof on the DNA-based nanostructure or self-assembling DNA-based building block, thereby improving the binding of the DNA-based nanostructure self-assembling DNA-based building block to the target virus and/or neutralisation of the target virus by of the DNA-based nanostructure self-assembling DNA-based building block. In some embodiments, the composition further comprises a neuraminidase inhibitor, optionally wherein the neuraminidase inhibitor is selected from the group comprising or consisting of: oseltamivir, zanamivir, ianamivir, and peramivir; optionally wherein the neuraminidase inhibitor is oseltamivir.

In one aspect, provided herein is the composition provided herein for use in medicine.

Also provided herein is the composition provided herein for use in treating Influenza infection.

Also provided herein is the composition provided herein for use in prophylaxis against Influenza infection.

In some embodiments, the composition is administered to a subject in need thereof. The term "a subject in need thereof" includes a subject currently suffering from an infection or disease; a subject thought to be suffering from an infection or disease; or a subject thought to be at risk of suffering from an infection or disease. In some embodiments, the infection or disease is selected from the group comprising or consisting of: influenza virus infection (influenza or flu), AAV2 infection, hepatitis B (HBV) infection (hepatitis or viral hepatitis), SARS-CoV-2 infection (COVID-19), chikungunya virus infection (chikungunya), and Zika virus infection (Zika).

Also provided herein is a method of treating a disease comprising administering the composition provided herein to the subject.

Also provided herein is a method of treating influenza infection in a subject, comprising administering the composition provided herein to the subject.

Also provided herein is a method of preventing influenza infection in a subject, comprising administering the composition provided herein to the subject.

Also provided herein is the composition provided herein for use in the manufacture of a medicament.

Also provided herein is the composition provided herein for use in the manufacture of a medicament for treating influenza infection.

In some embodiments of aspects relating to the the DNA-based nanostructure or self-assembling DNA-based

building block for use provided herein, or composition for use provided herein, the influenza infection is caused by an influenza virus selected from: Influenza A, Influenza B, Influenza C, and Influenza D. In some embodiments, the influenza virus is Influenza A or Influenza B. In some embodiments:

a) the influenza virus has a haemagglutinin subtype selected from the group comprising or consisting of: H1, H2, H3, H4, H5, H6, H7, H8, H9, H10, H11, H12, H13, H14, H15, H16, H17, and H18; and/or
b) the influenza virus has a neuraminidase subtype selected from: N1, N2, N3, N4, N5, N6, N7, N8, N9, N10, and N11.

In some embodiments, the influenza virus has a subtype selected from the group comprising or consisting of: H1N1, H2N2, H3N2, H5N1, H5N5, H5N6, H5N8, H6N1, H7N2, H7N7, H7N9, and H10N7. In some embodiments, the influenza has a subtype that is H3N2, H5N1, H7N9, or H1N1.

In some embodiments of aspect relating to the DNA-based nanostructure or self-assembling DNA-based building block for use provided herein, method provided herien, or composition for use provided herein, the DNA-based nanostructure or composition is co-administered with a neuraminidase inhibitor.

In some embodiments, the neuraminidase inhibitor is selected from the group comprising or consisting of: oseltamivir, zanamivir, ianinamivir, and peramivir; optionally wherein the neuraminidase inhibitor is oseltamivir.

Methods of producing the nanostructure provided herein

In one aspect, provided herein is a method of producing a DNA-based nanostructure provided herein, the method comprising the steps of:

a) providing at least one single-stranded DNA template strand and a set of oligonucleotides that are at least partially complementary to said single-stranded DNA template;
b) incubating an admixture of the at least one single-stranded DNA template strand and a set of oligonucleotides that are at least partially complementary to said single-stranded DNA template under conditions that permit annealing of the single-stranded DNA template strand and oligonucleotides of the set of oligonucleotides that are at least partially complementary to said single-stranded DNA template to form a plurality of DNA-based building blocks; and
c) incubating the plurality of DNA-based building blocks under conditions that permit assembly of the DNA-based building blocks into the DNA-based nanostructure.

The at least one single-stranded DNA template strand and a set of oligonucleotides that are at least partially complementary to said single-stranded DNA template may be obtained from any suitable source. For example, the single-stranded DNA template strand and/or oligonucleotides that are at least partially complementary to said single-stranded DNA template may be chemically synthesised, or may derived, for example, from an organism such as a bacterium, a fungus, an archaeon, or a bacteriophage. In some embodiments, the single-stranded DNA template strand and/or oligonucleotides that are at least partially complementary to said single-stranded DNA template is single-stranded DNA from a filamentous bacteriophage or is derived from single-stranded DNA of a filamentous bacteriophage. In some embodiments, the single-stranded DNA template strand and/or oligonucleotides that are at least partially complementary to said single-stranded DNA template is single-stranded DNA are obtained from different sources. For example, in some embodiments, the single-stranded DNA template strand is single-stranded DNA from a filamentous bacteriophage or is derived from single-stranded DNA of a filamentous bacteriophage, and the oligonucleotides that are at least partially complementary to said single-stranded DNA template is single-stranded DNA is chemically synthesised. Methods of chemically synthesising single-stranded DNA is known to the skilled person. In some embodiments, the single-stranded DNA template strand and/or oligonucleotides that are at least partially complementary to said single-stranded DNA template is single-stranded DNA are obtained from the same source, optionally from the same filamentous bacteriophage or is derived from single-stranded DNA of the same filamentous bacteriophage, for example using the methods disclosed in Praetorius et al., 2017, "Biotechnological mass production of DNA origami" Nature, 552:84-87, and International Patent Application No. PCT/EP2017/068051, published as WO 2018/054571 (each of which is herein incorporated by reference in its entirety).

As used herein, the term "filamentous bacteriophage" refers to a bacteriophage that is characterised by its filament-like shape that usually contains a genome of circular single-stranded DNA and infects Gram-negative bacteria. Exemplary filamentous bacteriophages include but are not limited to Ff phage such as M13, f1 and fd1 phage, and PF1 phage.

A single-stranded DNA template that is "derived from single-stranded DNA of filamentous bacteriophage" refers to a DNA construct that is derived from a naturally occurring of published DNA sequence of a filamentous bacteriophage by one or more of: (i) opening of the circular structure to a linear sequence; (ii) deletion of one or more nucleotides; (iii) insertion of one or more nucleotides; (iii) substitution of one or more nucleotides; (iv) addition of one or more nucleotides;

and (v) modification of one or more nucleotides.

In some embodiments, the single-stranded DNA template has a sequence of SEQ ID NO: 1 of International Patent Application No. PCT/EP2023/080373, published as WO 2024/094708 A1; SEQ ID NO: 1 of International Patent Application No. PCT/EP2023/061264, published as WO 2023/209161 A1; SEQ ID NOs: 1 or 2 of International Patent Application No. PCT/EP2021/054307, published as WO 2021/165528 A1; or a sequence with:

a) at least 80%, at least 90%, at least 95%, at least 96%, at least 97%, at least 98%, at least 99% or more sequence identity thereto;
b) about 80%, about 90%, about 95%, about 96%, about 97%, about 98%, about 99% sequence identity thereto; and/or
c) 80%, 90%, 95%, 96%, 97%, 98%, 99% or more sequence identity thereto.

In some embodiments, the single-stranded DNA-template has a sequence of SEQ ID NO: 1 or SEQ ID NO: 2 set out herein. In some embodiments, the single-stranded DNA-template has a sequence with:

a) at least 80%, at least 90%, at least 95%, at least 96%, at least 97%, at least 98%, at least 99% or more sequence identity to SEQ ID NO: 1 or SEQ ID NO: 2 set out herein;
b) about 80%, about 90%, about 95%, about 96%, about 97%, about 98%, about 99% sequence identity to SEQ ID NO: 1 or SEQ ID NO: 2 set out herein; and/or
c) 80%, 90%, 95%, 96%, 97%, 98%, 99% or more sequence identity to SEQ ID NO: 1 or SEQ ID NO: 2 set out herein.

In some embodiments, the oligonucleotides that are at least partially complementary to said single-stranded DNA template each have a sequence selected from SEQ ID NOs: 2-1407 of International Patent Application No. PCT/EP2023/080373, published as WO 2024/094708 A1; SEQ ID NOs: 2-1820 of International Patent Application No. PCT/EP2023/061264, published as WO 2023/209161 A1; SEQ ID NOs: 3-4089 of International Patent Application No. PCT/EP2021/054307, published as WO 2021/165528 A1; or a sequence with:

a) at least 80%, at least 90%, at least 95%, at least 96%, at least 97%, at least 98%, at least 99% or more sequence identity thereto;
b) about 80%, about 90%, about 95%, about 96%, about 97%, about 98%, about 99% sequence identity thereto; and/or
c) 80%, 90%, 95%, 96%, 97%, 98%, 99% or more sequence identity thereto.

In some embodiments, the oligonucleotides that are at least partially complementary to said single-stranded DNA template each have a sequence selected from SEQ ID NOs: 3-323 set out in **Table 1** herein. In some embodiments, the oligonucleotides that are at least partially complementary to said single-stranded DNA template each have a sequence selected from a sequence with:

a) at least 80%, at least 90%, at least 95%, at least 96%, at least 97%, at least 98%, at least 99% or more sequence identity to SEQ ID NO: 3-323 set out in **Table 1** herein;
b) about 80%, about 90%, about 95%, about 96%, about 97%, about 98%, about 99% sequence identity to SEQ ID NO: 3-323 set out in **Table 1** herein; and/or
c) 80%, 90%, 95%, 96%, 97%, 98%, 99% or more sequence identity to SEQ ID NO: 3-323 set out in **Table 1** herein.

In some embodiments:

a) the single-stranded DNA template has:

i) a sequence of SEQ ID NO: 1 set out herein;
ii) a sequence with at least 80%, at least 90%, at least 95%, at least 96%, at least 97%, at least 98%, at least 99% or more sequence identity to SEQ ID NO: 1 set out in herein;
iii) a sequence with about 80%, about 90%, about 95%, about 96%, about 97%, about 98%, about 99% sequence identity to SEQ ID NO: 1 set out in herein; and/or
iv) a sequence with 80%, 90%, 95%, 96%, 97%, 98%, 99% or more sequence identity to SEQ ID NO: 1 set out in herein;

and/or
b) the oligonucleotides that are at least partially complementary to said single-stranded DNA template each have:

i) a sequence set out in SEQ ID NOs: 3-131 set out in **Table 1**;
ii) a sequence with at least 80%, at least 90%, at least 95%, at least 96%, at least 97%, at least 98%, at least 99% or more sequence identity to SEQ ID NO: 3-131 set out in **Table 1** herein;

b) a sequence with about 80%, about 90%, about 95%, about 96%, about 97%, about 98%, about 99% sequence identity to SEQ ID NO: 3-131 set out in **Table 1** herein; and/or
c) a sequence with 80%, 90%, 95%, 96%, 97%, 98%, 99% or more sequence identity to SEQ ID NO: 3-131 set out in **Table 1** herein.

In some embodiments:

a) the single-stranded DNA template has:

i) a sequence of SEQ ID NO: 2 set out herein;
ii) a sequence with at least 80%, at least 90%, at least 95%, at least 96%, at least 97%, at least 98%, at least 99% or more sequence identity to SEQ ID NO: 2 set out in herein;
iii) a sequence with about 80%, about 90%, about 95%, about 96%, about 97%, about 98%, about 99% sequence identity to SEQ ID NO: 2 set out in herein; and/or
iv) a sequence with 80%, 90%, 95%, 96%, 97%, 98%, 99% or more sequence identity to SEQ ID NO: 2 set out in herein;
and/or

b) the oligonucleotides that are at least partially complementary to said single-stranded DNA template each have:

i) a sequence set out in SEQ ID NOs: 132-323 set out in **Table 1**;
ii) a sequence with at least 80%, at least 90%, at least 95%, at least 96%, at least 97%, at least 98%, at least 99% or more sequence identity to SEQ ID NO: 132-323 set out in **Table 1** herein;

b) a sequence with about 80%, about 90%, about 95%, about 96%, about 97%, about 98%, about 99% sequence identity to SEQ ID NO: 132-323 set out in **Table 1** herein; and/or
c) a sequence with 80%, 90%, 95%, 96%, 97%, 98%, 99% or more sequence identity to SEQ ID NO: 132-323 set out in **Table 1** herein.

The inventors have surprisingly found that by shortening the length of the single-stranded DNA template compared to the 8064 nt size of the DNA template previously disclosed, for example, in International Patent Application No. PCT/EP2023/080373, published as WO 2024/094708 A1; International Patent Application No. PCT/EP2023/061264, published as WO 2023/209161 A1; International Patent Application No. PCT/EP2021/054307, published as WO 2021/165528 A1; and International Patent Application No. PCT/EP2017/068051, published as WO 2018/054571, it is possible to reduce the processing required to obtain sufficient DNA to perform the methods provided herein to obtain the DNA-based nanostructure provided herein. Accordingly, in some embodiments, the single-stranded DNA template has a length of:

a) between 5000 nt and 7000 nt, 5100 nt and 6900 nt, 5200 nt and 6800 nt, 5300 nt and 6700 nt, 5400 nt and 6600 nt, 5500 nt and 6500 nt, 5600 nt and 6400 nt, 5700 nt and 6300 nt, or between 5800 nt and 6200 nt;
b) about 5000 nt, about 5100 nt, about 5200 nt, about 5300 nt, about 5400 nt, about 5500 nt, about 5600 nt, about 5700 nt, about 5800 nt, about 5900 nt, about 6000nt, about 6100 nt, about 6200 nt, about 6300 nt, about 6400 nt, about 6500 nt, about 6600 nt, about 6700 nt, about 6800 nt, about 6900 nt, or about 7000 nt; and/or
c) 5000 nt, 5100 nt, 5200 nt, 5300 nt, 5400 nt, 5500 nt, 5600 nt, 5700 nt, 5800 nt, 5900 nt, 6000nt, 6100 nt, 6200 nt, 6300 nt, 6400 nt, 6500 nt, 6600 nt, 6700 nt, 6800 nt, 6900 nt, or 7000 nt.

In one embodiment, the method further comprises a) i) admixing the at least one single-stranded DNA template strand and the set of oligonucleotides that are at least partially complementary to said single-stranded DNA template to form the admixture of the at least one single-stranded DNA template strand and a set of oligonucleotides that are at least partially complementary to said single-stranded DNA template.

Any suitable conditions may be used in step b). In one embodiment, in step b) the single-stranded DNA template strand and oligonucleotides of the set of oligonucleotides that are at least partially complementary to said single-stranded DNA template are:

a) incubated in a solution comprising $Mg^{2+}$ ions; and/or
b) subjected to a thermal annealing ramp.

The skilled person is aware of how to design a thermal annealing ramp. In some embodiments, the subjecting the single-stranded DNA template strand and oligonucleotides of the set of oligonucleotides that are at least partially complementary to said single-stranded DNA template to a thermal annealing ramp comprises:

i) heating the single-stranded DNA template strand and oligonucleotides of the set of oligonucleotides that are at least partially complementary to said single-stranded DNA template to a temperature of about 60°C, about 65°C, about 70°C, about 80°C, about 85°C, about 90°C, about 95°C, or about 98°C; and
ii) cooling the single-stranded DNA template strand and oligonucleotides of the set of oligonucleotides that are at least partially complementary to said single-stranded DNA template to 45°C or about 45°C.

In some non-limiting embodiments, cooling the single-stranded DNA template strand and oligonucleotides of the set of oligonucleotides that are at least partially complementary to said single-stranded DNA template to 45°C or about 45°C:

a) leaving the heated single-stranded DNA template strand and oligonucleotides of the set of oligonucleotides that are at least partially complementary to said single-stranded DNA template at room temperature until the single-stranded DNA template strand and oligonucleotides of the set of oligonucleotides that are at least partially complementary to said single-stranded DNA template have reached 45°C or about 45°C;
b) incubating the heated single-stranded DNA template strand and oligonucleotides of the set of oligonucleotides that are at least partially complementary to said single-stranded DNA template on ice or in a water bath until the single-stranded DNA template strand and oligonucleotides of the set of oligonucleotides that are at least partially complementary to said single-stranded DNA template have reached 45°C or about 45°C; and/or
c) decreasing the temperature of the heated single-stranded DNA template strand and oligonucleotides of the set of oligonucleotides that are at least partially complementary to said single-stranded DNA template by 1°C per hour until the single-stranded DNA template strand and oligonucleotides of the set of oligonucleotides that are at least partially complementary to said single-stranded DNA template have reached 45°C or about 45°C.

In some embodiments, the method further comprises b) i) purifying the DNA-based building blocks. In some embodiments, the method further comprises c) i) purifying the DNA-based nanostructure.

In some embodiments, the method further comprises d) stabilising the DNA-based nanostructures. In some embodiments, the DNA-based nanostructures are stabilised by UV-welding, chemical crosslinking, or coating.

In some embodiments, the method further comprises e) conjugating functional moieties to the DNA-based nanostructure. In some embodiments, the functional moieties are selected from the group comprising: virus-binding moieties, target-binding moieties, cell-binding moieties, and recruitment moieties as provided herein. The functional moieties may be conjugated to the DNA-based nanostructure in any suitable way. In some embodiments, the functional moieties are covalently conjugated to the DNA-based nanostructure, for example may be covalently conjugated to the DNA-based nanostructure in the same way as the virus-binding moieties described herein. Alternatively, the functional moieties may be or may be non-covalently conjugated to the DNA-based nanostructure, for example in the same way as the virus-binding moieties described herein.

In some embodiments, the method further comprises a final purification step.

Any suitable method of purifying nucleic acids may be used to purify the DNA-based building blocks, the DNA-based nanostructure, and/or in the final purification step. In some embodiments, purifying the DNA-based building blocks, purifying the DNA-based nanostructure, and/or the final purification step comprises gel purification. In some embodiments, purifying the DNA-based building blocks, purifying the DNA-based nanostructure, and/or the final purification step comprises ultrafiltration.

Also provided herein is a method of producing a self-assembling DNA-based subunit provided herein, comprising the steps of:

a) providing at least one single-stranded DNA template strand and a set of oligonucleotides that are at least partially complementary to said single-stranded DNA template; and
b) incubating an admixture of the at least one single-stranded DNA template strand and a set of oligonucleotides that are at least partially complementary to said single-stranded DNA template under conditions that permit annealing of the single-stranded DNA template strand and oligonucleotides of the set of oligonucleotides that are at least partially complementary to said single-stranded DNA template to form a plurality of DNA-based building blocks.

The single-stranded DNA template strand and a set of oligonucleotides that are at least partially complementary to said single-stranded DNA template may be any single-stranded DNA template strand and a set of oligonucleotides that are at least partially complementary to said single-stranded DNA template provided herein.

In some embodiments, the method further comprises a) i) admixing the at least one single-stranded DNA template strand and the set of oligonucleotides that are at least partially complementary to said single-stranded DNA template to form the admixture of the at least one single-stranded DNA template strand and a set of oligonucleotides that are at least partially complementary to said single-stranded DNA template.

In some embodiments, in step b) the single-stranded DNA template strand and oligonucleotides of the set of oligonucleotides that are at least partially complementary to said single-stranded DNA template are:

a) incubated in a solution comprising $Mg^{2+}$ ions; and/or
b) subjected to a thermal annealing ramp; optionally
wherein the single-stranded DNA template strand and oligonucleotides of the set of oligonucleotides that are at least partially complementary to said single-stranded DNA template:

i) heated to a temperature of about 60°C, about 65°C, about 70°C, about 80°C, about 85°C, about 90°C, about 95°C, or about 98°C; and
ii) cooled to 45°C or about 45°C.

In some embodiments, the method further comprises c) purifying the DNA-based building blocks. The DNA-based building blocks may be purified in any suitable manner, for example by gel purification and/or ultracentrifugation.

In some embodiments, the method further comprises d) conjugating functional moieties to the DNA-based nanostructure. In some embodiments, the functional moieties are selected from the group comprising: virus-binding moieties, target-binding moieties, cell-binding moieties, and recruitment moieties.

The functional moieties may be any functional moiety provided herein.

In some embodiments, the method further comprises a final purification step, for example by gel purification and/or ultracentrifugation.

Related aspects provided herein

Also provided herein is a kit comprising:

a) the DNA-based nanostructure provided herein;
b) the self-assembling DNA-based building block provided herein;
c) the DNA-based nanostructure provided herein; and/or
d) the composition provided herein.

Also provided herein is a kit comprising one or more components as described substantially herein.

Also provided herein is a neuraminic acid or derivative thereof is resistant to cleavage or degradation by a neuraminidase; or is a non-cleavable neuraminic acid or non-cleavable neuraminic acid derivative.

The Invention is also illustrated by the following non-limiting numbered paragraphs:

1. A DNA-based nanostructure, wherein the DNA-based nanostructure comprises at least a first plurality of virus-binding moieties, wherein each virus-binding moiety of the at least first plurality of virus-binding moiety is neuraminic acid or a derivative thereof.

2. The DNA-based nanostructure of paragraph 1, wherein the DNA-based nanostructure is formed by self-assembling DNA-based building blocks;

each of the self-assembling DNA-based building blocks is formed by a single-stranded DNA template strand and a set of oligonucleotides that are at least partially complementary to said single-stranded DNA template; and
wherein each of the oligonucleotides is either complementary to one contiguous DNA sequence stretch or to at least two non-contiguous DNA sequence stretches on said single-stranded DNA template.

3. The DNA-based nanostructure of any of the preceding paragraphs, wherein the DNA-based nanostructure comprises a second plurality of virus-binding moieties; optionally
wherein the DNA-based nanostructure comprises a third plurality of virus-binding moieties, a fourth plurality of virus binding moieties, a fifth plurality of virus binding moieties, and/or a sixth plurality of virus binding moieties.

4. The DNA-based nanostructure of paragraph 3, wherein the virus binding moieties of the second, and when present, third, fourth, fifth, and/or sixth pluralities of virus binding moieties are selected from the group comprising or consisting of: an antibody or a virus-binding fragment thereof, a nanobody or a virus-binding fragment thereof, an affimer, heparin, heparan sulfate, hybrid heparan sulfates, a host receptor protein or virus-binding fragment thereof, a de novo virus-binding peptide, an aptamer, and a neuraminidase inhibitor; optionally
wherein the neuraminidase inhibitor is selected from the group comprising or consisting of: oseltamivir, zanamivir, ianamivir, and peramivir; optionally wherein the neuraminidase inhibitor is oseltamivir.

5. The DNA-based nanostructure of paragraph 3 or 4, wherein at least two of the pluralities of the virus-binding moiety are different neuraminic acids or are derivatives thereof.

6. The DNA-based nanostructure of any one of paragraphs 1-5, wherein the virus-binding moieties are covalently bound to the nanostructure, optionally wherein:

a) the virus-binding moieties are covalently bound to the single-stranded DNA template strand; and/or
b) the virus-binding moieties are covalently bound to the oligonucleotides of the set of oligonucleotides complementary to said single-stranded DNA template.

7. The DNA-based nanostructure of paragraph 6, wherein the virus-binding moieties are covalently bound to nanostructure by a linker; optionally wherein the linker is selected from the group comprising or consisting of:

a) an oligo-PEG-linker, optionally wherein the oligo-PEG linker has the structure:

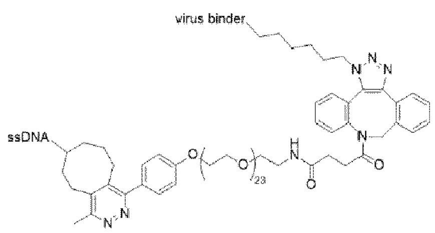

;

b) a PEG linker, optionally wherein the PEG linker has the structure:

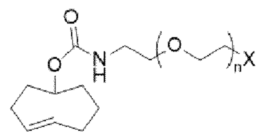

wherein n is an integer between 4 and 227 and X is a linker covalently bound to the virus-binding moiety, optionally wherein the linker is DBCO.

8. The DNA-based nanostructure of any one of paragraphs 1-7, wherein the structure comprises one or more handle sites; optionally wherein the one or more handle sites comprise single-stranded DNA overhangs.

9. The DNA-based nanostructure of paragraph 8, wherein the handle sites are comprised by the self-assembling DNA building blocks, optionally wherein each self-assembling DNA building block comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 handle sites.

10. The DNA-based nanostructure of any of paragraphs 8 or 9, wherein said handles are single-stranded oligonucleotides having a length of between 20 and 200 nucleotides, 50 and 150 nucleotides, 75 and 125 nucleotides, 20 and 60 nucleotides, 40 and 55 nucleotides; 20 and 30 nucleotides, 22 and 28 nucleotides, 25 and 27 nucleotides, 45 and 55 nucleotides, or 47 and 53 nucleotides.

11. The DNA-based nanostructure of any one of paragraphs 8-10, wherein the virus-binding moieties are non-covalently bound to the nanostructure; optionally wherein the virus-binding moieties are conjugated to a handle-binding oligonucleotide that is able to hybridise to the handle sites; optionally wherein the handle-binding oligonucleotide is complementary to the handle site.

12. The DNA-based nanostructure of paragraph 10 or 11, wherein the handle-binding oligonucleotide is covalently bound to the handle site; optionally wherein the handle-binding oligonucleotide is covalently bound to the handle site by UV-welding.

13. The DNA-based nanostructure of any of one of paragraphs 11 or 12, wherein the virus-binding moieties are conjugated to the handle-binding oligonucleotide:

a) by an ssDNA-PEG$_4$-azide linker; optionally wherein the ssDNA-PEG$_4$-azide linker has the structure:

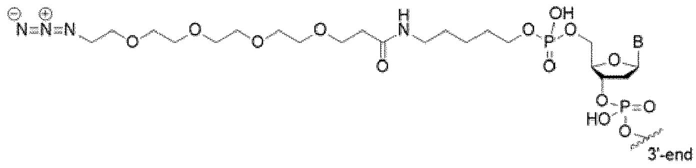
;

b) an ssDNA-DBCO linker; optionally wherein the ssDNA-DBCO linker has the structure:

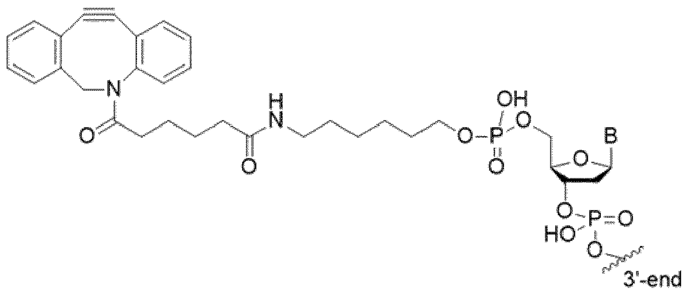
;

c) an ssDNA-SH linker; optionally wherein the ssDNA-SH linker has the structure:

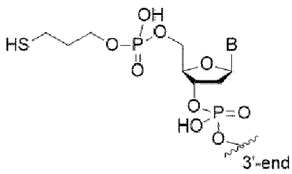
;

d) an ssDNA-PEGs-methyltetrazine linker; optionally wherein the ssDNA-PEGs-methyltetrazine linker has the structure:

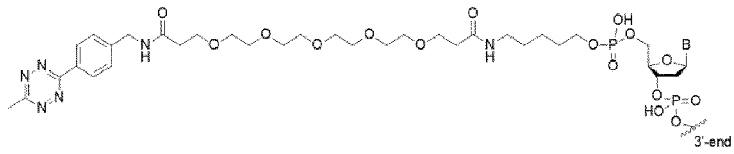
;

and/or

e) an oligo-PEG-linker, optionally wherein the oligo-PEG-linker has the structure:

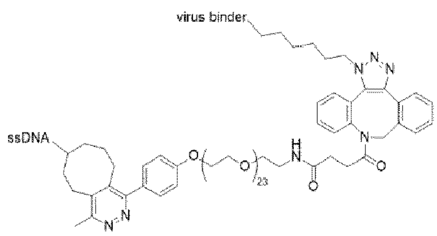
.

14. The DNA-based nanostructure of any of the preceding paragraphs, wherein the neuraminic acid or derivative thereof is resistant to cleavage or degradation by a neuraminidase; or is a non-cleavable neuraminic acid or non-cleavable neuraminic acid derivative.

15. The DNA-based nanostructure of any of the preceding paragraphs, wherein the neuraminic acid or derivative thereof has a structure selected from the group comprising or consisting of:

a)

Compound I:

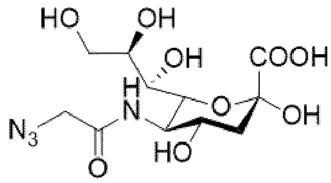

;

b)

Compound II:

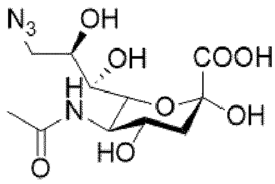

;

c)

Compound III:

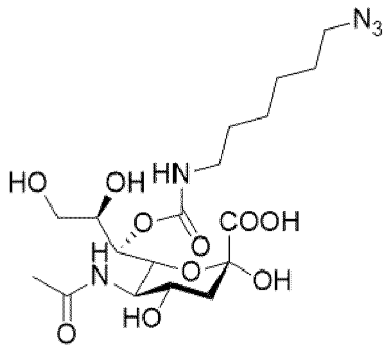

;

d)

Compound IV:

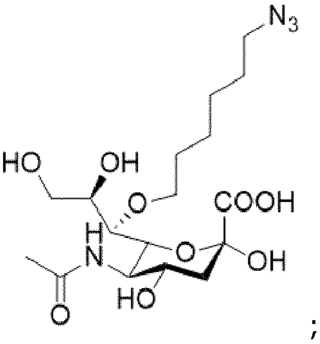

;

e)

Compound Va:

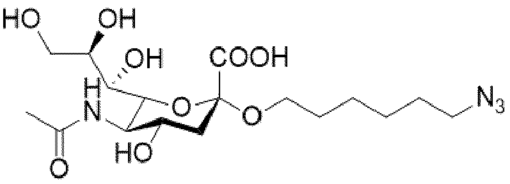

;

f)

Compound Vb:

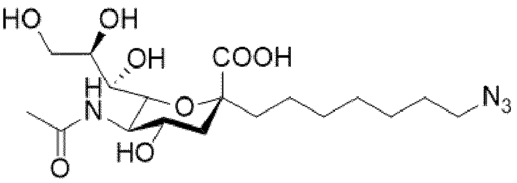

;

and

g)

Compound VI:

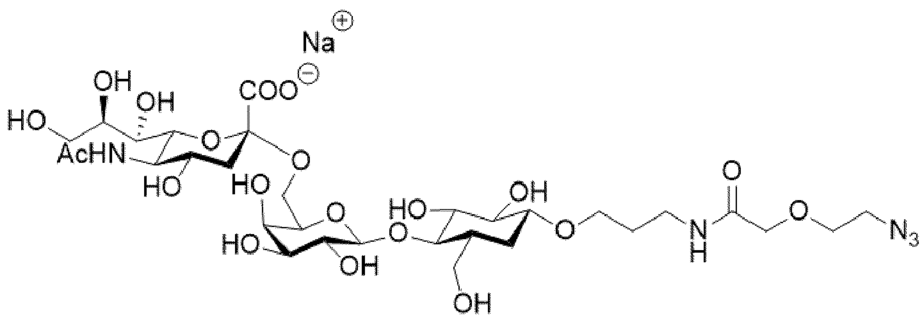

.

16. The DNA-based nanostructure of any of the preceding paragraphs, wherein each self-assembling DNA-based building block of the nanostructure comprises at least one virus-binding moiety of the first plurality of virus-binding moieties.

17. The DNA-based nanostructure of any of paragraphs 2-16, wherein each self-assembling DNA based building block comprises:

a) at least one virus-binding moiety of the first plurality of virus-binding moieties, at least one virus-binding moiety of the second plurality of virus-binding moieties, at least one virus-binding moiety of the third plurality of virus-binding moieties, at least one virus-binding moiety of the fourth plurality of virus binding moieties, at least one virus-binding moiety of the fifth plurality of virus binding moieties, and/or at least one virus-binding moiety of the sixth plurality of virus binding moieties;

b) at least one virus-binding moiety of the first plurality of virus-binding moieties, at least one virus-binding moiety of the second plurality of virus-binding moieties, at least one virus-binding moiety of the third plurality of virus-binding moieties, at least one virus-binding moiety of the fourth plurality of virus binding moieties, at least one virus-binding moiety of the fifth plurality of virus binding moieties, and at least one virus-binding moiety of the sixth plurality of virus binding moieties;

c) at least one virus-binding moiety of the first plurality of virus-binding moieties, at least one virus-binding moiety of the second plurality of virus-binding moieties, at least one virus-binding moiety of the third plurality of virus-binding moieties, at least one virus-binding moiety of the fourth plurality of virus binding moieties, at least one virus-binding moiety of the fifth plurality of virus binding moieties, or at least one virus-binding moiety of the sixth plurality of virus binding moieties.

18. The DNA-based nanostructure of any of the preceding paragraphs, wherein each self-assembling DNA-based building block of the nanostructure comprises:

a) between 1-30 virus-binding moieties, such as 2-28, 2-26, 4-24, 6-22, 8-20, 10-18, or 12-16 virus binding moieties;

b) at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 27, at least 28, at least 29, or more virus-binding moieties;

c) about 1, about 2, about 3, about 4, about 5, about 6, about 7, about 8, about 9, about 10, about 11, about 12, about 13, about 14, about 15, about 16, about 17, about 18, about 19, about 20, about 22, about 23, about 24, about 25, about 26, about 27, about 28, about 29, or about 30 virus-binding moieties; and/or

d) 1, 2, 3, 4, 5, 6, 7, 8, 9, 10, 11, 12, 13, 14, 15, 16, 17, 18, 19, 20, 21, 22, 23, 24 25, 26, 27, 28, 29, or 30 virus-binding moieties.

19. The DNA-based nanostructure of any of the preceding paragraphs, wherein the nanostructure is configured to bind to the surface of a target virus or to at least partially encapsulate a target virus, optionally wherein the target virus is an influenza virus.

20. The DNA-based nanostructure of paragraph 19, wherein the nanostructure is configured to bind to:

a) at least 5% of the surface of the target virus, optionally at least 10%, at least 20%, at least 30%, at least 40%, at least 50%, at least 60%, at least 70%, at least 75%, at least 80%, at least 85%, at least 90%, at least 95%, at least 96%, at least 97%, at least 98%, at least 99%, or more of the surface of the target virus;

b) about 5% of the surface of the target virus, optionally about 10%, about 20%, about 30%, about 40%, about 50%, about 60%, about 70%, about 75%, about 80%, about 85%, about 90%, about 95%, about 96%, about 97%, about 98%, about 99%, or 100% of the surface of the target virus; and/or

c) 5% of the surface of the target virus, optionally 10%, 20%, 30%, 40%, 50%, 60%, 70%, 75%, 80%, 85%, 90%, 95%, 96%, 97%, 98%, 99%, 100% of the target virus.

21. The DNA-based nanostructure of any of paragraphs 19-20, wherein the nanostructure is configured to fully encapsulate the target virus.

22. The DNA-based nanostructure of any of paragraphs 19-21, wherein the influenza virus is selected from: Influenza A, Influenza B, Influenza C, and Influenza D; optionally

Wherein the influenza virus is Influenza A or Influenza B.

23. The DNA-based nanostructure of any of paragraphs 19-22, wherein:

a) the influenza virus has a haemagglutinin subtype selected from the group comprising or consisting of: H1, H2, H3, H4, H5, H6, H7, H8, H9, H10, H11, H12, H13, H14, H15, H16, H17, and H18; and/or

b) the influenza virus has a neuraminidase subtype selected from: N1, N2, N3, N4, N5, N6, N7, N8, N9, N10, and N11.

24. The DNA-based nanostructure of any of paragraphs 19-23, wherein the influenza virus has a subtype selected from the group comprising or consisting of: H1N1, H2N2, H3N2, H5N1, H5N5, H5N6, H5N8, H6N1, H7N2, H7N7, H7N9, and H10N7; optionally
Wherein the influenza has a subtype that is H3N2, H5N1, H7N9, or H1N1.

25. The DNA-based nanostructure of any of paragraphs 19-24, wherein binding of the nanostructure to the target virus inhibits the virus from binding to and/or entering a host cell; optionally
wherein the host cell is an animal cell, optionally wherein the host cell is a human cell.

26. The DNA-based nanostructure of paragraphs 19-25, wherein binding of the nanostructure to the target virus neutralises the target virus, optionally wherein binding of the nanostructure to the target virus neutralises the target virus:

a) to at least 50%, at least 60%, at least 70%, at least 80%, at least 85%, at least 90%, at least 95%, at least 96%, at least 97%, at least 98%, at least 99%, or more;
b) to about 50%, about 60%, about 70%, about 80%, about 85%, about 90%, about 95%, about 96%, about 97%, about 98%, about 99%, or about 100%; and/or
c) to 50%, 60%, 70%, 80%, 85%, 90%, 95%, 96%, 97%, 98%, 99%, or 100%.

27. The DNA-based nanostructure of any one of paragraphs 1-27, wherein the nanostructure further comprises at least a first plurality of recruitment moieties, optionally wherein each recruitment moiety of the first plurality of recruitment moieties is selected from the group comprising or consisting of: an antibody Fc fragment, a TLR ligand, and an opsonin.

28. The DNA-based nanostructure of any of the preceding paragraphs, wherein:

a) each of the self-assembling DNA-based building blocks has an identical shape; or
b) the self-assembling DNA-based building blocks have a plurality of different shapes.

29. The DNA-based nanostructure of any of the preceding paragraphs, wherein the DNA-based nanostructure has a concave surface and/or a convex surface.

30. The DNA-based nanostructure of paragraph 29, wherein:

a) the at least a first plurality of virus-binding moieties is bound to the concave surface; optionally
wherein when present the second plurality of virus-binding moieties, third plurality of virus-binding moieties, fourth plurality of virus binding moieties, fifth plurality of virus binding moieties, and/or sixth plurality of virus binding moieties are bound to the concave surface; and/or
b) when present, the at least a first plurality of recruitment moieties is bound to the convex surface.

31. The DNA-based nanostructure of any of the preceding paragraphs, wherein each of the DNA-based building blocks is a triangular prismoid.

32. The DNA-based nanostructure of paragraph 31, wherein each said triangular prismoid is formed by m triangular planes, wherein m is an integer independently selected from 4, 5, 6, 7 and 8, in particular independently selected from 5, 6 and 7, more particularly wherein said integer is 6;

the three edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4;
wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes; and
wherein at least two of the three side trapezoids comprise a specific pattern of recesses and/or extrusions formed by missing or additional DNA double helical stretches for specific interaction with a complementary pattern on the side trapezoid of another one of said self-assembling DNA-based building blocks.

33. The DNA-based nanostructure of paragraph 32, wherein for at least part of the self-assembling DNA-based building blocks the length of at least one edge of each of said m planes is decreasing from the first to the $m^{th}$ plane, so that bevel angles results between planes perpendicular to said first plane and each of the trapezoid planes formed by said m edges.

34. The DNA-based nanostructure of paragraph 32 or 33 comprising at least one set of self-assembling DNA based-building blocks, wherein one, two, or all three side trapezoids comprises a specific pattern of recesses and/or extrusions formed by missing or additional DNA double helical stretches for specific interaction with a complementary pattern on the side trapezoid of another one of said self-assembling DNA-based building blocks.

35. The DNA-based nanostructure of paragraph 34, wherein two of the side trapezoids are capable of specifically interacting with a complementary pattern on the side trapezoid of another one of said self-assembling DNA-based building blocks, and one of the side trapezoids is not capable of said specific interaction.

36. The DNA-based nanostructure of any one of paragraphs 2-35, wherein the nanostructure comprises or consists of a pentamer of the DNA-based building blocks; optionally
wherein the DNA-based building blocks each have an identical shape.

37. The DNA-based nanostructure of any one of paragraphs 1-29, wherein the DNA-based nanostructure is a shell that defines a cavity at least partially enclosed by the DNA-based nanostructure.

38. The DNA-based nanostructure of paragraph 37, wherein:

a) the at least first plurality of virus-binding moieties is bound to the cavity facing surface of the shell; optionally wherein when present the second plurality of virus-binding moieties, third plurality of virus-binding moieties, fourth plurality of virus binding moieties, fifth plurality of virus binding moieties, and/or sixth plurality of virus binding moieties are bound the cavity facing surface of the shell; and/or
b) when present, the at least a first plurality of recruitment moieties is bound to the exterior surface.

39. The DNA-based nanostructure of any of paragraphs 37 and 38, wherein the nanostructure has a closed three-dimensional geometric shape, in particular a closed three-dimensional geometric shape selected from a sphere, a spherocylinder, and a polyhedron, in particular a tetrahedron, an octahedron or an icosahedron.

40. The DNA-based nanostructure of any one of paragraphs 37-39, wherein the nanostructure is a shell with an opening for accessing the cavity.

41. The DNA-based nanostructure of any one of paragraphs 37-40, wherein the nanostructure is a combination of a first and a second subshell, each with an opening to access a first and a second inner cavity, respectively, wherein said first and said second inner cavity together form said cavity, in particular wherein said first and said second subshell are connected by at least one linker.

42. The DNA-based nanostructure of any one of paragraphs 37-41, wherein the nanostructure is based on an icosahedral structure.

43. The DNA-based nanostructure of paragraph 44, wherein each of said self-assembling DNA-based building blocks is a triangular and/or a rectangular prismoid, particularly a triangular prismoid.

44. The DNA-based nanostructure of any one of paragraphs 42-43,

wherein each said triangular and/or a rectangular prismoid is formed by m triangular, or rectangular, respectively, planes, wherein m is an integer independently selected from 4, 5, 6, 7 and 8, in particular independently selected from 5, 6 and 7, more particularly wherein said integer is 6;
the three, or four, respectively, edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4;
wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two

of said planes; and
wherein at least two of the three, or four, respectively, side trapezoids comprise a specific pattern of recesses and/or extrusions formed by missing or additional DNA double helical stretches for specific interaction with a complementary pattern on the side trapezoid of another one of said self-assembling DNA-based building blocks.

45. The DNA-based nanostructure of paragraph 44, wherein for at least part of the self-assembling DNA-based building blocks the length of at least one edge of each of said m planes is decreasing from the first to the $m^{th}$ plane, so that bevel angles results between planes perpendicular to said first plane and each of the trapezoid planes formed by said m edges.

46. The DNA-based nanostructure of paragraph 44 or 45 comprising at least one set of self-assembling DNA based-building blocks, wherein all three or four, respectively, side trapezoids comprises a specific pattern of recesses and/or extrusions formed by missing or additional DNA double helical stretches for specific interaction with a complementary pattern on the side trapezoid of another one of said self-assembling DNA-based building blocks.

47. The DNA-based nanostructure of any one of paragraphs 37-46, wherein the DNA-based nanostructure is a half shell selected from

(a) a half octahedron DNA-based nanostructure based on T_octa self-assembling DNA-based building blocks, which consists of a set of four copies of a triangular frustum, wherein the base-pair stacking contacts on one of the triangular edges of the triangular frustum are inactivated by either strand shortening or by adding unpaired thymidines;
(b) a T=1 half shell based on T1_pentamer triangle self-assembling DNA-based building blocks, which consists of two sets of in each case five copies of two different triangular frusta, wherein the five copies of the first set form a closed pentamer, and the five copies of the second set dock onto the edges of said pentamer; and
(c) a "trap" T=1 half shell with a missing pentagon vertex based on a combination of T1_pentamer triangle and T1_ring triangle self-assembling DNA-based building blocks, which consists of three sets of in each case five copies of three different triangular frusta, wherein the five copies of the first set form a closed pentamer, the five copies of the second set dock onto the edges of said pentamer the five copies of the second set dock onto the edges of said pentamer, and the five copies of the third set dock into the gaps between the five copies of said second set;
(d) a T=3 icosahedral half shell based on T3_6_triangle based self-assembling DNA-based building blocks, which consists of a total of 30 triangular subunits partitioned as five copies of six different full-size DNA triangle designs with specific edge docking rules.

48. The DNA-based nanostructure of any one of paragraphs 43 to 47, further comprising

(a) one or more types of DNA brick constructs, each type of such DNA brick constructs being characterized by one or more interaction sites for specific interaction by edge-to-edge stacking contacts with one or more complementary interaction sites present on the plane of a triangular or rectangular frustum on the outer surface of said DNA-based nanostructure, wherein said DNA brick constructs cover the free space between the three, or four, respectively, edges of said plane;
(b) one or more cross-linkages within one of said triangular or rectangular prismoids, and/or between two of said triangular and/or rectangular prismoids; and/or
(c) at least one moiety specifically interacting with said viruses or viral particles.

49. The DNA-based nanostructure of any of paragraphs 19-48, wherein the nanostructure is formed *in situ* from the self-assembling DNA-based building blocks in the presence of the target virus.

50. The DNA-based nanostructure of any one of paragraphs 1-37, wherein the nanostructure is a three-dimensional open shell defining a cavity and comprising an opening for accessing said cavity, comprising:
an n-gonal pyramid formed by n identical copies of a first type of an acute isosceles triangular prismoid t1, wherein n is an integer selected from 7, 8, 9, 10, 11, 12, 13, 14 and 15, wherein the base plane of each prismoid points to the outside of said open shell, and the upper plane points to said cavity, wherein the two large side planes of each prismoid contain a first pattern and a second pattern of one or more protrusions and/or one or more receptacles, wherein said first and said second patterns are complementary to each other, and wherein the small side plane comprises a third pattern of one or more protrusions and/or one or more receptacles; wherein said first type of an acute isosceles triangular prismoid is a self-assembling DNA-based building block.

51. The DNA-based nanostructure of paragraph 50, further comprising n copies of a second type of an acute isosceles triangular prismoid t2, wherein a first side of each prismoid points to the outside of said open shell, and the opposite side points to said cavity and/or to said opening for accessing said cavity, wherein one plane of said second type of prismoid structure comprises a fourth pattern of one or more protrusions and/or one or more receptacles which is complementary to said third pattern.

52. The DNA-based nanostructure of any one of paragraphs 50-51, wherein n is an integer selected from 9, 10, 11, 12 and 13.

53. The DNA-based nanostructure of any one of paragraphs 2-52, further comprising chemical crosslinks between different self-assembling DNA-based building blocks; optionally wherein said chemical crosslinks are obtained by UV irradiation.

54. A self-assembling DNA-based building block formed by a single-stranded DNA template strand and a set of oligonucleotides complementary to said single-stranded DNA template, wherein:

each of the oligonucleotides is either complementary to one contiguous DNA sequence stretch or to at least two non-contiguous DNA sequence stretches on said single-stranded DNA template; and
the self-assembling DNA-based building block comprises at least one first virus-binding moiety, wherein the first virus-binding moiety is neuraminic acid or a derivative thereof.

55. The self-assembling DNA-based building block of paragraph 54, wherein the building block comprises at least a plurality of first virus-binding moieties that is a first plurality of virus-binding moieties; optionally
wherein the building block further comprises a second plurality of virus-binding moieties, a third plurality of virus-binding moieties, a fourth plurality of virus binding moieties, a fifth plurality of virus binding moieties, and/or a sixth plurality of virus binding moieties.

56. The self-assembling DNA-based building block of paragraph 55, wherein:

a) the virus-binding moiety is covalently bound to the single-stranded DNA template strand; and/or
b) the at least one virus-binding moiety is covalently bound to the oligonucleotides of the set of oligonucleotides complementary to said single-stranded DNA template.

57. The self-assembling DNA-based building block of paragraph 56, wherein the at least one virus-binding moiety is covalently bound to the building block by a linker; optionally wherein the linker is selected from the group comprising or consisting of:

a) an oligo-PEG-linker, optionally wherein the oligo-PEG linker has the structure:

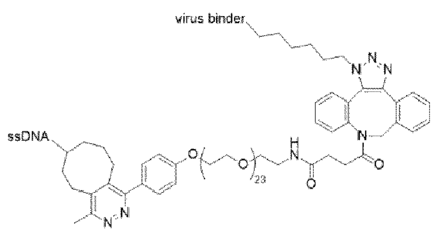

;

b) a PEG linker, optionally wherein the PEG linker has the structure:

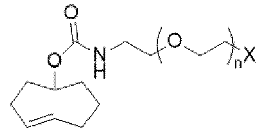

wherein n is an integer between 4 and 227 and X is a linker covalently bound to the virus-binding moiety, optionally wherein the linker is DBCO.

58. The self-assembling DNA-based building of paragraph 54, wherein the structure comprises one or more handle sites; optionally wherein the one or more handle sites comprise single-stranded DNA overhangs.

59. The self-assembling DNA-based building block of paragraph 58, wherein the handle sites are comprised by the self-assembling DNA building blocks, optionally wherein each self-assembling DNA building block comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 handle sites.

60. The self-assembling DNA-based building block of any of paragraphs 58 or 59, wherein said handles are single-stranded oligonucleotides having a length of between 20 and 200 nucleotides, 50 and 150 nucleotides, 75 and 125 nucleotides, 20 and 60 nucleotides, 40 and 55 nucleotides; 20 and 30 nucleotides, 22 and 28 nucleotides, 25 and 27 nucleotides, 45 and 55 nucleotides, or 47 and 53 nucleotides.

61. The self-assembling DNA-based building block of any one of paragraphs 58-60, wherein at least one virus-binding moiety is non-covalently bound to the building block; optionally wherein the at least one virus-binding moiety is conjugated to a handle-binding oligonucleotide that is complementary to the handle sites; optionally wherein the handle-binding oligonucleotide is hybridised to the handle site.

62. The DNA-based nanostructure of paragraph 61, wherein the handle-binding oligonucleotide is covalently bound to the handle site; optionally wherein the handle-binding oligonucleotide is covalently bound to the handle site by UV-welding.

63. The self-assembling DNA nanostructure of paragraph 61 or 62, wherein the virus-binding moieties are conjugated to the handle-binding oligonucleotide:

a) by an ssDNA-$PEG_4$-azide linker; optionally wherein the ssDNA-$PEG_4$-azide linker has the structure:

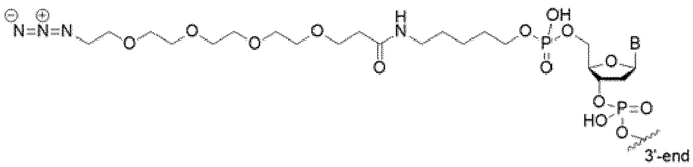
;

b) an ssDNA-DBCO linker; optionally wherein the ssDNA-DBCO linker has the structure:

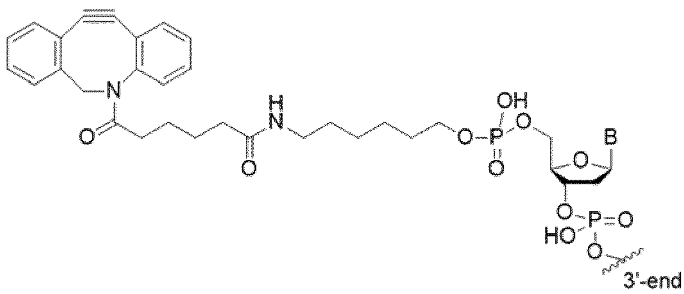
;

c) an ssDNA-SH linker; optionally wherein the ssDNA-SH linker has the structure:

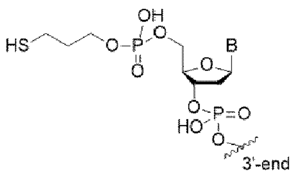
,

d) an ssDNA-PEGs-methyltetrazine linker; optionally wherein the ssDNA-PEGs-methyltetrazine linker has the structure:

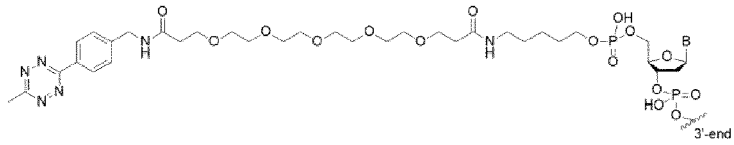 ;

and/or e) an oligo-PEG-linker, optionally wherein the oligo-PEG-linker has the structure:

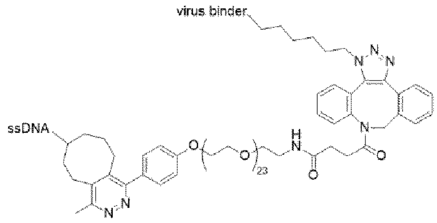 .

64. The self-assembling DNA-based building block of paragraph 60 or 61, wherein the handle-binding oligonucleotide is covalently bound to the handle site.

65. The self-assembling DNA-based building block of any of paragraphs 54-61, wherein the neuraminic acid or derivative thereof is resistant to cleavage or degradation by a neuraminidase; or is a non-cleavable neuraminic acid or non-cleavable neuraminic acid derivative.

66. The self-assembling DNA-based building block of any of paragraphs 54-65, wherein the neuraminic acid or derivative thereof has a structure selected from the group comprising or consisting of:

a)

Compound I:

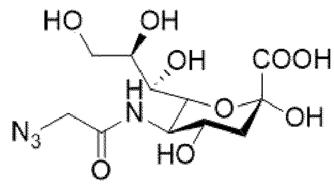 ;

b)

Compound II:

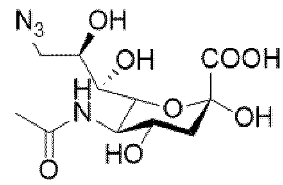 ;

c)

Compound III:

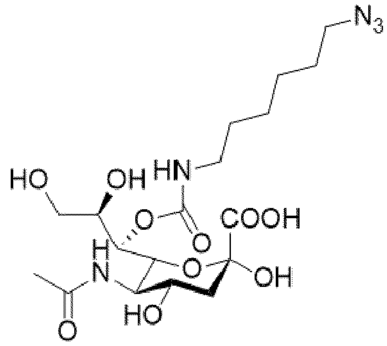

;

d)

Compound IV:

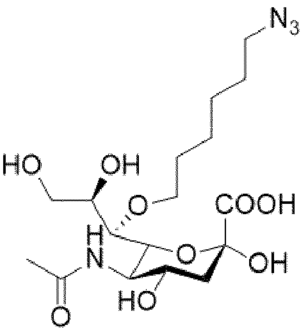

;

e)

Compound Va:

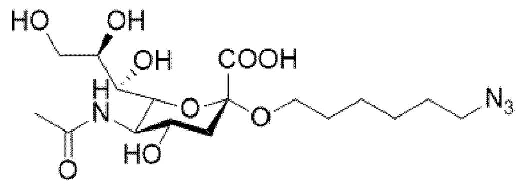

;

f)

Compound Vb:

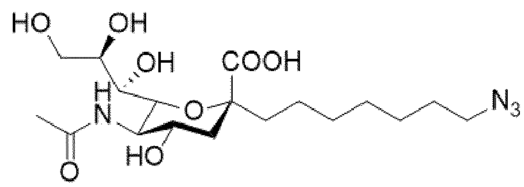

;

and

g)

Compound VI:

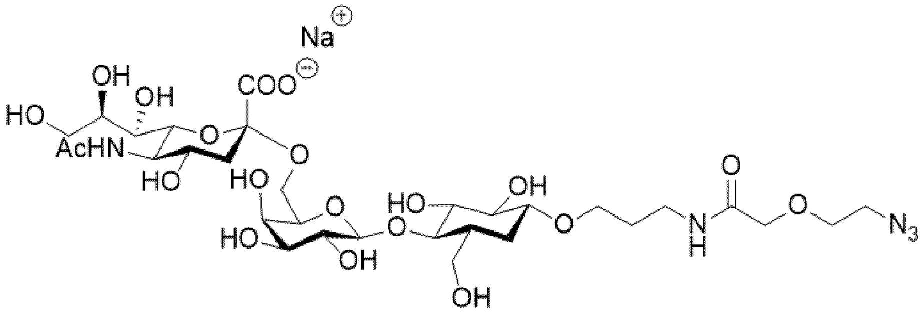

67. The self-assembling DNA-based building block of any one of paragraphs 54-66, wherein the self-assembling DNA-based building block comprises a plurality of virus binding moieties; optionally wherein:

a) each virus binding moiety is identical; or
b) each virus binding moiety is selected from a first virus-binding moiety, a second virus-binding moiety, a third virus-binding moiety, a fourth virus-binding moiety, a fifth virus-binding moiety, and/or a sixth virus binding moiety.

68. The self-assembling DNA-based building block of paragraph 67, wherein the self-assembling DNA-based building block comprises:

a) at least one first virus-binding moiety, at least one second virus-binding moiety, at least one third virus-binding moiety, at least one fourth virus-binding moiety, at least one fifth virus-binding moiety, and/or at least one sixth virus-binding moiety;
b) at least one first virus-binding moiety, at least one second virus-binding moiety, at least one third virus-binding moiety, at least one fourth virus-binding moiety, at least one fifth virus-binding moiety, and at least one sixth virus-binding moiety;
c) at least one first virus-binding moiety, at least one second virus-binding moiety, at least one third virus-binding moiety, at least one fourth virus-binding moiety, at least one fifth virus-binding moiety, or at least one sixth virus-binding moiety.

69. The self-assembling DNA-based building block of any of paragraphs 54-67, wherein the self-assembling DNA-based building block comprises:

a) between 1-30 virus-binding moieties, such as 2-28, 2-26, 4-24, 6-22, 8-20, 10-18, or 12-16 virus binding moieties;
b) at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 27, at least 28 at least 29, or more virus-binding moieties;
c) about 1, about 2, about 3, about 4, about 5, about 6, about 7, about 8, about 9, about 10, about 11, about 12, about 13, about 14, about 15, about 16, about 17, about 18, about 19, about 20, about 22, about 23, about 24, about 25, about 26, about 27, about 28, about 29, or about 30 virus-binding moieties; and/or
d) 1, 2, 3, 4, 5, 6, 7, 8, 9, 10, 11, 12, 13, 14, 15, 16, 17, 18, 19, 20, 21, 22, 23, 24 25, 26, 27, 28, 29, or 30 virus-binding moieties.

70. The self-assembling DNA-based building block of any of paragraphs 54-69, wherein the self-assembling DNA-based building block is a triangular and/or a rectangular prismoid, particularly a triangular prismoid.

71. The self-assembling DNA-based building block of paragraph 70, wherein the length of at least one edge of each of said m planes is decreasing from the first to the $m^{th}$ plane, so that bevel angles results between planes perpendicular to said first plane and each of the trapezoid planes formed by said m edges.

72. The self-assembling DNA based-building block of paragraph 70 or 71, wherein one, two, or all three side trapezoids comprises a specific pattern of recesses and/or extrusions formed by missing or additional DNA double helical stretches for specific interaction with a complementary pattern on the side trapezoid of another one of said self-assembling DNA-based building blocks.

73. The self-assembling DNA-based building block of paragraph 72, wherein two of the side trapezoids are capable of specifically interacting with a complementary pattern on the side trapezoid of another one of said self-assembling DNA-based building blocks, and one of the side trapezoids is not capable of said specific interaction.

74. The self-assembling DNA-based building block of any of paragraphs 54-73, wherein the self-assembling DNA-based building block is configured to bind a target virus, optionally wherein the target virus is an influenza virus.

75. The self-assembling DNA-based building block of paragraph 74, wherein the influenza virus is as defined in any one of paragraphs 22-24.

76. The self-assembling DNA-based building block of any of paragraphs 74-75, wherein the self-assembling DNA-based building block is configured to form the nanostructure of any of paragraphs 1-53 with a plurality of self-assembling DNA-based building blocks *in situ* in the presence of the target virus.

77. The self-assembling DNA-based building block of paragraph 76, wherein each self-assembling DNA-based building block of the plurality of self-assembling DNA-based building blocks is a self-assembling DNA-based building block according to any one of paragraphs 54-76.

78. A DNA-based nanostructure wherein the DNA-based nanostructure comprises at least five self-assembling DNA-based building blocks;

each of the self-assembling DNA-based building blocks is formed by a single-stranded DNA template strand and a set of oligonucleotides that are at least partially complementary to said single-stranded DNA template; and wherein each of the oligonucleotides is either complementary to one contiguous DNA sequence stretch or to at least two non-contiguous DNA sequence stretches on said single-stranded DNA template.

79. The DNA-based nanostructure of paragraph 78, wherein the DNA-based nanostructure has a concave surface and/or a convex surface.

80. The DNA-based nanostructure of any of the preceding paragraphs, wherein each of the DNA-based building blocks is a triangular prismoid; optionally
wherein the DNA-based building blocks form a symmetrical structure.

81. The DNA-based nanostructure of paragraph 80, wherein each said triangular prismoid is formed by m triangular planes, wherein m is an integer independently selected from 4, 5, 6, 7 and 8, in particular independently selected from 5, 6 and 7, more particularly wherein said integer is 6;

the three edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4;
wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes; and
wherein at least two of the three side trapezoids comprise a specific pattern of recesses and/or extrusions formed by missing or additional DNA double helical stretches for specific interaction with a complementary pattern on the side trapezoid of another one of said self-assembling DNA-based building blocks.

82. The DNA-based nanostructure of paragraph 81, wherein for at least part of the self-assembling DNA-based building blocks the length of at least one edge of each of said m planes is decreasing from the first to the $m^{th}$ plane, so that bevel angles results between planes perpendicular to said first plane and each of the trapezoid planes formed by said m edges.

83. The DNA-based nanostructure of paragraph 81 or 82 comprising at least one set of self-assembling DNA based-building blocks, wherein one, two, or all three side trapezoids comprises a specific pattern of recesses and/or extrusions formed by missing or additional DNA double helical stretches for specific interaction with a complementary pattern on the side trapezoid of another one of said self-assembling DNA-based building blocks; optionally
wherein two of the side trapezoids are capable of specifically interacting with a complementary pattern on the side

trapezoid of another one of said self-assembling DNA-based building blocks, and one of the side trapezoids is not capable of said specific interaction.

84. The DNA-based nanostructure of any one of paragraphs 78-83, wherein the nanostructure comprises at least a first plurality of target-binding moieties, optionally wherein:

a) the at least first plurality of target-binding moieties is a virus-binding moiety that is a neuraminic acid or a derivative thereof; or
b) the at least first plurality of target-binding moieties is a virus-binding moiety selected from the group comprising or consisting of: an antibody or a virus-binding fragment thereof, a nanobody or virus-binding fragment thereof, an affimer, heparin, heparan sulfate, hybrid heparan sulfates, a host receptor protein or virus-binding fragment thereof, a de novo virus-binding peptide, an aptamer, and a neuraminidase inhibitor; optionally
wherein the neuraminidase inhibitor is selected from the group comprising or consisting of: oseltamivir, zanamivir, ianamivir, and peramivir; optionally wherein the neuraminidase inhibitor is oseltamivir;
c) the at least first plurality of target-binding moieties is a cell-binding moiety, optionally a cell-binding moiety that is capable of binding to the surface of a cell selected from the group comprising or consisting of: a bacterial cell, an archaeal cell, a fungal cell, an animal cell, a mammalian cell, a plant cell, an amoeba cell, a protozoan cell, a helminth cell, a sporozoan cell, a ciliate cell, a trematode cell, a nematode cell, and a cestode cell; optionally
wherein the cell-binding moiety is selected from the group comprising or consisting of: an antibody or cell-binding fragment thereof, a nanobody or cell-binding fragment thereof, heparin, heparan sulfate, hybrid heparan sulfates, a host receptor protein or virus-binding fragment thereof, a de novo virus-binding peptide, an aptamer, and an affimer.

85. The DNA-based nanostructure of paragraph 84, wherein the at least a first plurality of target-binding moieties is bound to the concave surface; optionally
wherein when present the second plurality of target-binding moieties, third plurality of target-binding moieties, fourth plurality of target-binding moieties, fifth plurality of target-binding moieties, and/or sixth plurality of target-binding moieties are bound to the concave surface.

86. The DNA-based nanostructure of any one of paragraphs 78-85, wherein the nanostructure further comprises at least a first plurality of recruitment moieties, optionally wherein each recruitment moiety of the first plurality of recruitment moieties is selected from the group comprising or consisting of: an antibody Fc fragment, a TLR ligand, and an opsonin.

87. The DNA-based nanostructure of paragraph 85-86, wherein the nanostructure is a nanostructure according to any one of paragraphs 2-36, and/or wherein each self-assembling DNA-based building block is a self-assembling DNA-based building block of any one of paragraphs 54-77.

88. A nucleic acid conjugated to a neuraminic acid residue or a derivative thereof.

89. A composition comprising the self-assembling DNA building block of any one of paragraphs 54-77.

90. A composition comprising a plurality of self-assembling DNA building blocks of any one of paragraphs 54-77.

91. A plurality of self-assembling DNA-based building blocks, wherein each self-assembling DNA-based building block of the plurality of self-assembling DNA-based building blocks is a self-assembling DNA-based building block according to any one of paragraphs 54-77.

92. A composition comprising the plurality of self-assembling DNA-based building blocks of paragraph 90.

93. A composition comprising the DNA-based nanostructure of any one of paragraphs 78-87.

94. A method of encapsulating a virus, a viral particle, or a subviral particle, comprising contacting the virus, viral particle, or subviral particle with the DNA-based nanostructure of any one of paragraphs 1-53, the self-assembling DNA-based building block of any one of paragraphs 54-77, the DNA-based nanostructure of cany one of paragraphs 78-87, the composition of any one of paragraphs 89, 90, 92, and 93, or the plurality of self-assembling DNA based building blocks of paragraph 91.

95. A composition comprising a virus, a viral particle, or a subviral particle and the DNA-based nanostructure of any one of paragraphs 1-53, the self-assembling DNA-based building block of any one of paragraphs 54-77, the DNA-based nanostructure of any one of paragraphs 78-87, or plurality of self-assembling DNA based building blocks of paragraph 91.

96. The composition according to paragraph 95, wherein the virus, viral particle, or subviral particle is bound or at least partially encapsulated by the DNA-based nanostructure, self-assembling DNA-based building block, or plurality of self-assembling DNA-based building blocks.

97. The method of paragraph 94 or composition of paragraphs 95-96, wherein the virus, viral particle, or subviral particle is influenza virus according to any one of paragraphs 22-24.

98. The DNA-based nanostructure of any of paragraphs 1-53, self-assembling DNA-based building block of any of paragraphs 54-77, or the DNA-based nanostructure of any one of paragraphs 78-87 for use in medicine.

99. The DNA-based nanostructure of any of paragraphs 1-53, self-assembling DNA-based building block of any of paragraphs 54-77, or the DNA-based nanostructure of any one of paragraphs 78-87 for use in treating Influenza infection.

100. The DNA-based nanostructure of any of paragraphs 1-53, self-assembling DNA-based building block of any of paragraphs 54-77, or DNA-based nanostructure of any one of paragraphs 78-87 for use in prophylaxis against influenza infection.

101. The DNA-based nanostructure or self-assembling DNA-based building block for use of any of paragraphs 98-100, wherein the DNA-based nanostructure is administered to a subject in need thereof.

102. A method of treating a disease in a subject comprising administering the DNA-based nanostructure of any of paragraphs 1-53, self-assembling DNA-based building block of any of paragraphs 54-77, or DNA-based nanostructure of any one of paragraphs 78-87 to the subject.

103. A method of treating influenza infection in a subject comprising administering the DNA-based nanostructure of any of paragraphs 1-53, self-assembling DNA-based building block of any of paragraphs 54-77, or DNA-based nanostructure of any one of paragraphs 78-87 to the subject.

104. A method of preventing Influenza infection comprising administering the DNA-based nanostructure of any of paragraphs 1-53, self-assembling DNA-based building block of any of paragraphs 54-77, or the DNA-based nanostructure of any one of paragraphs 78-87 to a subject.

105. The DNA-based nanostructure of any of paragraphs 1-53, self-assembling DNA-based building block of any of paragraphs 54-77, or DNA-based nanostructure of any one of paragraphs 78-87 for use in the manufacture of a medicament.

106. The DNA-based nanostructure of any of paragraphs 1-53, the self-assembling DNA-based building block of any of paragraphs 54-77, or the DNA-based nanostructure of any one of paragraphs 78-87 for use in the manufacture of a medicament for treating influenza infection.

107. A composition comprising the DNA-based nanostructure of any of paragraphs 1-53, the self-assembling DNA-based building block of any of paragraphs 54-77, or the DNA-based nanostructure of any one of paragraphs 78-87.

108. The composition of any one of paragraphs 89, 90, 92, 95, and 107, wherein the composition further comprises a pharmaceutically acceptable excipient, buffer, or carrier.

109. The composition of paragraphs 89, 90, 92, 95, and 107-108, wherein the composition further comprises a neuraminidase inhibitor, optionally wherein the neuraminidase inhibitor is selected from the group comprising or consisting of: oseltamivir, zanamivir, ianamivir, and peramivir; optionally wherein the neuraminidase inhibitor is oseltamivir.

110. The composition of any one of paragraphs 89, 90, 92, 95, and 107-109 for use in medicine.

111. The composition of any one of paragraphs 89, 90, 92, 95, and 107-109 for use in treating Influenza infection.

112. The composition of any of paragraphs 89, 90, 92, 95, and 107-109for use in prophylaxis against Influenza infection.

113. The composition for use of any of paragraphs 110-112, wherein the composition is administered to a subject in need thereof.

114. A method of treating a disease comprising administering the composition of any of paragraphs 89, 90, 92, 95, and 107-109 to the subject.

115. A method of treating influenza infection in a subject, comprising administering the composition of any of paragraphs 89, 90, 92, 95, and 107-109 to the subject.

116. A method of preventing influenza infection in a subject, comprising administering the composition of any of paragraphs 89, 90, 92, 95, and 107-109 to the subject.

117. The composition of any of any of paragraphs 89, 90, 92, 95, and 107-109 for use in the manufacture of a medicament.

118. The composition of any of paragraphs 89, 90, 92, 95, and 107-109 for use in the manufacture of a medicament for treating influenza infection.

119. The DNA-based nanostructure or self-assembling DNA-based building block for use according to any of paragraphs 98-101, method of any of paragraphs 102-104 and 114-116, or composition for use according to any of 110-113 and 117-11888, wherein the influenza infection is caused by an influenza virus selected from: Influenza A, Influenza B, Influenza C, and Influenza D; optionally
wherein the influenza virus is Influenza A or Influenza B.

120. The DNA-based nanostructure for use, method, or composition for use of paragraph 119, wherein:

a) the influenza virus has a haemagglutinin subtype selected from the group comprising or consisting of: H1, H2, H3, H4, H5, H6, H7, H8, H9, H10, H11, H12, H13, H14, H15, H16, H17, and H18; and/or
b) the influenza virus has a neuraminidase subtype selected from: N1, N2, N3, N4, N5, N6, N7, N8, N9, N10, and N11.

121. The DNA-based nanostructnure for use, method, or composition for use of paragraphs 119-120, wherein the influenza virus has a subtype selected from the group comprising or consisting of: H1N1, H2N2, H3N2, H5N1, H5N5, H5N6, H5N8, H6N1, H7N2, H7N7, H7N9, and H10N7; optionally
Wherein the influenza has a subtype that is H3N2, H5N1, H7N9, or H1N1.

122. The DNA-based nanostructure or self-assembling DNA-based building block for use according to any of paragraphs 98-101 and 119-121, method of any of paragraphs 102-104, 114-116, and 119-121, or composition for use according to any of 110-113 and 117-121, wherein the DNA-based nanostructure of composition is co-administered with a neuraminidase inhibitor.

123. The DNA-based nanostructure for use, method, or composition for use of paragraph 122, wherein the neuraminidase inhibitor is selected from the group comprising or consisting of: oseltamivir, zanamivir, ianinamivir, and peramivir; optionally wherein the neuraminidase inhibitor is oseltamivir.

124. A kit comprising:

a) the DNA-based nanostructure of any of paragraphs 1-53;
b) the self-assembling DNA-based subunit of any of paragraphs 54-77;
c) the DNA-based nanostructure of any of paragraphs 78-87; and/or
d) the composition of any of paragraphs 89, 90, 92, 95, and 107-113 or 117-123.

125. A kit comprising one or more components as described substantially herein.

126. A neuraminic acid or derivative thereof is resistant to cleavage or degradation by a neuraminidase; or is a non-cleavable neuraminic acid or non-cleavable neuraminic acid derivative.

127. A method of producing a DNA-based nanostructure according to any one of paragraphs 1-53, or 78-87, the method comprising the steps of:

a) providing at least one single-stranded DNA template strand and a set of oligonucleotides that are at least partially complementary to said single-stranded DNA template;
b) incubating an admixture of the at least one single-stranded DNA template strand and a set of oligonucleotides that are at least partially complementary to said single-stranded DNA template under conditions that permit annealing of the single-stranded DNA template strand and oligonucleotides of the set of oligonucleotides that are at least partially complementary to said single-stranded DNA template to form a plurality of DNA-based building blocks; and
c) incubating the plurality of DNA-based building blocks under conditions that permit assembly of the DNA-based building blocks into the DNA-based nanostructure.

128. The method of paragraph 127, wherein the method further comprises a) i) admixing the at least one single-stranded DNA template strand and the set of oligonucleotides that are at least partially complementary to said single-stranded DNA template to form the admixture of the at least one single-stranded DNA template strand and a set of oligonucleotides that are at least partially complementary to said single-stranded DNA template.

129. The method of paragraph 127 or 128, wherein in step b) the single-stranded DNA template strand and oligonucleotides of the set of oligonucleotides that are at least partially complementary to said single-stranded DNA template are:

a) incubated in a solution comprising $Mg^{2+}$ ions; and/or
b) subjected to a thermal annealing ramp; optionally
wherein the single-stranded DNA template strand and oligonucleotides of the set of oligonucleotides that are at least partially complementary to said single-stranded DNA template:

i) heated to a temperature of about 60°C, about 65°C, about 70°C, about 80°C, about 85°C, about 90°C, about 95°C, or about 98°C; and
ii) cooled to 45°C or about 45°C.

130. The method of any one of paragraphs 127-129, wherein the method further comprises b) i) purifying the DNA-based building blocks.

131. The method of any one of paragraphs 127-130, wherein the method further comprises c) i) purifying the DNA-based nanostructure.

132. The method of any one of paragraphs 127-132, wherein the method further comprises d) stabilising the DNA-based nanostructures, optionally
wherein the DNA-based nanostructures are stabilised by UV-welding, chemical crosslinking, or coating.

133. The method of any one of paragraphs 127-132, wherein the method further comprises e) conjugating functional moieties to the DNA-based nanostructure, optionally
wherein the functional moieties are selected from the group comprising: virus-binding moieties, target-binding moieties, cell-binding moieties, and recruitment moieties.

134. The method of paragraph 133, wherein the method further comprises a final purification step.

135. A method of producing a self-assembling DNA-based subunit of any one of paragraphs 54-77, comprising the steps of:

a) providing at least one single-stranded DNA template strand and a set of oligonucleotides that are at least partially complementary to said single-stranded DNA template; and
b) incubating an admixture of the at least one single-stranded DNA template strand and a set of oligonucleotides that are at least partially complementary to said single-stranded DNA template under conditions that permit

annealing of the single-stranded DNA template strand and oligonucleotides of the set of oligonucleotides that are at least partially complementary to said single-stranded DNA template to form a plurality of DNA-based building blocks.

136. The method of paragraph 135, wherein the method further comprises a) i) admixing the at least one single-stranded DNA template strand and the set of oligonucleotides that are at least partially complementary to said single-stranded DNA template to form the admixture of the at least one single-stranded DNA template strand and a set of oligonucleotides that are at least partially complementary to said single-stranded DNA template.

137. The method of paragraph 135 or 136, wherein in step b) the single-stranded DNA template strand and oligonucleotides of the set of oligonucleotides that are at least partially complementary to said single-stranded DNA template are:

a) incubated in a solution comprising $Mg^{2+}$ ions; and/or
b) subjected to a thermal annealing ramp; optionally
wherein the single-stranded DNA template strand and oligonucleotides of the set of oligonucleotides that are at least partially complementary to said single-stranded DNA template:

i) heated to a temperature of about 60°C, about 65°C, about 70°C, about 80°C, about 85°C, about 90°C, about 95°C, or about 98°C; and
ii) cooled to 45°C or about 45°C.

138. The method of any one of paragraphs 135-137, wherein the method further comprises c) purifying the DNA-based building blocks.

139. The method of any one of paragraphs 135-138, wherein the method further comprises d) conjugating functional moieties to the DNA-based nanostructure, optionally
wherein the functional moieties are selected from the group comprising: virus-binding moieties, target-binding moieties, cell-binding moieties, and recruitment moieties.

140. The method of paragraph 139, wherein the method further comprises a final purification step.

**Figure legends**

Figure 1 Schematic representation of the DNA-based nanostructure provide herein, outlining the construction and operational mechanism of the antiviral countermeasures platform. The process begins with a variety of validated virus-binding moieties such as neuraminic acid derivatives, antibodies, peptides, host receptors, aptamers, *de-novo* proteins, and polymers. The virus-binding moieties are grafted onto a DNA chassis, forming a nanostructure that encapsulates and binds to viruses. Once assembled, these nanostructures adhere to the viral surface, effectively blocking the virus from entering host cells and suppressing viral replication. The avidity effect, due to multiple binding sites on the DNA chassis, ensures strong affinity across different virus families and mutations, enhancing the platform's effectiveness against a broad range of viruses.

Figure 2 An exemplary pentameric DNA-based nanostructure, in line with an embodiment of the invention provided herein. The DNA-based nanostructure is shown in perspective view, top view, and side view. For details of the design see **Example 1.**

Figure 3 TEM images of Scaffold 1 + Sialyllactose (S1-SL), Scaffold 2 + Sialyllactose (S2-SL), and Scaffold 1 Control (S1-C; without sialyllactose)nanostructures incubated with Influenza virus New Caledonia viral particles. Both a full field view and crop images are shown. S1-SL and S2-SL nanostructures conjugated to 2,6'-sialyllactose bind to the surface of the viral particles, as indicated by white arrows in crop images. Control S1-C nanostructures comprising no 2,6'-sialyllactose no not bind to the virus surface. For experimental details see **Example 2.**

Fiaure 4 **A** TEM images of S1-SL nanostructures incubated with Influenza A/Panama/2007/99 (H3N2), Influenza A/New Caledonia/20/99 (H1N1), Influenza B/Tokyo/53/99, Influenza B/Malaysia/2506/04, and Influenza A/Kiev/301/94 (H3N2). All tested viruses other than Influenza A/New Caledonia demonstrated reduced binding of

the functionalised nanostructure to the virus. **B** Fluorescence cleavage assay showing that all tested viruses have an active neuraminidase enzyme that is capable of cleaving sialyllactose, with the exception of the Influenza A/New Caledonia lineage. A recombinant neuraminidase from the California lineage (recomb. NA A/California/04/2009, Biozol, Catalogue No. SIN-11058-VNAHC) has neuraminidase activity; however, cleavage is inhibited by treating the recombinant neuraminidase with the neuraminidase inhibitor, oseltamivir carboxylate.

**Figure 5 A** TEM images demonstrating the interaction between the nanostructure functionalized with sialyllactose and various strains of Influenza A virus. The top row displays images of viruses without additional neuraminidase (NA) inhibition. The bottom row shows the same strains with inhibited neuraminidase activity, highlighting the enhanced trapping efficiency of the nanostructure. **B** and **C** TEM images demonstrating the interaction between nanostructure functionalized with sialyllactose and various strains of Influenza A and B virus. The left column displays images of viruses without additional neuraminidase (NA) inhibition. The right column shows the same strains with inhibited neuraminidase activity, highlighting the enhanced trapping efficiency of the nanostructure.

**Figure 6** Structural variation of the virus-binding moiety for broad-spectrum virus capture. **A** Overview of neuraminic acid (N-acetylneuraminic acid, Neu5Ac/NANA), highlighting points of possible modifications and attachment to the DNA nanostructures (arrows). **B** Molecular structures depicting various modifications of neuraminic acid designed to prevent cleavage by neuraminidase. These modifications at different attachment points (arrows) aim to enhance the stability and efficacy of neuraminic acid as an influenza binder, potentially improving resistance against enzymatic degradation.

**Examples**

**Example 1** - **Design and construction of a pentameric DNA-based nanostructure**

Scaffold 1 and Scaffold 2 nanostructures were generated as follows.

All self-assembly experiments were performed in standardized 'folding buffers' containing 20 mM $MgCl_2$ in addition to 5mM Tris base, 1 mM EDTA and 5mM NaCl at pH 8. Single-scaffold-chain DNA origami objects were self-assembled in one-pot folding reactions containing scaffold DNA and staple strands. The individual scaffolds were produced as described previously. DNA sequences are summarized below and in Table 1. All reaction mixtures were subjected to thermal annealing ramps in thermal cycling devices.

All subunits were purified by PEG precipitation. To assemble the purified (and concentrated) subunits into chassis we adjusted the subunit and $MgCl_2$ concentrations by adding $MgCl_2$ in suitable amounts. Typical subunit concentrations were in the range of 5-100 nM. Typical $MgCl_2$ concentrations for chassis self-assembly were in the range of 10-40 mM. Chassis self-assembly was performed at 40°C.

The assembled chassis were UV cross-linked (UV welded) for 1 h at 310 nm using a commercial UV LED (Gerling, T. et al., Sci. Adv. 4 (2018) eaau1157). Buffer exchange to 1× PBS containing 5 mM $MgCl_2$ was performed prior to virus encapsulation experiments using ultrafiltration (Amicon Ultra 500 mL with 100 kDa molecular weight cutoff) or dialysis (D-TubeTM Dialyzer Mini, MWCO 12-14 kDa, 2 ×500 ml exchanges over 8 h, r.t).

The chassis are functionalized with the virus binding molecules by DNA hybridization to the handles and/or chemical conjugation, to form Scaffold 1 + Sialyllactose (S1-SL), Scaffold 2 + Sialyllactose (S2-SL) nanostructures.

**Example 2** - **Influenza virus trapping using the pentameric DNA-based nanostructure**

Scaffold 1 and Scaffold 2 pentameric nanostructures were prepared as set out in Example 1 and functionalised with 2,6'-sialyllactose (SL)-conjugated handle-binding oligonucleotides, to generate Scaffold 1 + Sialyllactose (S1-SL), Scaffold 2 + Sialyllactose (S2-SL).

S1-SL, S2-SL, and negative control non-functionalised S1 shells (S1-C, lacking 2,6'-sialyllactose) nanostructures were suspended in 1 × PBS containing 5 mM $MgCl_2$ and mixed with Influenza A/New Caledonia in 1 × PBS containing 5 mM $MgCl_2$ to a final concentration of 20 nM nanostructure and 0.05 mg/mL virus. Samples were incubated at room temperature for at least 1 h, before being imaged by transmission electron microscopy. Samples were incubated on glow discharged (45 s, 35 mA) formvar carbon-coated Cu400 TEM grids (Electron Microscopy Sciences) for 90 to 120 s . Next, the grids were stained for 30 s with 2 % aqueous uranyl formate containing 25 mM NaOH. Imaging was performed with magnifications in between 10,000× and 42,000× by Transmission Electron Microscopy (TEM).

As shown in **Figure 3**, both S1-SL and S2-SL nanostructures are capable of binding to the surface of Influenza New Caledonia virus particles, whereas negative control S2-C nanostructures cannot bind the virus surface.

This data demonstrates that the neuraminic acid derivative 2,6'-sialyllactose can be used as a virus-binding moiety to direct both the S1 and S2 nanostructures to specifically bind Influenza virus.

**Example 3** - **Neuraminidase inhibitors improve virus trapping**

Following successful trapping of Influenza A/New Caledonia, the ability of the pentameric nanostructure to bind to other Influenza serotypes and lineages was tested.

S1-SL nanostructures were prepared and functionalised as set out in Examples 1 and 2, before being mixed with test viruses (Influenza A/Panama/2007/99 (H3N2), Influenza A/New Caledonia/20/99 (H1N1), Influenza B/Tokyo/53/99, Influenza B/Malaysia/2506/04, and Influenza A/Kiev/301/94 (H3N2)) to a final concentration of 20 nM nanostructure and 0.05 mg/mL virus. Samples were incubated at room temperature for at least 1 h, before being imaged by transmission electron microscopy. Briefly, samples were incubated on glow discharged (45 s, 35 mA) formvar carbon-coated Cu400 TEM grids (Electron Microscopy Sciences) for 90 to 120 s. Next, the grids were stained for 30 s with 2 % aqueous uranyl formate containing 25 mM NaOH. Imaging was performed with magnifications in between 10,000× and 42,000× by Transmission Electron Microscopy (TEM).

As shown in **Figure 4A**, all tested viruses other than Influenza A/New Caledonia demonstrated reduced binding of the functionalised nanostructure to the virus. The inventors hypothesised that this may be due to cleavage of sialyllactose residues from the surface of the nanostructure by viral neuraminidases.

To test this hypothesis, a fluorescence intensity assay with 4-MUNANA was performed. 4-MUNANA is a fluorescent substrate used in neuraminidase activity assays and is a neuraminidase substrate analogue. As shown in **Figure 4B**, all tested viruses have an active neuraminidase enzyme that is capable of cleaving the substrate analogue 4-MUNANA, with the exception of the Influenza A/New Caledonia lineage. A recombinant neuraminidase from the California lineage (recomb. NA A/California/04/2009) has neuraminidase activity and is capable of cleaving the substrate analogue 4-MUNANA; however, cleavage is inhibited by treating the recombinant neuraminidase with the neuraminidase inhibitor, oseltamivir carboxylate.

To test whether neuraminidase inhibitor treatment improved the nanostructure binding to viral subtypes and lineages other than Influenza A/New Caledonia, 0.5 mg/mL Influenza A/New Caledonia/20/99 (H1N1), Influenza A/Puerto Rico/8/34 (H1N1), Influenza A/Kiev/301/94 (H3N2), Influenza A/Panama/2007/99 (H3N2), Influenza A/Wisconsin/87/05 (H3N2), Influenza A/Shandong/9/93 (H3N2), Influenza A/Brisbane/10/07 (H3N2), Influenza B/Tokyo/53/99, Influenza B/Malaysia/2506/04, Influenza B/Victoria/504/0, Influenza B/Florida/04/06, and Influenza B/Florida/07/04 were treated for 1 hour with the neuraminic acid inhibitor oseltamivir carboxylate at a concentration of 25mg/mL to inactivate the neuraminidase. Inactivated and untreated virus was then incubated with S1-SL nanostructures prepared as set out in Examples 1 and 2, for 1 hour, before imaging by TEM. Briefly, samples were incubated on glow discharged (45 s, 35 mA) formvar carbon-coated Cu400 TEM grids (Electron Microscopy Sciences) for 90 to 120 s. Next, the grids were stained for 30 s with 2 % aqueous uranyl formate containing 25 mM NaOH. Imaging was performed with magnifications in between 10,000× and 42,000× by Transmission Electron Microscopy (TEM).

As shown in Figure 5, the nanostructure bound to different untreated influenza viruses to varying degrees. The binding of the nanostructure to all viruses that had been inactivated was significantly improved. This experiment therefore demonstrates that nanostructure binding to viruses with active neuraminidase may be improved by treating the virus with a neuraminidase inhibitor either prior to or during treatment with the nanostructure.

**Prophetic Example 4** - **Virus capture by a nanostructure comprising non-cleavable virus-binding moieties**

Following the successful trapping of Influenza A and B viruses with inactivated neuraminidase activity, the ability of the pentameric nanostructure functionalized with non-cleavable virus-binding moieties to bind to various Influenza serotypes and lineages will be tested.

S1 nanostructures functionalised with non-cleavable virus-binding moieties (S1-SLnc) will be prepared and functionalized as set out in Examples 1 and 2. The nanostructures will then be modified to incorporate non-cleavable sialyllactose analogues, designed to resist cleavage by viral neuraminidase enzymes. The non-cleavable nanostructures will then be mixed with test viruses (Influenza A/Panama/2007/99 (H3N2), Influenza A/New Caledonia/20/99 (H1N1), Influenza B/Tokyo/53/99, Influenza B/Malaysia/2506/04, and Influenza A/Kiev/301/94 (H3N2)) to a final concentration of 20 nM nanostructure and 0.05 mg/mL virus. Samples will be incubated at room temperature for at least 1 h before being imaged by transmission electron microscopy. Briefly, samples will be incubated on glow-discharged (45 s, 35 mA) formvar carbon-coated Cu400 TEM grids (Electron Microscopy Sciences) for 90 to 120 s. Next, the grids were stained for 30 s with 2 % aqueous uranyl formate containing 25 mM NaOH. Imaging will be performed with magnifications between 10,000× and 42,000× by Transmission Electron Microscopy (TEM).

It is anticipated that all tested viruses will demonstrate strong binding of the S1-SLnc functionalized nanostructure to the virus, indicating that the non-cleavable sialyllactose analogues effectively bind to influenza viruses and prevent neuraminidase-mediated cleavage.

**Prophetic Example 5 - Virus capture by a diverse range of nanostructure designs**

Following the demonstration of effective virus trapping with a pentameric nanostructure functionalized with non-cleavable virus-binding moieties, the ability of various nanostructure designs to bind Influenza viruses will be tested.

A variety of nanostructures including tetrameric, hexameric, heptameric, octameric, and dodecameric configurations will be prepared and functionalized as described in Examples 1 and 2. These nanostructures will then modified to incorporate a range of virus-binding moieties including non-cleavable sialyllactose analogues, non-cleavable glycans, and synthetic peptide ligands, each designed to resist cleavage by viral neuraminidase enzymes. The functionalized nanostructures will then be mixed with test viruses (Influenza A/Panama/2007/99 (H3N2), Influenza A/New Caledonia/20/99 (H1N1), Influenza B/Tokyo/53/99, Influenza B/Malaysia/2506/04, and Influenza A/Kiev/301/94 (H3N2)) to a final concentration of 20 nM nanostructure and 0.05 mg/mL virus. Samples will be incubated at room temperature for at least 1 h before being imaged by transmission electron microscopy. Briefly, samples will be incubated on glow-discharged (45 s, 35 mA) formvar carbon-coated Cu400 TEM grids (Electron Microscopy Sciences) for 90 to 120 s. Next, the grids will be stained for 30 s with 2 % aqueous uranyl formate containing 25 mM NaOH. Imaging was performed with magnifications between 10,000× and 42,000× by Transmission Electron Microscopy (TEM).

It is expected that all tested viruses will demonstrate strong binding of the functionalized nanostructures, regardless of their configuration and binding moiety, indicating that these diverse nanostructure designs effectively bind to influenza viruses and prevent neuraminidase-mediated cleavage.

## Equivalents

The foregoing embodiments, instances, and examples are applicable to any of the aspects of the present disclosure and should be construed as such.

While the present disclosure has been described in terms of various aspects, embodiments, and examples, it is understood that variations, improvements, and equivalents will occur to the person skilled in the art. Such variations, improvements, and equivalents are contemplated by the present disclosure and fall within the scope of the matter disclosed and claimed herein.

## References

All references to other documents made in the present application, to the extent that they provide exemplary procedural or other details supplementary to those set forth herein, are specifically herein incorporated by reference.

## Sequences of the disclosure

>SEQ ID NO: 1 Scaffold 1

cgagtctctaccacagcatatgtgaccccgggcgggccctcagatgccatctggtgctactgtttcatggagaggagggtctccaggtga
cgtgccgtggcgccgatgagtcaaaaggcacaagcagaaatcatatgactatggccgcctgaatcccccgtcacgaaccggtagcatcaa
cccagttgacagcttctgaccttccaacacttaacaggccagcgcagtaggcggcgtcttcacagtccctaagagccaaacctctagtgg
ggagctgcaccagagttcctcggatcgctgagcgccctgtagcggcgcattaagcgcggcgggtgtggtggttacgcgcagcgtgaccgc
tacacttgccagcgccctagcgcccgctcctttcgctttcttcccttcctttctcgccacgttcgccggctttccccgtcaagctctaaa
tcgggggctccctttagggttccgatttagtgctttacggcacctcgaccccaaaaaacttgattagggtgatggttcacgtagtgggcc
atcgccctgatagacggtttttcgccctttgacgttggagtccacgttctttaatagtggactcttgttccaaactggaacaacactcaa
ccctatctcggtccaagtctattagcctagctccttgccccttttgtgaaacaaattttatcgaaagaacttcgagcaattcccacatg
ctctcgtactttgggttacacacgcttggtcatcgttcgcccatacagacatttgcagcccgcatgtcctgggaacatccgctgtcggca
aagccggctgagcgattcaatacccgactcccggggcataacgggctaatcggacgcacgtcgcgctaggtttgaggtcgatctttccg
agaagctatatagtacggcctccggaccaagatacgcggtggactgataatgatgggcgaccctgccttatgcaacgacaccgcgaggct
cgcaaggaatatttcttgatccattcggttctacatagagatttactcgacggtccacctacctgtacaatctaactacgtttagacttg
taagcgtgagacgatatcactggcttagttaattgaacgccactagatcagtgattgtcagcactcaccccaatgcttttcggggaaatg
tgcgcggaaccttgatcgggcacgtaagaggttccaactttcaccataatgaaataagatcactaccgggcgtatttttgagttatcga
gattttcaggagctaaggaagctaaaatggagaaaaaaatcactggatataccaccgttgatatatcccaatggcatcgtaaagaacatt
ttgaggcatttcagtcagttgctcaatgtacctataaccagaccgttcagctggatattacggcctttttaaagaccgtaaagaaaaata
agcacaagttttatccggcctttattcacattcttgcccgcctgatgaatgctcatccggaatttcgtatggcaatgaaagacggtgagc
tggtgatatgggatagtgttcacccttgttacaccgttttccatgagcaaactgaaacgttttcatcgctctggagtgaataccacgacg
atttccggcagtttctacacatatattcgcaagatgtggcgtgttacggtgaaaacctggcctatttccctaaagggtttattgagaata
tgtttttcgtctcagccaatccctgggtgagtttcaccagttttgatttaaacgtggccaatatggacaacttcttcgcccccgttttca
ccatgggcaaatattatacgcaaggcgacaaggtgctgatgccgctggcgattcaggttcatcatgccgtttgtgatggcttccatgtcg
gcagaatgcttaatgaattacaacagtactgcgatgagtggcagggcggggcgtaatttgatatcgagctcgcttggactcctgttgata

gatccagtaatgacctcagaactccatctggatttgttcagaacgctcggttgccgccgggcgtttttattggtgagaatccaagcctc
gagctgtcagaccaagtttactcatatatactttagattgatttaaaacttcatttttaatttaaaaggatctaggtgaagatcctttttt
gataatctcatgaccaaaatcccttaacgtgagttttcgttccactgagcgtcagaccccgtagaaaagatcaaaggatcttcttgagat
cctttttttctgcgcgtaatctgctgcttgcaaacaaaaaaaccaccgctaccagcggtggtttgtttgccggatcaagagctaccaact
ctttttccgaaggtaactggcttcagcagagcgcagataccaaatactgttcttctagtgtagccgtagttaggccaccacttcaagaac
tctgtagcaccgcctacatacctcgctctgctaatcctgttaccagtggctgctgccagtggcgataagtcgtgtcttaccgggttggac
tcaagacgatagttaccggataaggcgcagcggtcgggctgaacggggggttcgtgcacacagcccagcttggagcgaacgacctacacc
gaactgagatacctacagcgtgagctatgagaaagcgccacgcttcccgaagggagaaaggcggacaggtatccggtaagcggcagggtc
ggaacaggagagcgcacgagggagcttccagggggaaacgcctggtatctttatagtcctgtcgggtttcgccacctctgacttgagcgt
cgatttttgtgatgctcgtcaggggggcggagcctatggaaaaacgccagcaacgcggcctttttacggttcctggccttttgctggcct
tttgctcacgaaattcacctcgaaagcaagctgataaaccgatacaattaaaggctccttttggagcctttttttttggagattttcaac
gtgaaaaaattattattcgcaattcctttagttgttcctttctattctcactccgctgaaactgttgaaagttgtttagcaaaaccccat
acagaaaattcatttactaacgtctggaaagacgacaaaactttagatcgttacgctaactatgagggctgtctgtggaatgctacaggc
gttgtagtttgtactggtgacgaaactcagtgttacggtacatgggttcctattgggcttgctatccctgaaaatgagggtggtggctct
gagggtggcggttctgagggtggcggttctgagggtggcggtactaaacctcctgagtacggtgatacacctattccgggctatacttat
atcaaccctctcgacggcacttatccgcctggtactgagcaaaaccccgctaatcctaatccttctcttgaggagtctcagcctcttaat
actttcatgtttcagaataataggttccgaaataggcagggggcattaactgtttatacgggcactgttactcaaggcactgaccccgtt
aaaacttattaccagtacactcctgtatcatcaaaagccatgtatgacgcttactggaacggtaaattcagagactgcgctttccattct
ggctttaatgaggatccattcgtttgtgaatatcaaggccaatcgtctgacctgcctcaacctcctgtcaatgctggcggcggctctggt
ggtggttctggtggcggctctgagggtggtggctctgagggtggcggttctgagggtggcggctctgagggaggcggttccggtggtggc
tctggttccggtgattttgattatgaaaagatggcaaacgctaataagggggctatgaccgaaaatgccgatgaaaacgcgctacagtct
gacgctaaaggcaaacttgattctgtcgctactgattacggtgctgctatcgatggtttcattggtgacgtttccggccttgctaatggt
aatggtgctactggtgattttgctggctctaattcccaaatggctcaagtcggtgacggtgataattcacctttaatgaataatttccgt
caatatttaccttccctccctcaatcggttgaatgtcgcccttttgtctttggcgctggtaaaccatatgaattttctattgattgtgac
aaaataaacttattccgtggtgtctttgcgtttcttttatatgttgccacctttatgtatgtattttctacgtttgctaacatactgcgt
aataaggagtcttaatcatgccagttcttttgggttaagtaactaaggagctttaactcctactttcatacgccaccgcacagacttcat
tgaccacgtatctgagactccttgtaaactgactactccagctccatcaacaatcttgaagaagttctatccgatcacggttatcgcgag
tcttagcgtagccaatgctgggccgtagcgtctacttaacggaaagaagggtgaaagctgccacagaaaataaattactatttacatttg
taagacaaagttagtatggttaaatactggcttagtctgcgggaaacttagccatacgacttggtttgcctatcgttacagcgtacgacc
cgttcaccaatcgcatttgcgatgcagataacaactcggacgaagtaaccacggagtatcctatctcttacgagtgttattctcattata
aagtgcattggcagggggtgtgcgggtatactaaacgtaataagggattaccatgaaggtaaatgttcaggacaactctgtgtattgcgt
taggatagcggaaaaactgcgcgtatgtggtaccagcacagaggatcgtcgcaagtgtcaagcctggggcacgactcttcgctctgctgt
caagatatttgcaccggaaatgcagcaccttaaaggataaacatagtagctgattctctgaagtcccagaagcagtaaagatcgaagttt
gcagaatgccataccgtcccagataaacgtattggtgattcggtcattaaggaaatccagcatttgttcccgcaaccatctctgtacgag
tattgttcttctttacgatccgaagaaggcctcgtcacgatgagatatcatagcttagattcacgtcattttacaattattcgcaagaac
atgtgggccttctcccgccctcttccggaaatgacaacgtcggccagcattctatggatatccgtcacactaccgtcacctcttcgccgt
tagatggcactgctattcccgatttacaagcttcacgggcgcaatatggccctgagttgttttgggcgagtacgcgagcgtaaaatatat
cgatcgcagatctctacgatcgggtcgtgtgaaccgcaccctataatacgttccaacttccaatagcacttcagactttccggcccacga
agaccttaatagcgtcgcatgcggcatgcaagtcacgctaaccgcacgattgctcatatccaatgcgcgtgcgacacgggattattgtta
gaaactctaagtgagcctctatcctccccttctgcacttatcgtgatttagttttgtggacgatctcgaccacgcagcctttaccgatgg
cgcagaaacactgacgctaatctcaggttccaaggcggaactagaaccagtaaaagcatgtagcccccaggatataaacgcgaaaccctg
cggtgcttgcgcacgttctacgtgaccggtcatcaaggagccctggcggctatgtgttggccgggtcctaagatagggctggtctaggaa
gccgttctgttcacgccctatgtgagtcgttactgcaggattgcgcgcgcggctcgttgtcgcagatccactctacacccgaagttgcat
tagccggcgacatatcagcgctcagacagtgtgaacttcagccagatggtactccatgataataatggtacgcgttgagccgagacgtac
atccattggcaagctagctcaggggctgggtttttctgttaagtttgatgagacagtgtgcatacgagatacccagtcccgtataccgaa
ccgtgaagggactccctgtcctgaatcatcgcgaattccaggttatcggttaccagtctggcaggcatcccactc

>SEQ ID NO: 2 Scaffold 2

tgatagacggtttttcgccctttgacgttggagtccacgttctttaatagtggactcttgttccaaactggaacaacactcaaccctatc
tcgggctattcttttgatttataagggattttgccgatttcggaaccaccatcaaacaggattttcgcctgctggggcaaaccagcgtgg
accgcttgctgcaactctctcagggccaggcggtgaagggcaatcagctgttgcccgtctcactggtgaaaagaaaaaccaccctggcgc

ccaatacgcaaaccgcctctccccgcgcgttggccgattcattaatgcagctggcacgacaggtttcccgactggaaagcgggcagtgag
cgcaacgcaattaatgtgagttagctcactcattaggcaccccaggctttacactttatgcttccggctcgtatgttgtgtggaattgtg
agcggataacaatttcacacaggaaacagctatgaccatgattacgaattcgagctcggtacccggggatcctcaactgtgaggaggctc
acggacgcgaagaacaggcacgcgtgctggcagaaacccccggtatgaccgtgaaaacggcccgccgcattctggccgcagcaccacaga
gtgcacaggcgcgcagtgacactgcgctggatcgtctgatgcagggggcaccggcaccgctggctgcaggtaacccggcatctgatgccg
ttaacgatttgctgaacacaccagtgtaagggatgtttatgacgagcaaagaaacctttacccattaccagccgcagggcaacagtgacc
cggctcataccgcaaccgcgcccggcggattgagtgcgaaagcgcctgcaatgaccccgctgatgctggacacctccagccgtaagctgg
ttgcgtgggatggcaccaccgacggtgctgccgttggcattcttgcggttgctgctgaccagaccagcaccacgctgacgttctacaagt
ccggcacgttccgttatgaggatgtgctctcgccggaggctgccagcgacgagacgaaaaaacggaccgcgtttgccggaacggcaatca
gcatcgtttaactttacccttcatcactaaaggccgcctgtgcggctttttttacgggatttttttatgtcgatgtacacaaccgcccaa
ctgctggcggcaaatgagcagaaattaagtttgatccgctgtttctgcgtctctttttccgtgagagctatcccttcaccacggagaaa
gtctatctctcacaaattccgggactggtaaacatggcgctgtacgtttcgccgattgtttccggtgaggttatccgttcccgtggcggc
tccacctctgaaagcttggcactggccgtcgttttacaacgtcgtgactgggaaaaccctggcgttacccaacttaatcgccttgcagca
catccccctttcgccagctggcgtaatagcgaagaggcccgcaccgatcgcccttcccaacagttgcgcagcctgaatggcgaatggcgc
ttcgcctggtttccggcaccagaagcggtgccggaaagctggctggagtgcgatcttcctgaggccgatactgtcgtcgtcccctcaaac
tggcagatgcacggttacgatgcgcccatctacaccaacgtgacctatcccattacggtcaatccgccgtttgttcccacggagaatccg
acgggttgttactcgctcacatttaatgttgatgaaagctggctacaggaaggccagacgcgaattatttttgatggcgttcctattggt
taaaaaatgagctgatttaacaaaaatttaatgcgaattttaacaaaatattaacgtttacaatttaaatatttgcttatacaatcttcc
tgtttttggggcttttctgattatcaaccggggtacatatgattgacatgctagttttacgattaccgttcatcgattctcttgtttgct
ccagactctcaggcaatgacctgatagcctttgtagatctctcaaaaatagctaccctctccggcattaatttatcagctagaacggttg
aatatcatattgatggtgatttgactgtctccggcctttctcacccttttgaatctttacctacacattactcaggcattgcatttaaaa
tatatgagggttctaaaaatttttatccttgcgttgaaataaaggcttctcccgcaaaagtattacagggtcataatgtttttggtacaa
ccgatttagctttatgctctgaggctttattgcttaattttgctaattctttgccttgcctgtatgatttattggatgttaatgctacta
ctattagtagaattgatgccaccttttcagctcgcgccccaaatgaaaatatagctaaacaggttattgaccatttgcgaaatgtatcta
atggtcaaactaaatctactcgttcgcagaattgggaatcaactgttatatggaatgaaacttccagacaccgtactttagttgcatatt
taaaacatgttgagctacagcattatattcagcaattaagctctaagccatccgcaaaaatgacctcttatcaaaaggagcaattaaagg
tactctctaatcctgacctgttggagtttgcttccggtctggttcgctttgaagctcgaattaaaacgcgatatttgaagtctttcgggc
ttcctcttaatctttttgatgcaatccgctttgcttctgactataatagtcagggtaaagacctgatttttgatttatggtcattctcgt
tttctgaactgtttaaagcatttgagggggattcaatgaatatttatgacgattccgcagtattggacgctatccagtctaaacatttta
ctattaccccctctggcaaaacttcttttgcaaaagcctctcgctattttggtttttatcgtcgtctggtaaacgagggttatgatagtg
ttgctcttactatgcctcgtaattccttttggcgttatgtatctgcattagttgaatgtggtattcctaaatctcaactgatgaatcttt
ctacctgtaataatgttgttccgttagttcgttttattaacgtagatttttcttcccaacgtcctgactggtataatgagccagttctta
aaatcgcataaggtaattcacaatgattaaagttgaaattaaaccatctcaagcccaatttactactcgttctggtgtttctcgtcaggg
caagccttattcactgaatgagcagctttgttacgttgatttgggtaatgaatatccggttcttgtcaagattactcttgatgaaggtca
gccagcctatgcgcctggtctgtacaccgttcatctgtcctctttcaaagttggtcagttcggttcccttatgattgaccgtctgcgcct
cgttccggctaagtaacatggagcaggtcgcggatttcgacacaatttatcaggcgatgatacaaatctccgttgtactttgtttcgcgc
ttggtataatcgctgggggtcaaagatgagtgttttagtgtattcttttgcctctttcgttttaggttggtgccttcgtagtggcattac
gtattttacccgtttaatggaaacttcctcatgaaaaagtctttagtcctcaaagcctctgtagccgttgctaccctcgttccgatgctg
tctttcgctgctgagggtgacgatcccgcaaaagcggcctttaactccctgcaagcctcagcgaccgaatatatcggttatgcgtgggcg
atggttgttgtcattgtcggcgcaactatcggtatcaagctgtttaagaaattcacctcgaaagcaagctgataaaccgatacaattaaa
ggctccttttggagcctttttttggagattttcaacgtgaaaaaattattattcgcaattcctttagttgttcctttctattctcactc
cgctgaaactgttgaaagttgtttagcaaaatcccatacagaaaattcatttactaacgtctggaaagacgacaaaactttagatcgtta
cgctaactatgagggctgtctgtggaatgctacaggcgttgtagtttgtactggtgacgaaactcagtgttacggtacatgggttcctat
tgggcttgctatccctgaaaatgagggtggtggctctgagggtggcggttctgagggtggcggttctgagggtggcggtactaaacctcc
tgagtacggtgatacacctattccgggctatacttatatcaaccctctcgacggcacttatccgcctggtactgagcaaaaccccgctaa
tcctaatccttctcttgaggagtctcagcctcttaatactttcatgtttcagaataataggttccgaaataggcagggggcattaactgt
ttatacgggcactgttactcaaggcactgaccccgttaaaacttattaccagtacactcctgtatcatcaaaagccatgtatgacgctta
ctggaacggtaaattcagagactgcgctttccattctggctttaatgaggatttatttgtttgtgaatatcaaggccaatcgtctgacct
gcctcaacctcctgtcaatgctggcggcggctctggtggtggttctggtggcggctctgagggtggtggctctgagggtggcggttctga
gggtggcggctctgagggaggcggttccggtggtggctctggttccggtgattttgattatgaaaagatggcaaacgctaataagggggc
tatgaccgaaaatgccgatgaaaacgcgctacagtctgacgctaaaggcaaacttgattctgtcgctactgattacggtgctgctatcga

tggtttcattggtgacgtttccggccttgctaatggtaatggtgctactggtgattttgctggctctaattcccaaatggctcaagtcgg
tgacggtgataattcacctttaatgaataatttccgtcaatatttaccttccctccctcaatcggttgaatgtcgcccttttgtctttgg
cgctggtaaaccatatgaattttctattgattgtgacaaaataaacttattccgtggtgtctttgcgtttcttttatatgttgccacctt
tatgtatgtattttctacgtttgctaacatactgcgtaataaggagtcttaatcatgccagttcttttgggtattccgttattattgcgt
ttcctcggtttccttctggtaactttgttcggctatctgcttacttttcttaaaaagggcttcggtaagatagctattgctatttcattg
tttcttgctcttattattgggcttaactcaattcttgtgggttatctctctgatattagcgctcaattaccctctgactttgttcagggt
gttcagttaattctcccgtctaatgcgcttccctgtttttatgttattctctctgtaaaggctgctattttcatttttgacgttaaacaa
aaaatcgtttcttatttggattgggataaataatatggctgtttattttgtaactggcaaattaggctctggaaagacgctcgttagcgt
tggtaagattcaggataaaattgtagctgggtgcaaaatagcaactaatcttgatttaaggcttcaaaacctcccgcaagtcgggaggtt
cgctaaaacgcctcgcgttcttagaataccggataagccttctatatctgatttgcttgctattgggcgcggtaatgattcctacgatga
aaataaaaacggcttgcttgttctcgatgagtgcggtacttggtttaatacccgttcttggaatgataaggaaagacagccgattattga
ttggtttctacatgctcgtaaattaggatgggatattatttttcttgttcaggacttatctattgttgataaacaggcgcgttctgcatt
agctgaacatgttgtttattgtcgtcgtctggacagaattactttaccttttgtcggtactttatattctcttattactggctcgaaaat
gcctctgcctaaattacatgttggcgttgttaaatatggcgattctcaattaagccctactgttgagcgttggctttatactggtaagaa
tttgtataacgcatatgatactaaacaggctttttctagtaattatgattccggtgtttattcttatttaacgccttatttatcacacgg
tcggtatttcaaaccattaaatttaggtcagaagatgaaattaactaaaatatatttgaaaaagttttctcgcgttctttgtcttgcgat
tggatttgcatcagcatttacatatagttatataacccaacctaagccggaggttaaaaaggtagtctctcagacctatgattttgataa
attcactattgactcttctcagcgtcttaatctaagctatcgctatgttttcaaggattctaagggaaaattaattaatagcgacgattt
acagaagcaaggttattcactcacatatattgatttatgtactgtttccattaaaaaaggtaattcaaatgaaattgttaaatgtaatta
attttgttttcttgatgtttgtttcatcatcttcttttgctcaggtaattgaaatgaataattcgcctctgcgcgattttgtaacttggt
attcaaagcaatcaggcgaatccgttattgtttctcccgatgtaaaaggtactgttactgtatattcatctgacgttaaacctgaaaatc
tacgcaatttctttatttctgttttacgtgcaaataattttgatatggtaggttctaacccttccattattcagaagtataatccaaaca
atcaggattatattgatgaattgccatcatctgataatcaggaatatgatgataattccgctccttctggtggtttctttgttccgcaaa
atgataatgttactcaaacttttaaaattaataacgttcgggcaaaggatttaatacgagttgtcgaattgtttgtaaagtctaatactt
ctaaatcctcaaatgtattatctattgacggctctaatctattagttgttagtgctcctaaagatattttagataaccttcctcaattcc
tttcaactgttgatttgccaactgaccagatattgattgagggtttgatatttgaggttcagcaaggtgatgctttagatttttcatttg
ctgctggctctcagcgtggcactgttgcaggcggtgttaatactgaccgcctcacctctgttttatcttctgctggtggttcgttcggta
tttttaatggcgatgttttagggctatcagttcgcgcattaaagactaatagccattcaaaaatattgtctgtgccacgtattcttacgc
tttcaggtcagaagggttctatctctgttggccagaatgtcccttttattactggtcgtgtgactggtgaatctgccaatgtaaataatc
catttcagacgattgagcgtcaaaatgtaggtatttccatgagcgtttttcctgttgcaatggctggcggtaatattgttctggatatta
ccagcaaggccgatagtttgagttcttctactcaggcaagtgatgttattactaatcaaagaagtattgctacaacggttaatttgcgtg
atggacagactcttttactcggtggcctcactgattataaaaacacttctcaggattctggcgtaccgttcctgtctaaaatccctttaa
tcggcctcctgtttagctcccgctctgattctaacgaggaaagcacgttatacgtgctcgtcaaagcaaccatagtacgcgccctgtagc
ggcgcattaagcgcggcgggtgtggtggttacgcgcagcgtgaccgctacacttgccagcgccctagcgcccgctcctttcgctttcttc
ccttcctttctcgccacgttcgccggctttccccgtcaagctctaaatcgggggctccctttagggttccgatttagtgctttacggcac
ctcgaccccaaaaaacttgatttgggtgatggttcacgtagtgggccatcgccc

**Table 1**

| SEQ ID NO | Description | Sequence |
|---|---|---|
| 3 | Scaffold 1 Staple 1 | TCACATATGTTGGAAAACGTTTACAGGAGTTAGCGTCATACATGGCTCCGGATGAT |
| 4 | Scaffold 1 Staple 2 | TAATTGTAAAATGATTTTTGTGAATCTCGTAGAGATCTGTTCCAAAACT |
| 5 | Scaffold 1 Staple 3 | TAGAATGGATTGCCTTGAGTAACAGTGCCCGTATAAATTTTTAGTTAATGT |
| 6 | Scaffold 1 Staple 4 | TTGGGAATTGCTGATATGAGCAATCTTTCTATCAGTTGCAAAATCTTACACAAGTGTT |
| 7 | Scaffold 1 Staple 5 | TGCGGTGGTTCAGAAAAATTTAGGATTTTGGTCAGTTTAAGCGCAGTCTCTGAT |
| 8 | Scaffold 1 Staple 6 | TTCAAACCTTTATGTCTGTATGGGCGTTTCGGCTCAACGCGTTTTTTCCATTATTATCATT |
| 9 | Scaffold 1 Staple 7 | TAACACTGATTTAAGATTTTTGCTGAGACTTGATAGCAATTAAAAAAAAGAATAGGTT |
| 10 | Scaffold 1 Staple 8 | TAAGTTTCTGTTCCGCCGTTTTTGCTTATTCCCACTATTGGACTCCTTAGTTCTTTTTATAGG GTTTTCTAGCTTGT |
| 11 | Scaffold 1 Staple 9 | TAAAATCATTATTGGCCATTTCAAGATTTTGTTAGGAGTTAAAGCTCCTTAGTTT |
| 12 | Scaffold 1 Staple 10 | TCGGGGGATTTTGGGTTGATTCCCGGGGTTTTGCCTGCCTTGAAAACGTT |
| 13 | Scaffold 1 Staple 11 | TTCGTGGTATTTCGCCCCCCGTTCAGCCCGACCGCTTCACCCTCATTGCTAGGCGGT |
| 14 | Scaffold 1 Staple 12 | TCTGACAGCTTTTTTGAGGCTTGGTTACATATTTTTTCTCAATAAT |
| 15 | Scaffold 1 Staple 13 | TAAACACTCCTTTTTTTTTACGCAGTATTTCTTTAGCGTTTTTCAGACTT |
| 16 | Scaffold 1 Staple 14 | TCTAAAGTTTGTTGGTATTTTTGGTATTACACGACTTTTGCTACAGTTACATAGGGT |
| 17 | Scaffold 1 Staple 15 | TTGCCCCGGGAGTCGGTTAGTGCTGTTTATCAGTTTCACTATCTTGCATCTGAT |

(continued)

| SEQ ID NO | Description | Sequence |
|---|---|---|
| 18 | Scaffold 1 Staple 16 | TGGCTTTATCAGCTTTTTTCTATGTTTGCGCCCGTTTTTTAAGCTTGTAAATCGGT |
| 19 | Scaffold 1 Staple 17 | TTCCAACCCTTATCCGGTATTGTAGGTCGTTCGCTCCTTAACGACTCTTTTTCACCGT |
| 20 | Scaffold 1 Staple 18 | TGGCCGCGTTTCTTCGGGAAGCGTGGCGCTTTTTTTCTCTTAAAAGGCT |
| 21 | Scaffold 1 Staple 19 | TTGACGGATTTCGGAAGAGGGCGGGAGTTCCCTGCCATTAGTAGTCATTGCGTATGT |
| 22 | Scaffold 1 Staple 20 | TACATTTACCTTTTTTCATGGTAATCCCTTATTTTATGCACTTTATAATGT |
| 23 | Scaffold 1 Staple 21 | TTTAGGACCCGGCCAACACATAGCCGCCAGGTTTGTCCGCTTCGCAGGGT |
| 24 | Scaffold 1 Staple 22 | TGAATAACACTCGTATTTCTGCGCCATCGGTTTTTAAAGGCTGCGTGGTCGAGATCGTCT |
| 25 | Scaffold 1 Staple 23 | TAAAGGATCTTCACCTTTTTTGATCCTTTTAAATTAAAAAAGCTTGTTCCTTGCT |
| 26 | Scaffold 1 Staple 24 | TCCAGAACCTTACCCTCAGTTATTTGCCCATGGCAAACCATTTTTCGCTGGTAT |
| 27 | Scaffold 1 Staple 25 | TATGATTTTCGGCCCATTTTCTTTCTTCGCTGTAATTTGTCTTACTTAACCTGAGT |
| 28 | Scaffold 1 Staple 26 | TCCATATCACCAGCTTATTAAGTTGGATTTGATCTAGTGGCGTTCTTCCCGATTAGCCCGTTA TTGTTCCTTGGAGTT |
| 29 | Scaffold 1 Staple 27 | TAAAGCATTGGGGTGTTGTATTGAATTTTCACTCCTTGGTGTAGATTGATAGAGTCCACTATT T |
| 30 | Scaffold 1 Staple 28 | TTACATACAGATTCCAATGAAACCATCCGTTGTGCGGTTTTAGTCTGAAGTGCGAATT |
| 31 | Scaffold 1 Staple 29 | TTGAGCAAAAGGCCAGCTTATAGCTCACGCTGTAGTTTTCCTAGACCAGCCCTATT |
| 32 | Scaffold 1 Staple 30 | TCAGGACATGCGGGCTTTTTTCAATTAGCGCGTTTTTCGTGCGTTTAATTAACTAT |

(continued)

| SEQ ID NO | Description | Sequence |
|---|---|---|
| 33 | Scaffold 1 Staple 31 | TAAGAACGTTTCGTGAACCATTTTTCACCTTGTATCTTGTTCCCATCATTTACAATCACT |
| 34 | Scaffold 1 Staple 32 | TATAAGTGTTGTTTGCAATTGAAAAAGATTATATATGAGTAACCCCACTAT |
| 35 | Scaffold 1 Staple 33 | TCCCCCTGTTGAAAGTATTTGTTTCGTCTTGCGAATAATAATTTTTTTCGTAAAAAT |
| 36 | Scaffold 1 Staple 34 | TGTAGCGCGTTACGAAAATTATTCTCATTTCAAAACTTTTTGTCCATT |
| 37 | Scaffold 1 Staple 35 | TGCATTGGCTTTTTTCGCTACCGGAAACGTCATTACTGGCT |
| 38 | Scaffold 1 Staple 36 | TAGGAACTTTCACGTTGTTTTTAAATCTCCATTGCCCAATAGGAACT |
| 39 | Scaffold 1 Staple 37 | TTGTCTGGCGCGCGCAATCCTGCAGTTTAAGCTGGGTTTTGCGAATATATGAAGT |
| 40 | Scaffold 1 Staple 38 | TGAACCTGTTTGAAACATTTCCTATTTCTTTGACAGGAGGTTGAGGCAGGTCAGT |
| 41 | Scaffold 1 Staple 39 | TGCCAGTGATTCGCCCGGTAGTTTTTTATCTTATTTCATTTAGGCCGGATAAAACTT |
| 42 | Scaffold 1 Staple 40 | TGATCAAGGTTAGGGTGAATTAGAGCGATTTAGATTTGGGGTCGAGGTGCCGT |
| 43 | Scaffold 1 Staple 41 | TATTTTAGCTTCCTCACCGTCTTTCATTTCAGGCGGGCAAGAATGTGAATAATTTATGGTGAT TGCTCCTGT |
| 44 | Scaffold 1 Staple 42 | TATTATTCTTAATCGCCATTATTCTGCCGACATGGAAGTTTTTCATCACAAACGATCTCAT |
| 45 | Scaffold 1 Staple 43 | TTTATTTTGTTACGCAAAGTTTGAGCCATTTCACCATTATTGGCCGGAATTAGCGTGACT |
| 46 | Scaffold 1 Staple 44 | TCTCAGCGATTTTTCCGAGGAACTCTTCGGTATACTTTTTGGACTGT |
| 47 | Scaffold 1 Staple 45 | TCTAGGGCGCTGGTATAAAAGAATTTCACAATTTAAAGACAAT |

(continued)

| SEQ ID NO | Description | Sequence |
|---|---|---|
| 48 | Scaffold 1<br>Staple 46 | TGTGCTTATTTTTCTTTACGGTCTTTAAAATTTGTACTCAAAAAATATTTATCGTCT |
| 49 | Scaffold 1<br>Staple 47 | TCCGCCTCCCTTTGTATCGGTTTTTTTTTCAGCTTGCTTTCAGCCACT |
| 50 | Scaffold 1<br>Staple 48 | TGGTCTGGTTATAGGTACATTCTTTACGATGCCATTGTTATGGTTTATTGCCCCCGAT |
| 51 | Scaffold 1<br>Staple 49 | TTGTACTGGTAATAAGTTTTTTCTGCTTTGCAGCAGATTTTGGTCAT |
| 52 | Scaffold 1<br>Staple 50 | TTGACGAGCTTGTGCGCTCTTACGATCTAAAGTTTTGTTTGTATGGGTTGGACGGTAT |
| 53 | Scaffold 1<br>Staple 51 | TATTTACCGTTAGAACCGCTTTTTGCTCAGTACCCATTTCTGTGGTATTTGTGCCTTTTGCCA<br>TAGTT |
| 54 | Scaffold 1<br>Staple 52 | TGAGGTTTCACTTGTAACCGATAACCAGTTTGGAAATCGTTTTCAGTTTTTGAATCCCCGAT |
| 55 | Scaffold 1<br>Staple 53 | TTATATTTTTTCATTCAATTACCGACTTTACACCACGAAGTTTTTCTGTGCGGTGT |
| 56 | Scaffold 1<br>Staple 54 | TAAAATGTTTTTTGAGCAACTGACTGAACGCTCGCGTACTCGCTTCGATCGAT |
| 57 | Scaffold 1<br>Staple 55 | TGAATTATTTCACACGACCCGATAATTTAAGGTGCTTGAGCGAAGAGTCGTGCCCCT |
| 58 | Scaffold 1<br>Staple 56 | TCGATAGGTTGATACTCTTCGCAAATCACAGAGTTTCCTGCTGGTTCAAATCTGTTTCGTGAC<br>GTTTTAAAGGT |
| 59 | Scaffold 1<br>Staple 57 | TTGCGCCTTTTGGTTGTAGAAACTGCCTTTACCTTCGTTGAGACTCGTTCTGTTATCAAT |
| 60 | Scaffold 1<br>Staple 58 | TAGAAGATCCTTTTTTTAACGGTTAGCGGGGTTTCACCCTCATTGGAGGTTTAGTACCGCT |
| 61 | Scaffold 1<br>Staple 59 | TCATGTTCTTACGGCGAAGAGGTGACGGTAGTGTTTGGCATTCTTCGTAAAGAAGAACACCT |
| 62 | Scaffold 1<br>Staple 60 | TTGCTACCGGAAATTTTTGAGCGGGCGTTTTTAGAGTTGGAACCCTTAAATATTCT |

(continued)

| SEQ ID NO | Description | Sequence |
|---|---|---|
| 63 | Scaffold 1 Staple 61 | TGATAGAGGTTATCACGATAAGTGCAGAAACCCAGCCCCTGAGTTATCAAACTT |
| 64 | Scaffold 1 Staple 62 | TACTGTCTCGAGTGTTGTTCCTTTTTGTTTGGAACATCAGGACAGGGT |
| 65 | Scaffold 1 Staple 63 | TCACAAAACTAATTCTCACTTACAGACTAAGCCAGTATTTTTTTAACCATACTAACTTT |
| 66 | Scaffold 1 Staple 64 | TTTGCATGCTTGAGTTTCTTTGTATTCTGTGGCAGTTTTTTTTCACCCT |
| 67 | Scaffold 1 Staple 65 | TGTCATTTCTTATCCATAGAATGCTGGAGCCGCCGTTAACGCCTGT |
| 68 | Scaffold 1 Staple 66 | TCGCGATTTGTGGATCTGCGACATCTTCTGTGTGCACGAACTCAGCCGGCTTTTTCACACT |
| 69 | Scaffold 1 Staple 67 | TGGAACCTACCACCAGTTATTAAGCTTGCGGCATTTCGAAAACTCTTTTTCGTTTTTCTACGG T |
| 70 | Scaffold 1 Staple 68 | TAGCGGTCACGCTGTTTTTGCGTAACCACCAGGCTCTTAGGGATTGAGTGGGAT |
| 71 | Scaffold 1 Staple 69 | TGTATAATTTAACCGCCATTAGAGCCACTTCGCCCCCCTTAGACTTACCGGATACCTTGCTCC TTGT |
| 72 | Scaffold 1 Staple 70 | TTTTAAATTCGAAGAAGTTGGTGAAATTTTATATCCTGGGGCGTTTCTCAAGTCT |
| 73 | Scaffold 1 Staple 71 | TAAATCTCGATAACATTGTGTCGTTGCATTTTTAAGGCAGGGTCGTTGTCCGGAGGT |
| 74 | Scaffold 1 Staple 72 | TTTCGAGGTTTTCCGTTTTTGCAAATGAAAACGGGGGTTTCAATT |
| 75 | Scaffold 1 Staple 73 | TTATCGCCTTGTTCGGTTTACTATCGGAATTTCGTTGTATCACCTTATATAAGTTTAGAAGGA TTTAAGGGCATGAT |
| 76 | Scaffold 1 Staple 74 | TGGAGCTGGTTCTCATCGCTTTCAAATTACGCCCCGTCAGATACGTGGTTTTTCAATGGAATA AGTT |
| 77 | Scaffold 1 Staple 75 | TCATGTACCGTTTACGATTGGCCTTGTTTTTTATTCACAAACGAATGT |

(continued)

| SEQ ID NO | Description | Sequence |
|---|---|---|
| 78 | Scaffold 1 Staple 76 | TACGTGGCGTTGGATATATTTGAGTAAATCTCTATGTAGAACCTATTTAGAGCCATTCCAGCG CCT |
| 79 | Scaffold 1 Staple 77 | TTCACGCTTTTATCGACCTTCGTACTATTTCACAATTTTTAAGGGGCAAGGTGCTTCAGCGGA TT |
| 80 | Scaffold 1 Staple 78 | TGAATAGCAGTTTTCTGGGACTTCAGAGATTTGACACTTGCGACGATTTTGTCCTGAT |
| 81 | Scaffold 1 Staple 79 | TCGTACAGTTGAACTTCTTTAGACGCTATTAAGGCCCGGCGGCAACCGTGAAGTT |
| 82 | Scaffold 1 Staple 80 | TAAAGTCTGAACAAAGTCCAAGAATTCAGCACCTTGTCGGATTTACCCTCAGT |
| 83 | Scaffold 1 Staple 81 | TAAAGCACTAAATCTTCTTGACGGTTGACCGTCTTCAACGGTTTATATCCAGCTGAACT |
| 84 | Scaffold 1 Staple 82 | TGATCCTCATTAAAGCCTTTTTTCAGGTTTCCTCAAGTTATAGCCCGGGCTCT |
| 85 | Scaffold 1 Staple 83 | TGCGGGAATTATTTCCTTTTCGATCTTTACTGCTTTGCCATCTATTTTGCGAATT |
| 86 | Scaffold 1 Staple 84 | TCTTAACCCAAAATAAAGGTGGCATTACCAGTAGTTTTGGGAATTTTTGGAAGTT |
| 87 | Scaffold 1 Staple 85 | TCATATGATGATCTTTTTAAGGGATTTTTTACGCGTTTTTTTTTTTCCGTCGAGT |
| 88 | Scaffold 1 Staple 86 | TGGATCAAGTTTAAAGGGATTGGCGATGGTTATGCGCCGCTACGTAGAAAAT |
| 89 | Scaffold 1 Staple 87 | TCATTTTCGAAATGTAAATAGTTTTTAATTTATTATCAGTAGCGAT |
| 90 | Scaffold 1 Staple 88 | TCAATAGAAAATTCATAGAAAGGAAGGGTTTTTAGAAAGCGTTCGTGAT |
| 91 | Scaffold 1 Staple 89 | TATGACCTTTTACTGGTTCTAGTTCTTAATCGACGTTTTGCCATCTTTTCGTATTCGTTAAGT T |

(continued)

| SEQ ID NO | Description | Sequence |
|---|---|---|
| 92 | Scaffold 1 Staple 90 | TAGAACAGTATTGCTCTTGATTTCTTGAGTTAGGGTTGTTGTACTCATTGAGCCACCACCCTCAT |
| 93 | Scaffold 1 Staple 91 | TCCATTAGCAAGGAGACTCGTTTTTGATAACCGTGATTATTATTCATTAGGCCTTT |
| 94 | Scaffold 1 Staple 92 | TAAGGGCGATTAATGCCTCTTTGGAACGTATTATAGGTTTCTTCGTGT |
| 95 | Scaffold 1 Staple 93 | TGTCTGACGCTCAGTGGCGAGCTCGATATTAGTACTGTTGTAATTCT |
| 96 | Scaffold 1 Staple 94 | TTCTGCGCTTTGGCCAGGTAGTTCTTGAAGTGTTTTTTGGCCTAACTACCCAGGGATT |
| 97 | Scaffold 1 Staple 95 | TGTCACCTGGAGACCCTCCTCTTTTTCCATGAAAAAGCCGGCGAT |
| 98 | Scaffold 1 Staple 96 | TAAAAGGAGCCTTTAACAGTTAAGGAATTTTACCAGTACAAACTACTTCCAGCATT |
| 99 | Scaffold 1 Staple 97 | TGGTAACAGGCACTTCTTTCTCCTTTGCTGGCGTTTTTCCTTACAACTATTAGCCGCCT |
| 100 | Scaffold 1 Staple 98 | TAACTCAGGGCCATATTTTTTATCCTTTTGCTTTTTTTGATATCTCATTTGCTGGTAT |
| 101 | Scaffold 1 Staple 99 | TACCTCTTACGTGCCCTTGCATTCATTTTGCCATACGAAATTTTACGGCACTTGGGCCCGT |
| 102 | Scaffold 1 Staple 100 | TGTGTAACATTTTCCGCGCACATTCCGCCACCCTCTTTTCCAGTAT |
| 103 | Scaffold 1 Staple 101 | TTGAGATGAAGACAACTTTCAGGCGTTTTGACTCATCGGCGCCTTTTTGATGAT |
| 104 | Scaffold 1 Staple 102 | TGTTTACATTTCGGATATTAGATGGTTTTGTGAACGGTTATCTGCATTTCGTGGTTATTGATACT |
| 105 | Scaffold 1 Staple 103 | TCCGACGTTTTAGCCGCCATTTTCAACAGTTTCACTGTTGTTTTGCTAAACAACTTAGCATTCCAT |

(continued)

| SEQ ID NO | Description | Sequence |
|---|---|---|
| 106 | Scaffold 1 Staple 104 | TGGTAAATATTGACGGAAAGGAGTCTTAAGGCCCATTCTTCGGATTTTGCAAACTTTTAATGA CCTTCCAGAT |
| 107 | Scaffold 1 Staple 105 | TCACATACGCGCAGCTTTGACAGCATTTGCATTTCCGGTGCATAGT |
| 108 | Scaffold 1 Staple 106 | TCCGGAACCTTCCCTCAGATTTATGCGGAGTGAGAATTTTTAGCGTATTTCCTGTTCT |
| 109 | Scaffold 1 Staple 107 | TATCCTATTGTCTTTTTTATGGCTAAGTTCCTGCCATTACGTTTAGTATACCCGCGT |
| 110 | Scaffold 1 Staple 108 | TTTTCCCCCTGGAATTACTATAAATTCTTCGTCCTTCGCCTTGGTTGTCATAGCT |
| 111 | Scaffold 1 Staple 109 | TTAAATGAATTTTCTTTCGTCTTTTTGAATCACCTTGAGTTGTTTTGTCATAATCT |
| 112 | Scaffold 1 Staple 110 | TCAGCGTAGTTAGATTTAGGCCGTATTGGTATATCCAGTGATTTTTTTCTCT |
| 113 | Scaffold 1 Staple 111 | TGCCCTCATAGTTAGAAAGGATTATAGGCTCTTCACCGGAATTTTTCGCGTTTCTCACGGCTA CACTAGT |
| 114 | Scaffold 1 Staple 112 | TTGCCGGGTGGCGAAACCCGACAGGTTGCTCCCTCTTATCACAAATTAATACGTTTTGCCAAT ACTT |
| 115 | Scaffold 1 Staple 113 | TCGAACCCAAAGTACGATCGGAAAGTTACAAGTCTTTGCCTCGCGTTGGTAGGTGTTGGACAG TATTTTTCACCAGATGT |
| 116 | Scaffold 1 Staple 114 | TCGTAATATCTTTTATTAAGGTTGTGCGGTTTTCACCGTCTTCCGATTGAGGGAGGGAT |
| 117 | Scaffold 1 Staple 115 | TCAGGCACACCCTTTTTCCCGTTCGCATGCGACGTTTTTCCGCT |
| 118 | Scaffold 1 Staple 116 | GCAGTAGAGTAGGTAGAGATTAGGCATTGGCTGAGTTTAGGCTACATGCGGTCACGTAGAAT |
| 119 | Scaffold 1 Staple 117 | GCAGTAGAGTAGGTAGAGATTAGGCATATTAGCGTCAGTGTTTTAGAGATAGTTCAAACCAAT TACGCAATAGCGATTGT |

(continued)

| SEQ ID NO | Description | Sequence |
|---|---|---|
| 120 | Scaffold 1 Staple 118 | GCAGTAGAGTAGGTAGAGATTAGGCATCGTGCGCAAGATTAGCTTTTTGAGCGAGGTATGTAG GCGGT |
| 121 | Scaffold 1 Staple 119 | GCAGTAGAGTAGGTAGAGATTAGGCATCCCCTTATTTTTCATCGGTTCGTTTAAATTCCAATA AAT |
| 122 | Scaffold 1 Staple 120 | GCAGTAGAGTAGGTAGAGATTAGGCATTAACAGAATTGTATGCACTTAGGGCGAATTAACAGG GCGT |
| 123 | Scaffold 1 Staple 121 | GCAGTAGAGTAGGTAGAGATTAGGCATCAGAATCATTAAGGGGAGTTTCCCGTGTCGCACGCG CATTGCGATTAACGTCAAT |
| 124 | Scaffold 1 Staple 122 | GCAGTAGAGTAGGTAGAGATTAGGCATGGTATCTCAGTTTGCTTGTTAGCATTAAACGATAGC AGCACCTTAACAATAAT |
| 125 | Scaffold 1 Staple 123 | GCAGTAGAGTAGGTAGAGATTAGGCATCCAATGGATGTACGTCTTAACGATGATTATTTGTTT TTATAGCTTCGAGCATGTTCTTGCGATTAAAACAT |
| 126 | Scaffold 1 Staple 124 | GCAGTAGAGTAGGTAGAGATTAGGCATACCCTTTACACGGTTTTTGGTGCATTGCCTGGAATT T |
| 127 | Scaffold 1 Staple 125 | GCAGTAGAGTAGGTAGAGATTAGGCATACCATCTGGCTGAAGTTTTGCCGATTAACTTCGTTC CACCGCTTCTAATCT |
| 128 | Scaffold 1 Staple 126 | GCAGTAGAGTAGGTAGAGATTAGGCATCGTGAACAGAACGGCTTGTATCTCATTACTGGCAGT |
| 129 | Scaffold 1 Staple 127 | GCAGTAGAGTAGGTAGAGATTAGGCATTAACACGCTTGGGAAATATTCTGCTGAATTGCTACT TGGTT |
| 130 | Scaffold 1 Staple 128 | GCAGTAGAGTAGGTAGAGATTAGGCATAGTCCCTTTTCACAACGAGCCAGCGCTGATATGTT |
| 131 | Scaffold 1 Staple 129 | GCAGTAGAGTAGGTAGAGATTAGGCATCGCCGGCTAAAGCTAGGCTTTTTTATAGACTTGGAC CGAGTTCGATAAATTCCAAGCGTGTGTACCCT |
| 132 | Scaffold 2 staple 1 | TAATAAGATGTTTAGTGAATCGCCACGCAATAATAACGGAATACCTTTTT |
| 133 | Scaffold 2 staple 2 | ATACCAGTGAAAGATTACAGACCAGGCGCTTTTTTAGGCTGGCTGA |
| 134 | Scaffold 2 staple 3 | ACAATTTTAGATTTTGAAGTTTTTCTTAAATCCTGATGCAAAT |

(continued)

| SEQ ID NO | Description | Sequence |
|---|---|---|
| 135 | Scaffold 2 staple 4 | CCTTGATATTCAGAGCCGCCACCAGATTTTTCCACCACCAG |
| 136 | Scaffold 2 staple 5 | GTGTAGCGTTTTTTCACGCTGTGATTGCCGTTCCGGGGTGTACAT |
| 137 | Scaffold 2 staple 6 | CAATCGCAAGACAATAGGTTGGAACCTTGCTTCTGTAAATCGTCGC |
| 138 | Scaffold 2 staple 7 | ACAAAAGGAGAAAACTAATAAGGCAGCGCGTTGACAGAATCA |
| 139 | Scaffold 2 staple 8 | TATCAGGGCGGGGAAAAGCACTAAGAGCGGGATTAGACAG |
| 140 | Scaffold 2 staple 9 | CATCGGGAAAATAAAGGTTTAACGAATAGAGATAACCCACTAGACTTT |
| 141 | Scaffold 2 staple 10 | AGGCGGTCAGTATTAACCGAACGATATCTAAATTAGAGCCGTCGGTGG |
| 142 | Scaffold 2 staple 11 | AACGGTGTTACAGAGGGCCTGAGAGTTTTTTTGGAGCAAA |
| 143 | Scaffold 2 staple 12 | CCAGAATGGATAGCAGCACCATTATCATATGGCACGTTGACGATTAA |
| 144 | Scaffold 2 staple 13 | CCGATTTAAATCCTGAAAGAGACGCAGAAACAGCGGATCA |
| 145 | Scaffold 2 staple 14 | ATGTGAGCGAGTGGCGGATTGACCATCAAAAAGATTAAG |
| 146 | Scaffold 2 staple 15 | TAGGTCACGTTGGTGTCTCCGTGGAACAGGTCGATTCACC |
| 147 | Scaffold 2 staple 16 | CTTGTAGAACGTCAGCCGTCTCGTCTTTGCCCGAACGGTAACCACCACACC |
| 148 | Scaffold 2 staple 17 | CAACTTTGTTGACCCCCAGCGATTATACCAAGCGCGAAA |
| 149 | Scaffold 2 staple 18 | TTTGAGAGATATGTACGATTCAAACAAGGATAAAAATTTTGCCTTTAT |

(continued)

| SEQ ID NO | Description | Sequence |
|---|---|---|
| 150 | Scaffold 2 staple 19 | CCAACAGGAAATGGATTATTTATGCGGAACAAGTTTGAGTAACAT |
| 151 | Scaffold 2 staple 20 | AAATCAGAGTAACAGTAGTTACAATCAGAACCGGTTGATA |
| 152 | Scaffold 2 staple 21 | CAAAAGGGGAGCCAGCTGGCGAAAGGGGGATGTGTAGAAAATACATTTTT |
| 153 | Scaffold 2 staple 22 | TTTTTCAAAAGAACTGGCAGCAAACGCTGCAAGGAAATCTCCAAAAAAA |
| 154 | Scaffold 2 staple 23 | AATGGGAACCGTGCATCTGCCAGTTTGAGGATGTTTA |
| 155 | Scaffold 2 staple 24 | AGTCACGACGTTGTAAAACGACGGTTTTTCAGTGCCAAGCTT |
| 156 | Scaffold 2 staple 25 | ATTGAGGGAGGGACGGAAACAATCGGAGTTAAGCAATCCAAATAAGAAAC |
| 157 | Scaffold 2 staple 26 | TTTTTACTGGCTCATTACCAGACGACGTTTTT |
| 158 | Scaffold 2 staple 27 | CGATGGCCTTTGGGGTCGAGGTGTTCCACACTGTGAAAT |
| 159 | Scaffold 2 staple 28 | ACCACCAGAGATAGAACCCTTCTGCATTCTGGCAATTCGACAACATT |
| 160 | Scaffold 2 staple 29 | ACCCTCAAGAGGCTGAGACTCCTAAACATGACAGAGCCAATAGGAAC |
| 161 | Scaffold 2 staple 30 | TTTTGCTTGAATTTACCTTTTTTTCCAGTAAGCGTCATACATGG |
| 162 | Scaffold 2 staple 31 | GAGTGAGAAAGGCCGGAACATTATGACCCAGCGCAGTCTC |
| 163 | Scaffold 2 staple 32 | TCAAATATCAAACCCTCAATTTTTTAATATGTCATACC |
| 164 | Scaffold 2 staple 33 | CCACCCTCAGAACTTTAGCGTCATTTTTACTGT |

(continued)

| SEQ ID NO | Description | Sequence |
|---|---|---|
| 165 | Scaffold 2 staple 34 | TTTTTTAATTTAGGCAGAAAGTACCGTATTAATAGACTACC |
| 166 | Scaffold 2 staple 35 | ATCGCCTGTGTTACTTAGCCGGAGAGAGCCAACGGTGTC |
| 167 | Scaffold 2 staple 36 | TCCGCGACACGGCTGGAGGTGTCCGCAGCACCGTCAATAGTTTACAAA |
| 168 | Scaffold 2 staple 37 | CAACAATAGCTTATCCGGTATTCTCCATATTATTTTGCACCCAGC |
| 169 | Scaffold 2 staple 38 | AATCGGTTTAGCCAGCATAATTCGCGTCTGGAGGCTC |
| 170 | Scaffold 2 staple 39 | TCGTCATAAACATCCCTTACTTTTTCTGGTGTGTTCAGCAAATCGTTAACGGCA |
| 171 | Scaffold 2 staple 40 | TAGCTCTCCAGCCTTTTACCATATCAAACCCCGTACTATGATGATGGTTATCATC |
| 172 | Scaffold 2 staple 41 | AGTCACAAAATTAAGCAATAAAGCTTTTTTCAGAGCATATCCAATAAATC |
| 173 | Scaffold 2 staple 42 | CTTAATTGGCATAACCACAATGACAACAACCAATCAAAAATCACGCCAT |
| 174 | Scaffold 2 staple 43 | TTAGCGTTTTTAACCTCCCTAATTTTTTTTCCAGTTACAAAATAAACAGAAGAACGC |
| 175 | Scaffold 2 staple 44 | AAAATCTACGTTAATAAAACGATGGGCTTGAGATGGTACTCATCT |
| 176 | Scaffold 2 staple 45 | CAGAGCCACCTAAGTATAGCCCGGAAACCTGAGCAGAGGCGA |
| 177 | Scaffold 2 staple 46 | TGACGCTCTTTTTATCGTCTGCCATTGCATTTTTCAGGAAAAAC |
| 178 | Scaffold 2 staple 47 | GAACATTTTTTATTTTTGAGGACAGTTGAACGGGGTCAGCGGTTGC |
| 179 | Scaffold 2 staple 48 | TTTTTTTCTTACCAGTAACATAGCATAGTGAATTTATCATGATAAAT |

(continued)

| SEQ ID NO | Description | Sequence |
|---|---|---|
| 180 | Scaffold 2 staple 49 | AACACCGGAATCATAACGGCCAACGCTAACGACCTTTTTA |
| 181 | Scaffold 2 staple 50 | GGTCTGGTCAGCAGCTATAGCCCGAAGAGTCCACTATTAA |
| 182 | Scaffold 2 staple 51 | GATATATTCGGTCGCTAAAGACAGTGTCAATCATCTACAA |
| 183 | Scaffold 2 staple 52 | TTGTATAACAGGGAGTTCTTAAACAGCTTGATCGCAACTG |
| 184 | Scaffold 2 staple 53 | AACATACCATTAATTGCGTTGCCGGGTTAGCGGGCCGTTTTCACG |
| 185 | Scaffold 2 staple 54 | AATGAATTTGTCGTCTTTCCAGATTCTGAATAATGGTTTCTCCGTGGTGAAGGGA |
| 186 | Scaffold 2 staple 55 | GAGATTATTTGCACGGCACGTATGGGATAGCATGCCCCC |
| 187 | Scaffold 2 staple 56 | ATTGCTTTATCGTAGGAATCAGGGAGCCGTTTTTATTTTCATATCCCA |
| 188 | Scaffold 2 staple 57 | TAGCGTTTGACATGCAATGCCTGAGTAATGTGAATATGAT |
| 189 | Scaffold 2 staple 58 | AAAACTGGTAATAAGTTTTAACGGAACAGTTAAAGCCCACCACCCT |
| 190 | Scaffold 2 staple 59 | ATTACGCAAACGCAACCGAGGAAATATTTAACAACGCCAACATGTTTTT |
| 191 | Scaffold 2 staple 60 | GTTAAATAATCCTGAATCTTAAATACAAAAGGGCGACATTCAACC |
| 192 | Scaffold 2 staple 61 | AACGAGTAGATTTAGTACGAGGCGATATTCATTACCCA |
| 193 | Scaffold 2 staple 62 | GTGCACTCTGTGGTGGGTTTTGCAGTGTTTTTCGATTTCGGAACCTA |
| 194 | Scaffold 2 staple 63 | TACAGGCAAGGTCAATTCTACTAATTTTTTGTAGTAGTAAACGTTAACTAGCA |

(continued)

| SEQ ID NO | Description | Sequence |
|---|---|---|
| 195 | Scaffold 2 staple 64 | TGTTCTTGGGGGTTTCTTTTTGCCAGCACACTCAATCCGCCGGGC |
| 196 | Scaffold 2 staple 65 | GAGAGGGTAGCTATTACGCTGAGGTCACCCTCAGCAGCG |
| 197 | Scaffold 2 staple 66 | TTTGCGGATTTTTGGCTTAGAGCGTTTTATTTTTTTCGAGCTTCAAAG |
| 198 | Scaffold 2 staple 67 | ATCCTCATTAAAGGCAGGTCACATTTTCGTTCATAATTATATTTT |
| 199 | Scaffold 2 staple 68 | CACCAATGCCAAAAACTCATATATTTTAAAGGAGGTTGAG |
| 200 | Scaffold 2 staple 69 | GTGTAAAGAACTCGTAATCATGGTCCTGAGAAACGTGCT |
| 201 | Scaffold 2 staple 70 | AATCCCTTATAAAGTGAGACGGGCAACATTTTTCTGATTGCCCTTCACC |
| 202 | Scaffold 2 staple 71 | GTGCCGGATGCCATCCCACCGTAACAAAGCTACTAAAGTCCTTTAAT |
| 203 | Scaffold 2 staple 72 | AACTGATAGCCCTAAAAGTCTTTACAATATTTTTGAA |
| 204 | Scaffold 2 staple 73 | TTGCAGGCCGCGCGGGGTTTTTCTTTTCACCATCAAAAGA |
| 205 | Scaffold 2 staple 74 | AATTATTTTTCACCGTCACCCGGATAACCTCACAGGGGAGCCGC |
| 206 | Scaffold 2 staple 75 | GCGTCTTTCCAGAGCCGACTTGCGGGAGGATATTCTGTCCAGACGACG |
| 207 | Scaffold 2 staple 76 | GGTAATGGGTAAAGGTTTCTTTGCTCGGCCAAGCTTTCGCGCGTGCC |
| 208 | Scaffold 2 staple 77 | AGATTAAGTTTCCATTAAACGGGTCTTTTTCATGAGGAAG |
| 209 | Scaffold 2 staple 78 | CCGGAGACAGTCAAATCACCATCTAGGTAAACCCGGTTG |

(continued)

| SEQ ID NO | Description | Sequence |
|---|---|---|
| 210 | Scaffold 2 staple 79 | CCAGTCGGGAAACCTGTCGTTTTTGCCAGCTGCATTAATGAA |
| 211 | Scaffold 2 staple 80 | CTTATCATTCCAAGAAAACAAGCAGTGCCTAACCCTGCAT |
| 212 | Scaffold 2 staple 81 | ACAGTATCGGCCTGCCAGTTACGAGGCATAGTAAGAGC |
| 213 | Scaffold 2 staple 82 | TAGGTGTATTTTTCACCGTACTCACTCCGGCTAGAACGCGTAAAGTAA |
| 214 | Scaffold 2 staple 83 | TTAAATCCGCTGGCAATGAAGGGAAGCCGCACAGGCGGCAGATGGGCGCATCGTA |
| 215 | Scaffold 2 staple 84 | AGGGTGAGAATGTTTATTTTTCTATATTTTCATTTGGTAGATACA |
| 216 | Scaffold 2 staple 85 | CATCGGAAACTAAAGAAAAATACGTAATGCCACTACGAAGCGATTTTAAGATTTTT |
| 217 | Scaffold 2 staple 86 | GCTATTAGGTGGCACAAAGAATTGCTCCTTACCGGAAGTCCCCCT |
| 218 | Scaffold 2 staple 87 | CCCCTTATAAACCATCGAATGTAATACTTTTGCGGGAGAA |
| 219 | Scaffold 2 staple 88 | GCCGCGCTTAATGCGCCGCTTAGAATCAATCGGAACGTTTCCTG |
| 220 | Scaffold 2 staple 89 | ATAATACAACCGCCACAGAAGATAACTTGTTTTTCTGAGTAGAAGAACTTCTTTGA |
| 221 | Scaffold 2 staple 90 | CTTTAGTGGCCTCCGGCCAGAGCACATCCGAACAAACTCCGAACAAACAACAACCC |
| 222 | Scaffold 2 staple 91 | GAGTGAATGTTATATTTTTTACTATATTTTAATGGAAACAGTACA |
| 223 | Scaffold 2 staple 92 | AGAAAAGTGTTTTCCCATTCATTAAAGATAATAATTTTTTTTTACCAG |
| 224 | Scaffold 2 staple 93 | ACCAGCTTCTGCTCCAATAAATTGTGTCGAAACTGTTGCC |

(continued)

| SEQ ID NO | Description | Sequence |
|---|---|---|
| 225 | Scaffold 2 staple 94 | TTTTTAACGGAACCAGAGCCACCAAGAACCGCCACCC |
| 226 | Scaffold 2 staple 95 | GCACTCCAGCCAGCTAATAGCGAGAGGCTTTATCATAACCCTCGTTT |
| 227 | Scaffold 2 staple 96 | AGCCCTCATGAAGGGTTAGAACCACAGAGAGTCAAAAATCAGAGGGT |
| 228 | Scaffold 2 staple 97 | AAATCAATATATGTACAATAAACAATTTTTATGTTCAG |
| 229 | Scaffold 2 staple 98 | AAGTTTATTCAGTAGCTTCATCGGGACGATTG |
| 230 | Scaffold 2 staple 99 | GGTCTGAGTAATTTTCCCTTAGAATCCTTGAAATAAAGCCTCATCTT |
| 231 | Scaffold 2 staple 100 | AGGGCGAAAAACCGTCAACTTAAAAAGGAGCG |
| 232 | Scaffold 2 staple 101 | ATACACTAAAACTTAATTTCATTTTTCTTTAATCATTGGGAAG |
| 233 | Scaffold 2 staple 102 | ACACTTGCTTTTTAAAAGAAGTTTTCAGGAAGAT |
| 234 | Scaffold 2 staple 103 | AAAGAGTTTTTACAGATGCTAAAACGAAAGAGGCAAAAG |
| 235 | Scaffold 2 staple 104 | ATACGTAAAAGGGAACCTGAAATAATAGAATATCTT |
| 236 | Scaffold 2 staple 105 | ATAAAAGAGTATGTTATGATTAAGACTCCTTAGCCGAACAAAGTTTTTT |
| 237 | Scaffold 2 staple 106 | GCCGATTTTTTTAAGGGATTGCTAAATTTTTAGGAGTTTAGAAG |
| 238 | Scaffold 2 staple 107 | ACCAGTCCCGGAATTTGACCGATCTAAAGTTTTTCTGTATGGG |
| 239 | Scaffold 2 staple 108 | CCAATAATAAGAGCAATCAGAGGTTAAATATTTTTTTGACGGAAATT |

(continued)

| SEQ ID NO | Description | Sequence |
|---|---|---|
| 240 | Scaffold 2 staple 109 | AAATATTCATTTCATAACGGAACAGAGAGTACTAACAAC |
| 241 | Scaffold 2 staple 110 | AATATCCAGAACGGTAGTGAGGCCTGAGGATCAGAATGCGCCTGCAGC |
| 242 | Scaffold 2 staple 111 | GAAACAATGAAATAGCTTTTTATAGCTATCTTACCGAAGC |
| 243 | Scaffold 2 staple 112 | TCAGTACCGGATTAGGATTAGCGGAGCAATACTCAAACTATATAATCA |
| 244 | Scaffold 2 staple 113 | TTATTCTGCAAGAGAAAGGCGGATAAGTGCCGTGTCACT |
| 245 | Scaffold 2 staple 114 | AAACAACTGAACAACTAAAGGAATTGCGAGTGGCAAAATC |
| 246 | Scaffold 2 staple 115 | GAATATAGGCATTTTCGAGCCAGAACGCTCAACAGTTTTTT |
| 247 | Scaffold 2 staple 116 | TTCAGAAAACGAGAATGACTTTTTATAACATCAGTTTTTTGAGATT |
| 248 | Scaffold 2 staple 117 | GGTTGCTTGATACAGGAGTGTTACCTACATTTGAGCGGAACAATTCATCAATGGCAA |
| 249 | Scaffold 2 staple 118 | TCATTCCAAGAACCGGCAGACGGTCAAAGTACAACGGAGATTTGTATC |
| 250 | Scaffold 2 staple 119 | ATAGTTAGCGTAATTGAGCCATTTGGGAATTTTTTAGAGCCA |
| 251 | Scaffold 2 staple 120 | AACGATGCCGCTTATTAATTTTAAAAAGAAACCACTTTTTAGAAG |
| 252 | Scaffold 2 staple 121 | GGTTTGCCCTTTTTAGCAGGCGAAAATCCTAAGCCGGCGAACGTGGC |
| 253 | Scaffold 2 staple 122 | TGCCTGCCTTGCTGGTGCTCATGGTTCCTCGTACAGGGCG |
| 254 | Scaffold 2 staple 123 | AATCAAGCATAGGAGCAGCAGCAAAGCAACAGTGCCACGCTTTGACCAT |

(continued)

| SEQ ID NO | Description | Sequence |
|---|---|---|
| 255 | Scaffold 2 staple 124 | TTTTTTACATAAAGGTGGCAACATGAAGCTTGCTTTCGA |
| 256 | Scaffold 2 staple 125 | GAGGCGTTCTAATGCAGAACGCGCATTACCTTTGTAAATGTTTCAATT |
| 257 | Scaffold 2 staple 126 | TAGCACGACCAGTAATGGCACAGAATGCGCGGCGTAA |
| 258 | Scaffold 2 staple 127 | CTGGTCTGTCCATCACTTAGTAATATTTTTCATCAAAC |
| 259 | Scaffold 2 staple 128 | TTTAACCAATAGGTTCAAATATCGAATATTTTTTGCGCCG |
| 260 | Scaffold 2 staple 129 | TTTTTTACCAGAAGGAAAAGACACCATTACTAGAGTGTGATA |
| 261 | Scaffold 2 staple 130 | AAAAGGAAAGGGGGTAATAGTAAAGGACGACGAGTCAGAA |
| 262 | Scaffold 2 staple 131 | TTAAACAGAACTAATGAGGTCATTAGCTCAACATGTTTTA |
| 263 | Scaffold 2 staple 132 | TCGCCATTCAGGCTGACCGATAGGTTAAAATTTAAATTGCATTAAC |
| 264 | Scaffold 2 staple 133 | CAATCATAAGGGAACCCAATCCCAATTCTGCGTTTCGCAAATG |
| 265 | Scaffold 2 staple 134 | GGAAGCCCGAAAAAACCAGGCAAAGGGTCCGCTTCT |
| 266 | Scaffold 2 staple 135 | TTCGCCTGGCGCGCCTCAGACGATCCAGCGCACAAGAAAAGAAAAATA |
| 267 | Scaffold 2 staple 136 | AAAAGAAGATTACATTTAACAATTTCATTTGACTGTTTATATTATTCA |
| 268 | Scaffold 2 staple 137 | TTTTTAGGGCTTAATTGAATCATATGCGTTATACAAATTTTT |
| 269 | Scaffold 2 staple 138 | TAGATTTTTACAAACTCGTAACACTGAGTTTCCGCCACCCAATCGCGC |

(continued)

| SEQ ID NO | Description | Sequence |
|---|---|---|
| 270 | Scaffold 2 staple 139 | CTGCGGCTGGTATGAGCCGGGTCAATCACCTTAGGAAGGT |
| 271 | Scaffold 2 staple 140 | GGCGCGAGTGGAAGTTAATATGCAGCTCATTC |
| 272 | Scaffold 2 staple 141 | GAATACCAACCTTTTACCATGTACACAACGCCCCTTGAGCAAACAAATA |
| 273 | Scaffold 2 staple 142 | CGAGGGTAGCAACGGCTCGCCCACCTGATTTTTTATAATGCTGT |
| 274 | Scaffold 2 staple 143 | AGTTTGCCGTCACCAGCAGGTTTAACGTCCTATTTATCCC |
| 275 | Scaffold 2 staple 144 | TATAACAGTTGATGAGAATCGATTTTTGAACGGTAATCGTAA |
| 276 | Scaffold 2 staple 145 | TTTTCAAACAAAATCACCGGAGGTTTAGTACC |
| 277 | Scaffold 2 staple 146 | AATTTTTGTTAACGTGGTGAATTTAAAGGCCGCTCTAAATTTAATG |
| 278 | Scaffold 2 staple 147 | TAGAACCCAGGAAGAATAATCAGCGGGATCAAGAGTCAGATAGCTT |
| 279 | Scaffold 2 staple 148 | AGCCGCCGCCACCCTCCCGGAACCGCCTCCCTAAATCATAAGTTAATT |
| 280 | Scaffold 2 staple 149 | ACCAGTAGCACCGTAATTTGTCACTACCGACCAAAAGCC |
| 281 | Scaffold 2 staple 150 | CGAACCAGTTGATAAGATCGTCATAGTGAATAAGGCTTGCTCTTGACA |
| 282 | Scaffold 2 staple 151 | TAACAGTGCCCGTATAGGTCAGTGTGTAGCATTCCACGATAAAACAG |
| 283 | Scaffold 2 staple 152 | TCAGATGCGCTCACTGTGAGCCTCTGTTATCCCTGAGAGAGTTG |
| 284 | Scaffold 2 staple 153 | CAGCGGTGCCGGTGCCTGAGTGAGCCGAGCTCGAATGGACATTTTCA |

(continued)

| SEQ ID NO | Description | Sequence |
|---|---|---|
| 285 | Scaffold 2 staple 154 | CGGTGCGGGACAACGCCATCAAAATTTCATCAACATTA |
| 286 | Scaffold 2 staple 155 | TTATTACAGTTTTTGTACAGGACGTTGTGAATTACCTTATGGCACCAAC |
| 287 | Scaffold 2 staple 156 | ATGATGAAACAAACATGTCGAGAGGCCACCCTAAGTATTA |
| 288 | Scaffold 2 staple 157 | CTGAAAACATCGCCATTAAAAATACACCGCCTTGAAAAATCTAAAGC |
| 289 | Scaffold 2 staple 158 | TCAATAACCAGGAGGCTATCAGGTCATTCTTTGAGG |
| 290 | Scaffold 2 staple 159 | TTTTTATAAAAACCAATTCCGGCACTTTACCCTTTTTTACTATTAT |
| 291 | Scaffold 2 staple 160 | CTCACAGTACCGAGTAAAAGAGTCAGTTTTTTGGCAAATCAA |
| 292 | Scaffold 2 staple 161 | TAATCGGCTAGCAAGCGATTTTTTAAATTGCGACAGCGCC |
| 293 | Scaffold 2 staple 162 | CGCGTCCGCCCGCTTTGCCTGGCCGCTCACAACCGTGTTTGATGGTGG |
| 294 | Scaffold 2 staple 163 | TCATTTTTGAGCCTTTAATTGTATCGGTTTATCAAATCCATTAGCATAGAAAGTTCAACAG |
| 295 | Scaffold 2 staple 164 | GAGGCTTGGCAAATATTCGCATTATTCAACGAAAGCTA |
| 296 | Scaffold 2 staple 165 | CCAGCATTGCCATCTTGTCATAGCGTTTGAAAAATCAATA |
| 297 | Scaffold 2 staple 166 | GGGCGGTTAAGAATTTTTAGCGATTTCTGCTCATTTGCCG |
| 298 | Scaffold 2 staple 167 | CAGCAGTTAGAACGTGGATTTTTTCCAACGTCAATTCCGAAATCGGCA |
| 299 | Scaffold 2 staple 168 | CTGACTTTGAAAAGCCTAATGCCGATTCAACCGTTCTAGCCAGAGCCG |

(continued)

| SEQ ID NO | Description | Sequence |
|---|---|---|
| 300 | Scaffold 2 staple 169 | TACTGAACACCCTGGCATTAGAGAACCATACGGAAATTGTTTGGATTATACGATTATCA |
| 301 | Scaffold 2 staple 170 | TCGCACTCATCGAGCGGGTATTAGCATAAACACCCAAA |
| 302 | Scaffold 2 staple 171 | TATCAGAACATAAAAACAGGGAAGCAACAAAGTGAAAATAG |
| 303 | Scaffold 2 staple 172 | TAGAAACCAATCAATCCTAATTCTGAACAACAAAATTA |
| 304 | Scaffold 2 staple 173 | GCAAATTAACCGTTGTCTGCGGCCCCCGGGTACTAACTCAGAGCCGGAAAACCT |
| 305 | Scaffold 2 staple 174 | AAGATTAGTTGAGATGAATATACATATAGAAGGATAAGTCTACGAT |
| 306 | Scaffold 2 staple 175 | GAAACAATCCTTTACCGCGCCCAATGTCTTTCAATTGAGCT |
| 307 | Scaffold 2 staple 176 | CGCCAGAATCATAGCTCCTAAAGGTCAAGTTTCACTACGTCGGGAT |
| 308 | Scaffold 2 staple 177 | TGCTCCAATACTGCGGACAGATACATAACGCCCCACATTC |
| 309 | Scaffold 2 staple 178 | TGAATGTTCCAGCGTTTTTTGTGGTGCTTATTAGAC |
| 310 | Scaffold 2 staple 179 | TAAGATTGGGCGTGCCAACGAGCATCAGAGGAATTGGCTGAACCAGAGGTG |
| 311 | Scaffold 2 staple 180 | TGCAAGAACGAGAAGAGTAACCTGACGAGAACAACATAGGAATA |
| 312 | Scaffold 2 staple 181 | AGAAAGGAAGGCAAACGCGGTCTTTGGAACAGATAGGGTTGAGTGTTTAT |
| 313 | Scaffold 2 staple 182 | GAACTGACCCTTCATCTAGTAAATACTAACGGAAACACCTGTT |
| 314 | Scaffold 2 staple 183 | AGCAAGCGGTCCACGCAACCGCAAGAACCAGGGTGGAGAGGCGGTTTGCGTTACT |

(continued)

| SEQ ID NO | Description | Sequence |
|---|---|---|
| 315 | Scaffold 2 staple 184 | GCAGTAGAGTAGGTAGAGATTAGGCACACGGGAAGTGAGAGAAAGAATTGCGAAACGT |
| 316 | Scaffold 2 staple 185 | GCAGTAGAGTAGGTAGAGATTAGGCAATATTCCTCGTTAGTACTTTTGATTGACAGAC |
| 317 | Scaffold 2 staple 186 | GCAGTAGAGTAGGTAGAGATTAGGCAGGCGCTAGGGCGCTATGAGCTTGA |
| 318 | Scaffold 2 staple 187 | GCAGTAGAGTAGGTAGAGATTAGGCACGACATAAAAAAATCCCGTAAAAATAAAGTTA |
| 319 | Scaffold 2 staple 188 | GCAGTAGAGTAGGTAGAGATTAGGCAGTCGGATTTATCATTTCATTGGCAAGGTCGTA |
| 320 | Scaffold 2 staple 189 | GCAGTAGAGTAGGTAGAGATTAGGCAGCAAAGCGGATTGCGTCAAATGCTGACTGGATAGCGT AATT |
| 321 | Scaffold 2 staple 190 | GCAGTAGAGTAGGTAGAGATTAGGCAGGTGCCGGGCCTCTTCGCTATTACAGGGCGAT |
| 322 | Scaffold 2 staple 191 | GCAGTAGAGTAGGTAGAGATTAGGCATTTCAGCGCCTTCCTGGTACCAAAAAAATCAGC |
| 323 | Scaffold 2 staple 192 | GCAGTAGAGTAGGTAGAGATTAGGCAGTTGGGTAACGCCAGGAAGCAGATCGCCAAAG |
| 324 | Handle 1 | GCAGTAGAGTAGGTAGAGATTAGGCA |
| 325 | Handle 2 | ACGGATTAGAGATGGATGAGATGACG |
| 326 | Handle 3 | GTGTCTGGTATGGTGCTGGGATCGTG |
| 327 | Handle 4 | TGGTGCTATGTGTCTGGTCTGGGTAT |
| 328 | Handle 5 | GTGTGATGTGGAGTGAGGTGGAGGTG |
| 329 | Handle 6 | AGCAGTAGAGTAGGTAGAGATTAGGC |
| 330 | Handle 7 | AGGGATTAGAGATGGATGAGATGACG |
| 331 | Handle-binding | GCAGTAGAGTAGGTAGAGATTAGGCA |

## Claims

1. A DNA-based nanostructure, wherein the DNA-based nanostructure comprises at least a first plurality of virus-binding moieties, wherein each virus-binding moiety of the at least first plurality of virus-binding moiety is neuraminic acid or a derivative thereof.

2. The DNA-based nanostructure of claim 1, wherein the DNA-based nanostructure is formed by self-assembling DNA-based building blocks;

   each of the self-assembling DNA-based building blocks is formed by a single-stranded DNA template strand and a set of oligonucleotides that are at least partially complementary to said single-stranded DNA template; and wherein each of the oligonucleotides is either complementary to one contiguous DNA sequence stretch or to at least two non-contiguous DNA sequence stretches on said single-stranded DNA template.

3. The DNA-based nanostructure of any of the preceding claims, wherein the DNA-based nanostructure comprises a second plurality of virus-binding moieties; optionally

   wherein the DNA-based nanostructure comprises a third plurality of virus-binding moieties, a fourth plurality of virus binding moieties, a fifth plurality of virus binding moieties, and/or a sixth plurality of virus binding moieties.; optionally
   wherein the virus binding moieties of the second, and when present, third, fourth, fifth, and/or sixth pluralities of virus binding moieties are selected from the group comprising or consisting of: an antibody or a virus-binding fragment thereof, a nanobody or a virus-binding fragment thereof, an affimer, heparin, heparan sulfate, hybrid heparan sulfates, and a neuraminidase inhibitor; optionally
   wherein the neuraminidase inhibitor is selected from the group comprising or consisting of: oseltamivir, zanamivir, ianamivir, and peramivir; optionally wherein the neuraminidase inhibitor is oseltamivir; optionally
   wherein at least two of the pluralities of the virus-binding moiety are different neuraminic acids or are derivatives thereof.

4. The DNA-based nanostructure of any one of claims 1-3, wherein the virus-binding moieties are covalently bound to the nanostructure, optionally wherein:

   a) the virus-binding moieties are covalently bound to the single-stranded DNA template strand; and/or
   b) the virus-binding moieties are covalently bound to the oligonucleotides of the set of oligonucleotides complementary to said single-stranded DNA template; optionally

   wherein the virus-binding moieties are covalently bound to nanostructure by a linker; optionally wherein the linker is selected from the group comprising or consisting of:

   a) an oligo-PEG-linker, optionally wherein the oligo-PEG linker has the structure:

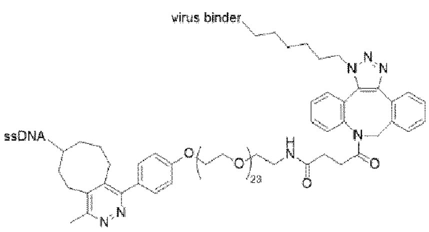

;

   b) a PEG linker, optionally wherein the PEG linker has the structure:

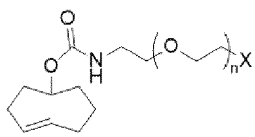

   wherein n is an integer between 4 and 227 and X is a linker covalently bound to the virus-binding moiety,

optionally wherein the linker is DBCO.

5. The DNA-based nanostructure of any one of claims 1-4, wherein the structure comprises one or more handle sites; optionally wherein the one or more handle sites comprise single-stranded DNA overhangs; optionally wherein:

a) wherein the handle sites are comprised by the self-assembling DNA building blocks, optionally wherein each self-assembling DNA building block comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 handle sites;
b) said handles are single-stranded oligonucleotides having a length of between 20 and 200 nucleotides, 50 and 150 nucleotides, 75 and 125 nucleotides, 20 and 60 nucleotides, 40 and 55 nucleotides; 20 and 30 nucleotides, 22 and 28 nucleotides, 25 and 27 nucleotides, 45 and 55 nucleotides, or 47 and 53 nucleotides;
c) the virus-binding moieties are non-covalently bound to the nanostructure; optionally wherein the virus-binding moieties are conjugated to a handle-binding oligonucleotide that is able to hybridise to the handle sites; optionally wherein the handle-binding oligonucleotide is complementary to the handle site; optionally wherein the virus-binding moieties are conjugated to the handle-binding oligonucleotide:

a) by an ssDNA-$PEG_4$-azide linker; optionally wherein the ssDNA-$PEG_4$-azide linker has the structure:

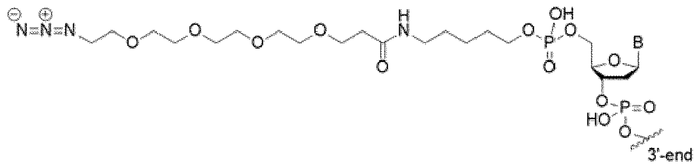
;

b) an ssDNA-DBCO linker; optionally wherein the ssDNA-DBCO linker has the structure:

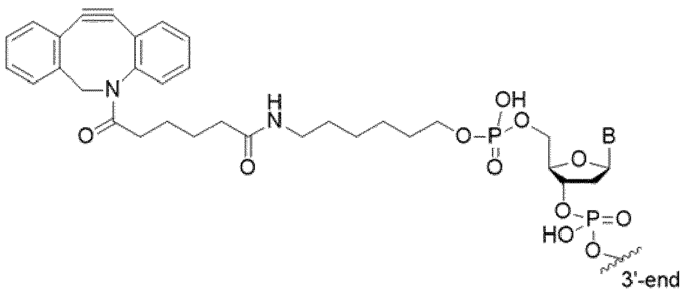
;

c) an ssDNA-SH linker; optionally wherein the ssDNA-SH linker has the structure:

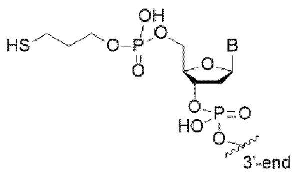
;

d) an ssDNA-$PEG_5$-methyltetrazine linker; optionally wherein the ssDNA-$PEG_5$-methyltetrazine linker has the structure:

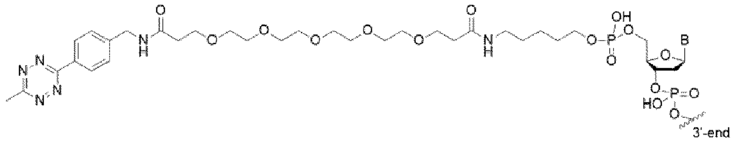
;

and/or e) an oligo-PEG-linker, optionally wherein the oligo-PEG-linker has the structure:

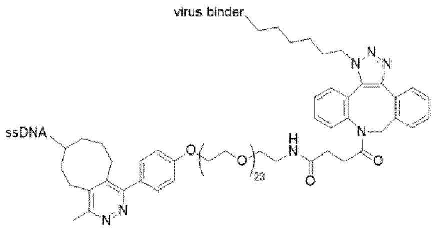

.

**6.** The DNA-based nanostructure of any of the preceding claims, wherein:

i) the neuraminic acid or derivative thereof is resistant to cleavage or degradation by a neuraminidase; or is a non-cleavable neuraminic acid or non-cleavable neuraminic acid derivative; and/or
ii) the neuraminic acid or derivative thereof has a structure selected from the group comprising or consisting of:

a) Compound I:

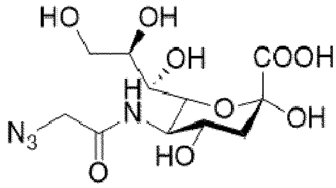

;

b) Compound II:

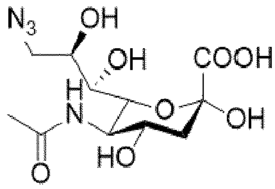

;

c) Compound III:

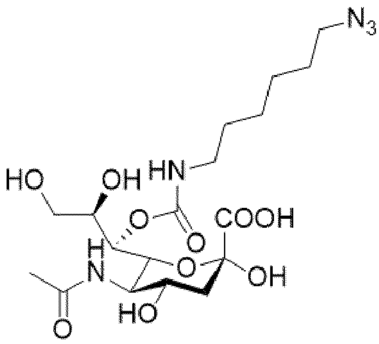

;

d) Compound IV:

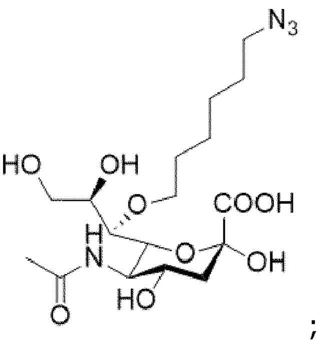

;

e) Compound Va:

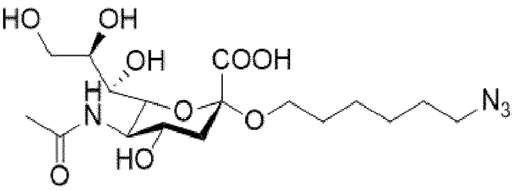

;

f) Compound Vb:

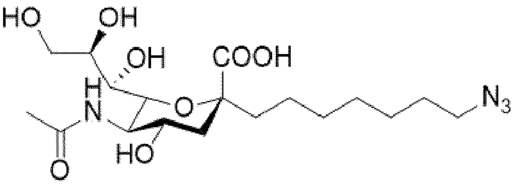

;

and

g) Compound VI:

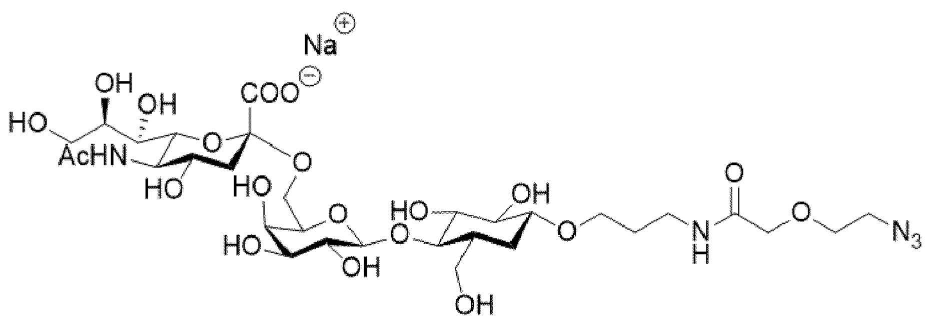

.

**7.** The DNA-based nanostructure of any of the preceding claims, wherein the nanostructure is configured to bind to the surface of a target virus or to at least partially encapsulate a target virus, optionally wherein the target virus is an influenza virus; optionally wherein:

i) the nanostructure is configured to bind to:

a) at least 5% of the surface of the target virus, optionally at least 10%, at least 20%, at least 30%, at least 40%, at least 50%, at least 60%, at least 70%, at least 75%, at least 80%, at least 85%, at least 90%, at least 95%, at least 96%, at least 97%, at least 98%, at least 99%, or more of the surface of the target virus;
b) about 5% of the surface of the target virus, optionally about 10%, about 20%, about 30%, about 40%, about 50%, about 60%, about 70%, about 75%, about 80%, about 85%, about 90%, about 95%, about 96%, about 97%, about 98%, about 99%, or 100% of the surface of the target virus; and/or
c) 5% of the surface of the target virus, optionally 10%, 20%, 30%, 40%, 50%, 60%, 70%, 75%, 80%, 85%,

90%, 95%, 96%, 97%, 98%, 99%, 100% of the target virus;

ii) the nanostructure is configured to fully encapsulate the target virus;
iii) binding of the nanostructure to the target virus inhibits the virus from binding to and/or entering a host cell; optionally
wherein the host cell is an animal cell, optionally wherein the host cell is a human cell; and/or
iv) binding of the nanostructure to the target virus neutralises the target virus, optionally wherein binding of the nanostructure to the target virus neutralises the target virus:

a) to at least 50%, at least 60%, at least 70%, at least 80%, at least 85%, at least 90%, at least 95%, at least 96%, at least 97%, at least 98%, at least 99%, or more;
b) to about 50%, about 60%, about 70%, about 80%, about 85%, about 90%, about 95%, about 96%, about 97%, about 98%, about 99%, or about 100%; and/or
c) to 50%, 60%, 70%, 80%, 85%, 90%, 95%, 96%, 97%, 98%, 99%, or 100%.

**8.** The DNA-based nanostructure of any of the preceding claims, wherein:

i) the DNA-based nanostructure has a concave surface and/or a convex surface; optionally

wherein the at least a first plurality of virus-binding moieties is bound to the concave surface; optionally wherein when present the second plurality of virus-binding moieties, third plurality of virus-binding moieties, fourth plurality of virus binding moieties, fifth plurality of virus binding moieties, and/or sixth plurality of virus binding moieties are bound to the concave surface;

and/or
ii) each of the DNA-based building blocks is a triangular prismoid; optionally wherein:

a) each said triangular prismoid is formed by m triangular planes, wherein m is an integer independently selected from 4, 5, 6, 7 and 8, in particular independently selected from 5, 6 and 7, more particularly wherein said integer is 6;

the three edges of each of said m planes are formed by n parallel stretches of DNA double helices, wherein n is an integer independently selected from 1, 2, 3, 4, 5 and 6 in particular independently selected from 2, 3, 4 and 5, more particularly independently selected from 3 and 4;
wherein each plane is connected to a plane above and/or a plane beyond said plane (i) by stacking interactions between the DNA double helices forming said planes, and (ii) partially by DNA stretches within said single-stranded DNA template and/or said oligonucleotides forming said DNA-based building block bridging at least two of said planes; and
wherein at least two of the three side trapezoids comprise a specific pattern of recesses and/or extrusions formed by missing or additional DNA double helical stretches for specific interaction with a complementary pattern on the side trapezoid of another one of said self-assembling DNA-based building blocks; optionally
wherein for at least part of the self-assembling DNA-based building blocks the length of at least one edge of each of said m planes is decreasing from the first to the $m^{th}$ plane, so that bevel angles results between planes perpendicular to said first plane and each of the trapezoid planes formed by said m edges;

and/or
b) the DNA-based nanostructure comprises at least one set of self-assembling DNA based-building blocks, wherein one, two, or all three side trapezoids comprises a specific pattern of recesses and/or extrusions formed by missing or additional DNA double helical stretches for specific interaction with a complementary pattern on the side trapezoid of another one of said self-assembling DNA-based building blocks; optionally wherein two of the side trapezoids are capable of specifically interacting with a complementary pattern on the side trapezoid of another one of said self-assembling DNA-based building blocks, and one of the side trapezoids is not capable of said specific interaction;

and/or
iii) the nanostructure comprises or consists of a pentamer of DNA-based building blocks; optionally wherein the DNA-based building blocks each have an identical shape.

**9.** A self-assembling DNA-based building block formed by a single-stranded DNA template strand and a set of oligonucleotides complementary to said single-stranded DNA template, wherein:

each of the oligonucleotides is either complementary to one contiguous DNA sequence stretch or to at least two non-contiguous DNA sequence stretches on said single-stranded DNA template; and
the self-assembling DNA-based building block comprises at least one first virus-binding moiety, wherein the first virus-binding moiety is neuraminic acid or a derivative thereof; optionally
wherein the self-assembling DNA-based building block is a self-assembling DNA-based building block as defined in any one of claims 2-8.

**10.** A DNA-based nanostructure wherein the DNA-based nanostructure comprises at least five self-assembling DNA-based building blocks;

each of the self-assembling DNA-based building blocks is formed by a single-stranded DNA template strand and a set of oligonucleotides that are at least partially complementary to said single-stranded DNA template; and wherein each of the oligonucleotides is either complementary to one contiguous DNA sequence stretch or to at least two non-contiguous DNA sequence stretches on said single-stranded DNA template.

**11.** The DNA-based nanostructure of claim 10, wherein the nanostructure comprises at least a first plurality of target-binding moieties, optionally wherein:

a) the at least first plurality of target-binding moieties is a virus-binding moiety that is a neuraminic acid or a derivative thereof; or
b) the at least first plurality of target-binding moieties is a virus-binding moiety selected from the group comprising or consisting of: an antibody or a virus-binding fragment thereof, a nanobody or virus-binding fragment thereof, an affimer, heparin, heparan sulfate, hybrid heparan sulfates, and a neuraminidase inhibitor; optionally
wherein the neuraminidase inhibitor is selected from the group comprising or consisting of: oseltamivir, zanamivir, ianamivir, and peramivir; optionally wherein the neuraminidase inhibitor is oseltamivir;
c) the at least first plurality of target-binding moieties is a cell-binding moiety, optionally a cell-binding moiety that is capable of binding to the surface of a cell selected from the group comprising or consisting of: a bacterial cell, an archaeal cell, a fungal cell, an animal cell, a mammalian cell, a plant cell, an amoeba cell, a protozoan cell, a helminth cell, a sporozoan cell, a ciliate cell, a trematode cell, a nematode cell, and a cestode cell; optionally
wherein the cell-binding moiety is selected from the group comprising or consisting of: an antibody or cell-binding fragment thereof, a nanobody or cell-binding fragment thereof, heparin, heparan sulfate, hybrid heparan sulfates, and an affimer; optionally

i)

wherein the at least a first plurality of target-binding moieties is bound to the concave surface; optionally wherein when present the second plurality of target-binding moieties, third plurality of target-binding moieties, fourth plurality of target-binding moieties, fifth plurality of target-binding moieties, and/or sixth plurality of target-binding moieties are bound to the concave surface;

and/or
ii) wherein the nanostructure is a nanostructure according to any one of claims 1-8, and/or wherein each self-assembling DNA-based building block is a self-assembling DNA-based building block of claim 9.

**12.** A composition comprising the DNA-based nanostructure of any of claims 1-8, the self-assembling DNA-based building block of claim 9, or the DNA-based nanostructure of any one of claim 10.

**13.** The DNA-based nanostructure of any one of claims 1-8, self-assembling DNA-based building block of claim 9, the DNA-based nanostructure of claim 10 or 11, or the composition of claim 12 for use in medicine.

**14.** The DNA-based nanostructure of any one of claims 1-8, self-assembling DNA-based building block of claim 9, the DNA-based nanostructure of claim 10 or 11, or the composition of claim 12 for use in treating Influenza infection or prophylaxis against influenza infection.

**15.** A method of encapsulating or neutralising a virus, a viral particle, or a subviral particle, comprising contacting the virus,

viral particle, or subviral particle with the DNA-based nanostructure of any one of claims 1-8, the self-assembling DNA-based building block of claim 9, the DNA-based nanostructure of claim 10 or 11, or the composition claim 12.

Figure 1

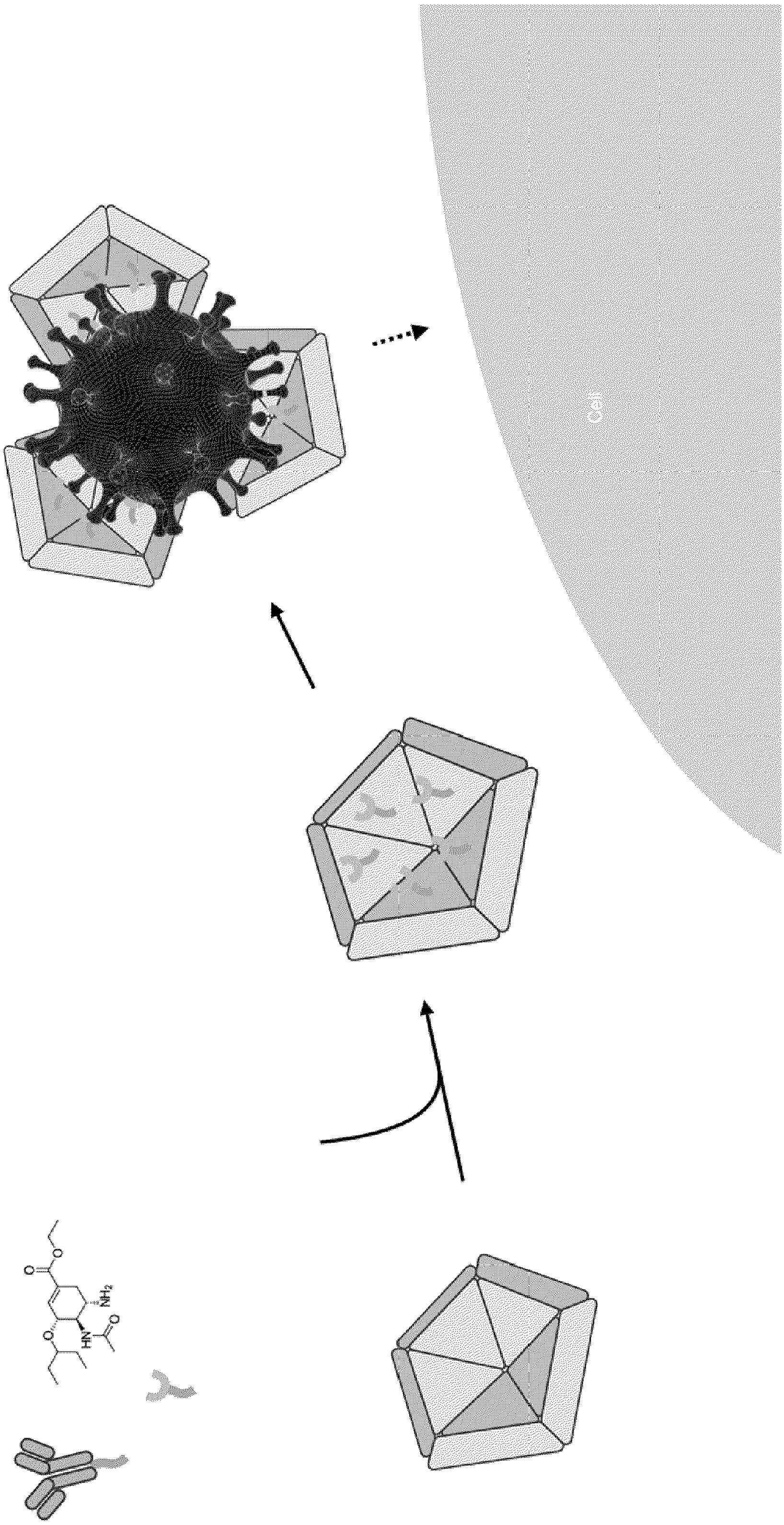

Figure 2

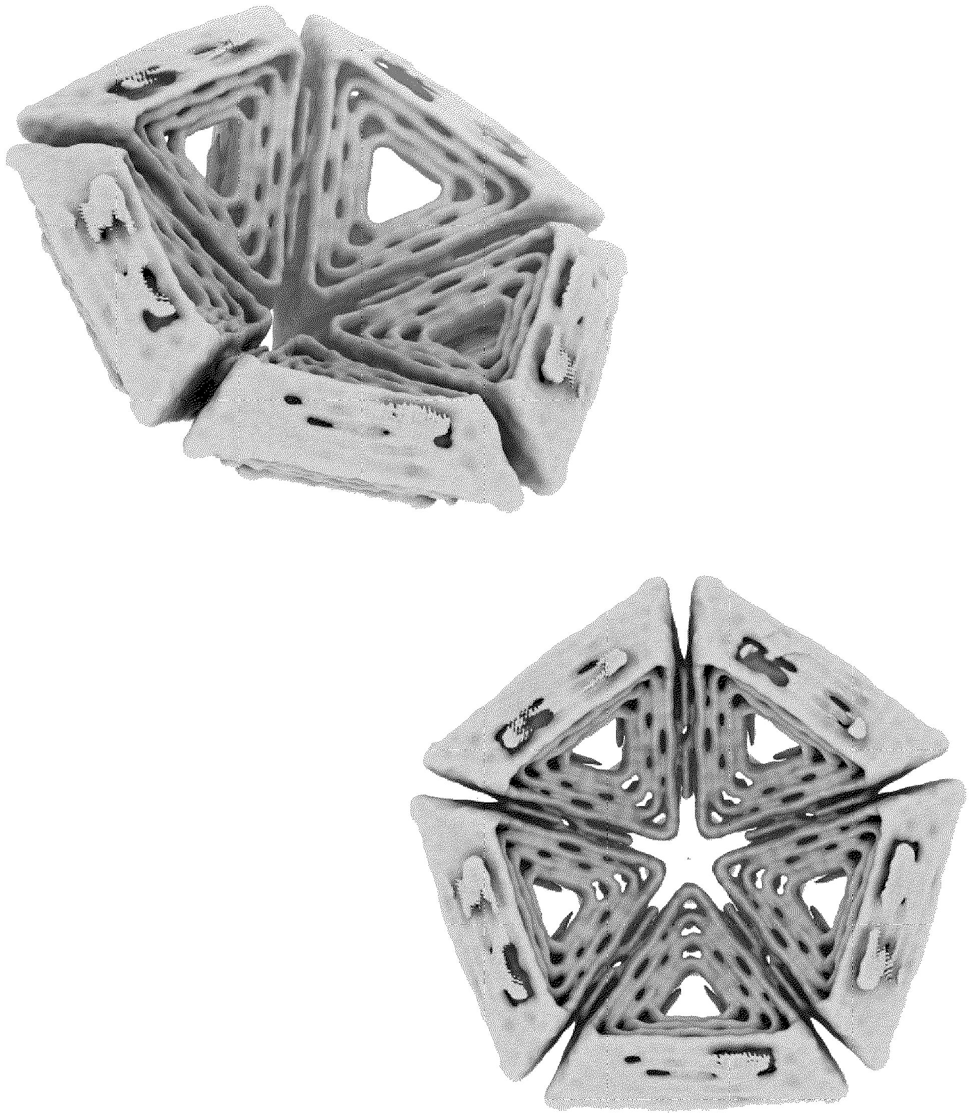

Figure 3

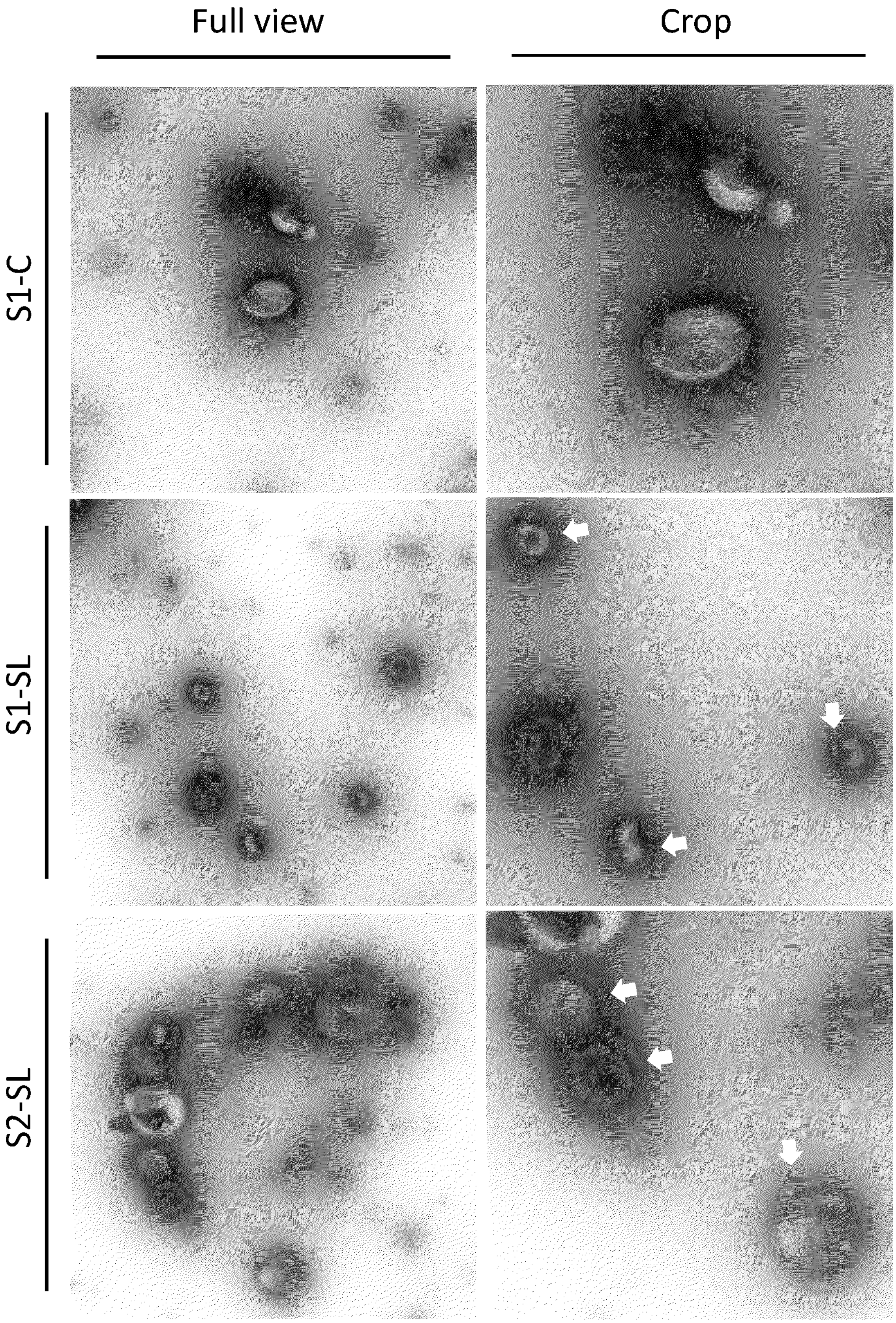

Figure 4 (part 1 of 2)

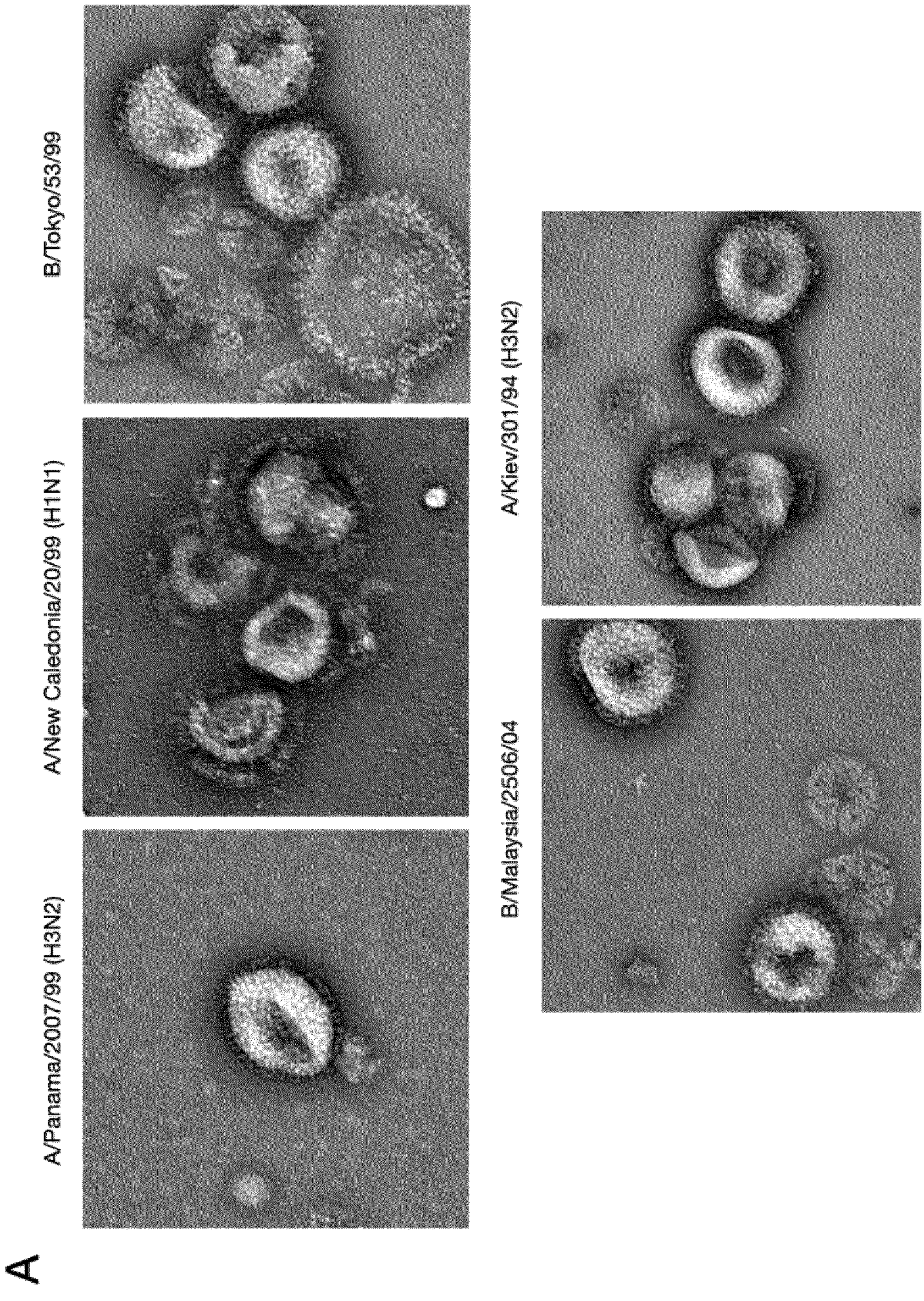

Figure 4 (part 2 of 2)

B

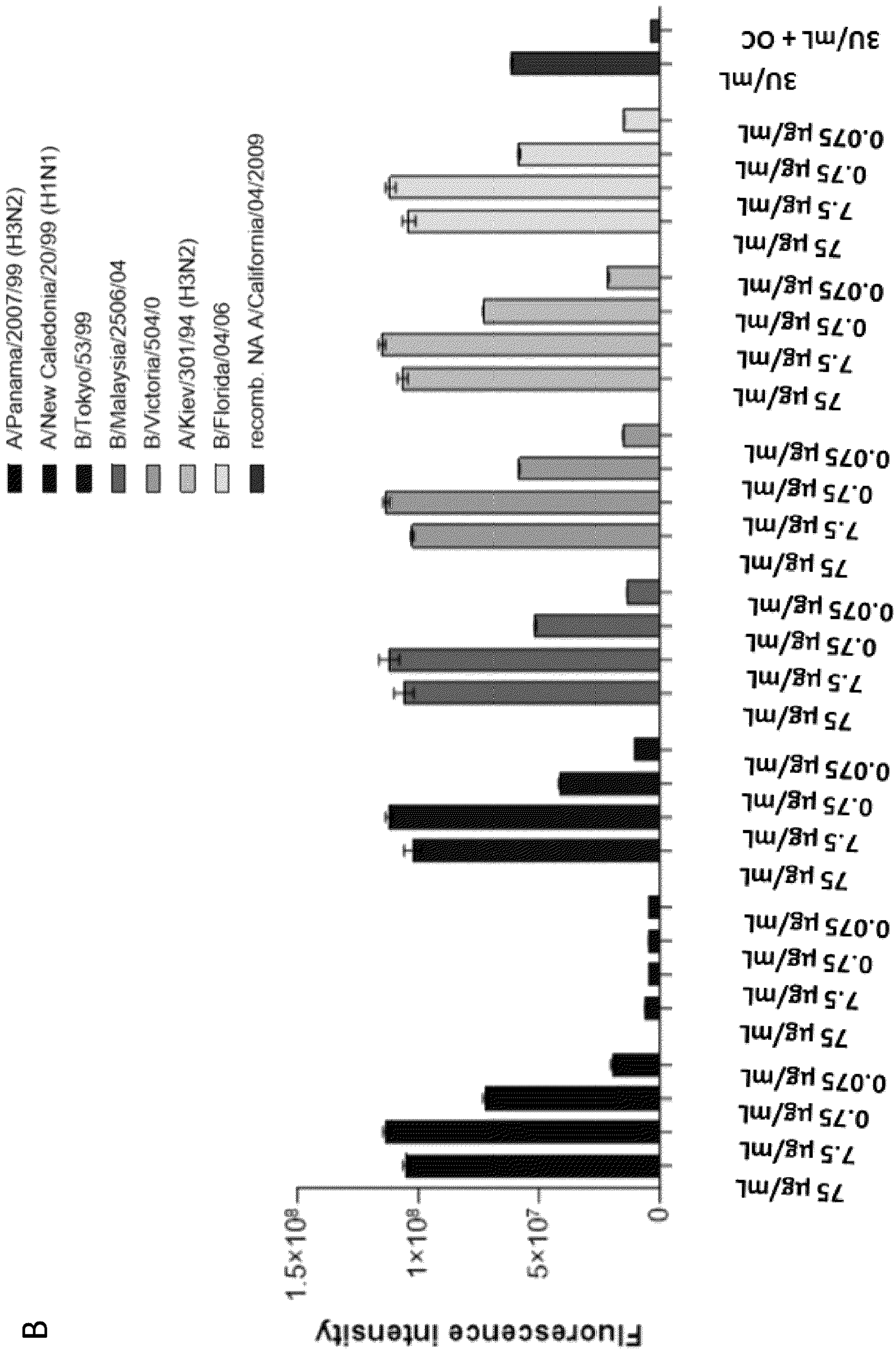

Figure 5 (Part 1 of 3)

A

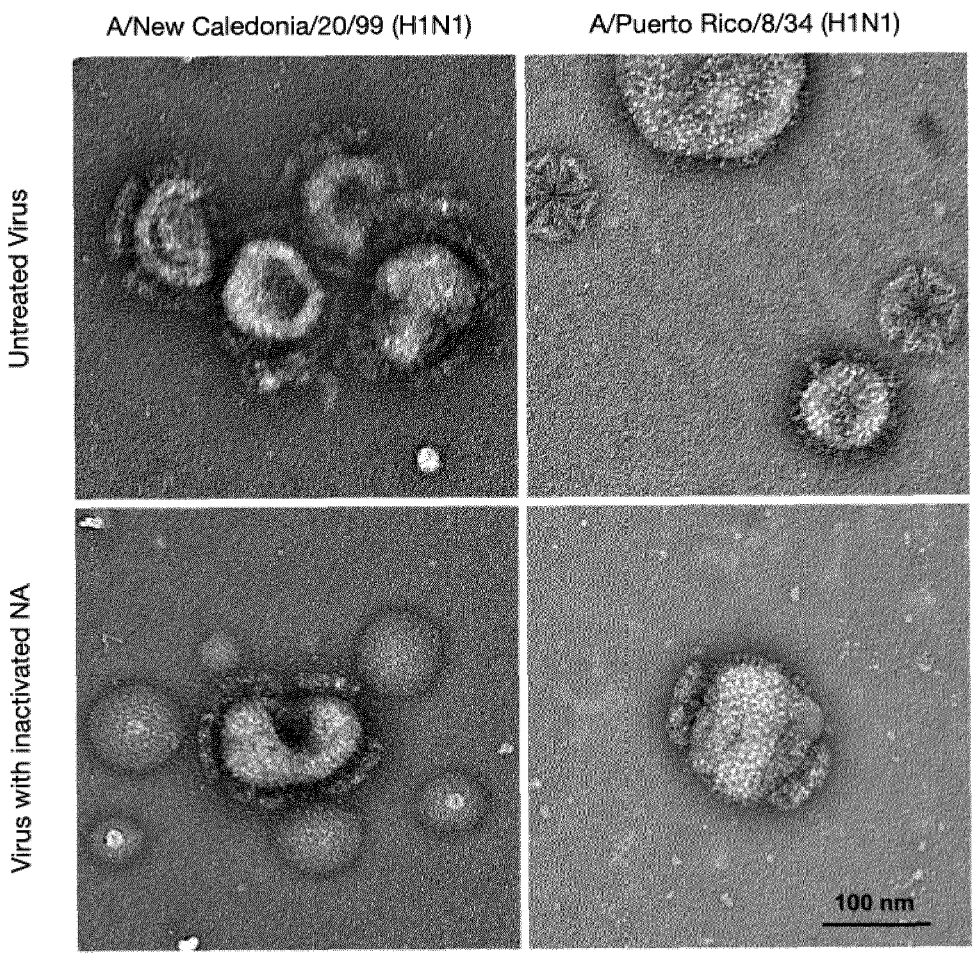

Figure 5 (Part 2 of 3)

B

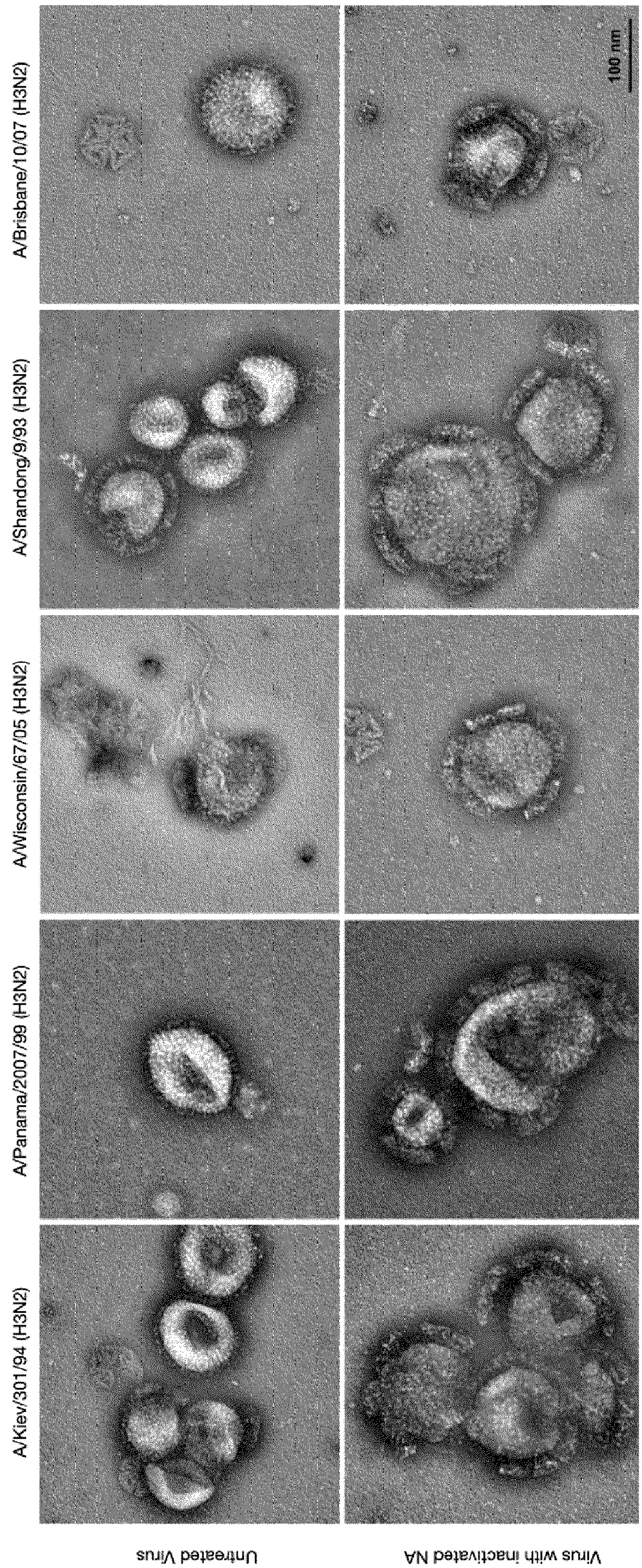

# Figure 5 (Part 3 of 3)

C

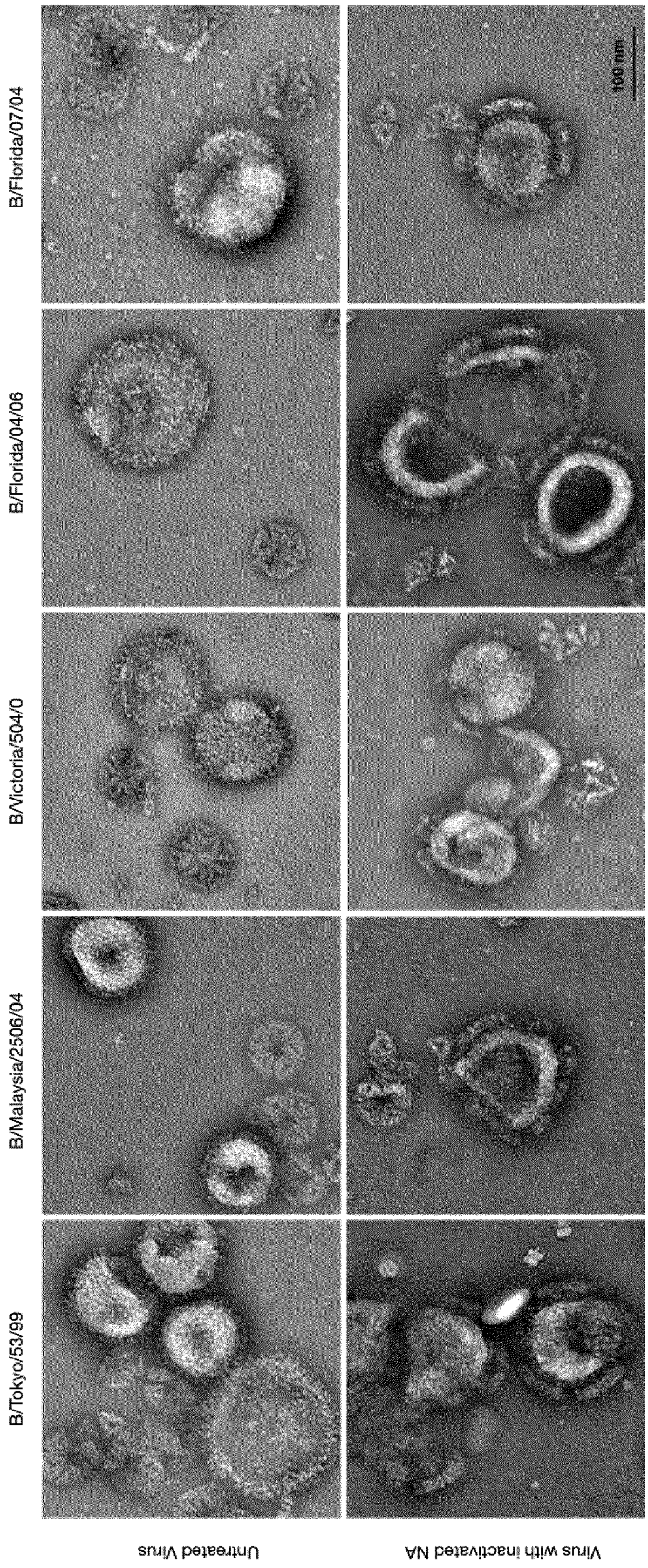

Figure 6

A

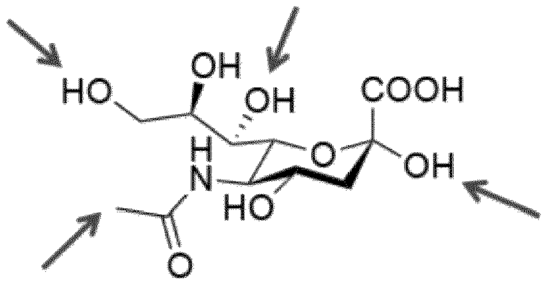

B

Attachement #1

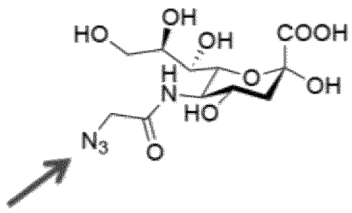

Attachement #2

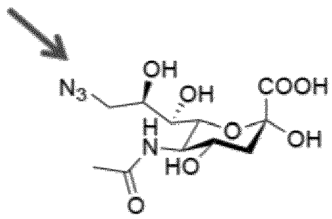

Attachement #3a

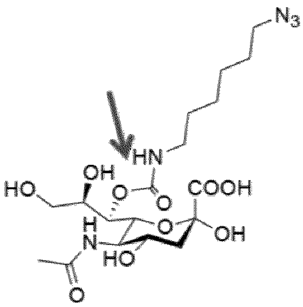

Attachement #3b

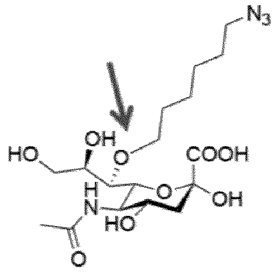

Attachement #5

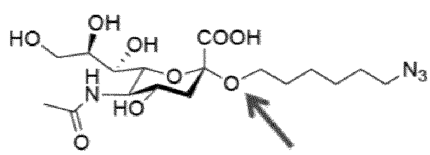

Europäisches Patentamt
European Patent Office
Office européen des brevets

# EUROPEAN SEARCH REPORT

Application Number
EP 24 18 6463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2024/094708 A1 (UNIV MUENCHEN TECH [DE]) 10 May 2024 (2024-05-10)<br>* the whole document *<br>----- | 1-3,5-15 | INV.<br>C12Q1/70<br>B82Y5/00 |
| X,D | WO 2023/209161 A1 (UNIV MUENCHEN TECH [DE]) 2 November 2023 (2023-11-02)<br>* the whole document *<br>----- | 1,2,5,<br>7-13,15 | |
| A | XUEZHENG SONG ET AL: "A Sialylated Glycan Microarray Reveals Novel Interactions of Modified Sialic Acids with Proteins and Viruses",<br>JOURNAL OF BIOLOGICAL CHEMISTRY,<br>vol. 286, no. 36,<br>12 July 2011 (2011-07-12), pages 31610-31622, XP055459906,<br>US<br>ISSN: 0021-9258, DOI:<br>10.1074/jbc.M111.274217<br>----- | 1-15 | |
| A | GANSUKH ENKHTAIVAN ET AL:<br>"Nanotherapeutic Anti-influenza Solutions: Current Knowledge and Future Challenges",<br>JOURNAL OF CLUSTER SCIENCE, SPRINGER US, NEW YORK,<br>vol. 29, no. 6, 4 July 2018 (2018-07-04), pages 933-941, XP036606416,<br>ISSN: 1040-7278, DOI:<br>10.1007/S10876-018-1417-Z<br>[retrieved on 2018-07-04]<br>----- | 1-15 | TECHNICAL FIELDS SEARCHED (IPC)<br>C12Q<br>B82Y |

The present search report has been drawn up for all claims

1

| Place of search | Date of completion of the search | Examiner |
|---|---|---|
| The Hague | 13 December 2024 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 18 6463

This annex lists the patent family members relating to the patent documents cited in the above-mentioned European search report. The members are as contained in the European Patent Office EDP file on
The European Patent Office is in no way liable for these particulars which are merely given for the purpose of information.

13-12-2024

| Patent document cited in search report | Publication date | Patent family member(s) | Publication date |
|---|---|---|---|
| WO 2024094708 A1 | 10-05-2024 | NONE | |
| WO 2023209161 A1 | 02-11-2023 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2017068051 W
- WO 2018054571 A
- EP 2023080373 W
- WO 2024094708 A1
- EP 2023061264 W
- WO 2023209161 A1
- EP 2021054307 W
- WO 2021165528 A1
- WO 2023061264 A
- WO 2023080373 A

### Non-patent literature cited in the description

- **PRAETORIUS et al.** Biotechnological mass production of DNA origami. *Nature*, 2017, vol. 552, 84-87
- **SONG et al.** A Sialylated Glycan Microarray Reveals Novel Interactions of Modified Sialic Acids with Proteins and Viruses. *J Biol. Chem*, 2011, vol. 286 (36), 31610-31622
- Programmable DNA Origami Shell Platform for Virus Neutralization. **SIGL, C.** Doctoral thesis. Technische Universität München, 2021
- **GERLING, T. et al.** *Sci. Adv.*, 2018, vol. 4, eaau1157